# FASCICULE DE BREVET EUROPEEN

(11) **EP 1 315 417 B1**
(45) Date de publication et mention de la délivrance du brevet: **01.07.2009**
(21) Numéro de dépôt: 01972136.4
(22) Date de dépôt: 04.09.2001
(51) Int. Cl.: A01K 67/027, C07K 14/705, A61K 49/00, G01N 33/50, C12N 5/10

(54) **MAMMIFERE NON-HUMAIN MUTANT POUR L'EXPRESSION DE LA SOUS-UNITE ALPHA6 DU RECEPTEUR NICOTINIQUE DE L'ACETYLCHOLINE ET UTILISATION POUR LE CRIBLAGE DE SUBSTANCES SUSCEPTIBLES D'INTERAGIR AVEC CE RECEPTEUR**
NICHT-MENSCHLICHES SÄUGETIER MIT FEHLENDER EXPRESSION DER ALPHA6 NIKOTINISCHEN ACETYLCHOLIN REZEPTORUNTEREINHEIT UND VERWENDUNG ZUM SCREENING
MUTANT NON-HUMAN MAMMAL FOR EXPRESSING THE ALPHA6 ACETYLCHOLINE NICOTINIC RECEPTOR SUBUNIT AND USE FOR SCREENING SUBSTANCES CAPABLE OF INTERACTING WITH SAID RECEPTOR

(30) Priorité: 04.09.2000 FR 0011247
(43) Date de publication de la demande: 04.06.2003
(60) Demande divisionnaire: 07009607.8
(73) Titulaire: INSTITUT PASTEUR, 75724 Paris Cedex 15 (FR); CENTRE NATIONAL DE LA RECHERCHE SCIENTIFIQUE (CNRS), 75794 Paris Cedex 16 (FR)
(72) Inventeur: CHAMPTIAUX, Nicolas, F-78240 Chambourcy (FR); CHANGEUX, Jean-Pierre, F-75006 Paris (FR); BESSIS, Alain, F-75013 Paris (FR)
(74) Mandataire: Desaix, Anne
(86) Numéro de dépôt international: PCT/FR2001/002741
(87) Numéro de publication internationale: WO 2002/019812

(56) Documents cités:
- WO-A-00/26392
- WO-A-96/03504
- WO-A-96/41876
- DATABASE EM_GSS E.B.I. Hinxton U.K.; Accession Number: AZ273390, 30 juillet 2000 (2000-07-30) ZHAO S ET AL: "Mouse BAC End Sequences from Library RPCI-23" XP002170975
- DATABASE EM_RO E.B.I. Hinxton U.K.; Accession Number: AJ245706, 24 août 1999 (1999-08-24) LE NOVERE N: "Mus musculus mRNA for nicotinic acetylcoline receptor subunit alpha6 (NICA6 gene)" XP002170976
- ZOLI M ET AL: "Identification of four classes of brain nicotinic receptors using beta2 mutant mice" JOURNAL OF NEUROSCIENCE, vol. 18, no. 12, 15 juin 1998 (1998-06-15), pages 4461-4472, XP001009895
- LE NOVERE N ET AL: "Involvement of alpha6 nicotinic receptor subunit in nicotine-elicited locomotion, demonstrated by in vivo antisense oligonucleotide infusion" NEUROREPORT, vol. 10, no. 12, 20 août 1999 (1999-08-20), pages 2497-2501, XP001009740 cité dans la demande
- CORDERO-ERAUSQUIN M ET AL: "Nicotinic receptor function: new perspectives from knockout mice" TRENDS IN PHARMACOLOGICAL SCIENCES,ELSEVIER TRENDS JOURNAL, CAMBRIDGE,GB, vol. 21, no. 6, 1 juin 2000 (2000-06-01), pages 211-217, XP004213307 ISSN: 0165-6147
- STITZEL J ET AL: "Potential role of the alpha4 and alpha6 nicotinic receptor subunits in" JOURNAL OF PHARMACOLOGY AND EXPERIMENTAL THERAPEUTICS, vol. 293, no. 1, avril 2000 (2000-04), pages 67-74, XP001009945

## Description

La présente demande décrit des mammifères non-humains, mutants pour l'expression de la sous-unité alpha6 du récepteur nicotinique de l'acétylcholine, ci-après désigné par l'expression abrégée « nAChR » et vise l'utilisation de ces mammifères pour l'identification ou le criblage de molécules susceptibles d'interagir avec ce récepteur.

A ce jour, le rôle des récepteurs nicotiniques de l'acétylcholine dans le système nerveux central est peu connu. On sait que ces récepteurs (Marubio, L.M., et al. Reduced antinociception in mice lacking neuronal nicotinic receptor subunits. Nature, 1999. 398 : p. 805-810) sont impliqués dans la nociception, la mémoire, le contrôle de la locomotion, la dépendance à la nicotine, et certains désordres neurodégénératifs tels que la maladie de Parkinson, la maladie d'Alzeimer (Newhouse, P., et al, Nicotinic system involvement in Alzheimer's and Parkinson's diseases. Implications for therapeutics. Durgs Aging, 1997. 11(3) p. 206-228), les mécanismes de cette implication n'ayant néanmoins pas été identifiés.

En ce qui concerne la dépendance à la nicotine, des travaux récents ont fait état de l'importance de la sous-unité beta2 du nAChR vis à vis des propriétés de dépendance à la nicotine chez les rongeurs (Picciotto, M. et al, Acetylcholine receptors containing beta2-subunit are involved in the reinforcing properties of nicotine. Nature, 1998. 391 : p. 173-177). Dans ces travaux antérieurs, des souris dépourvues de sous-unité beta2, étaient incapables de maintenir un comportement d'auto-administration lorsque la cocaïne était remplacée par la nicotine, Cette étude soulignait également l'absence de réponse électrophysiologique des neurones dopaminergiques mésencéphaliques des souris mutantes, en réponse à la nicotine. De plus, des expériences de microdialyse réalisées sur des souris mutantes ont établi que la libération de dopamine était affectée dans le noyau accumbens (correspondant à la partie ventrale du striatum) après l'administration de nicotine par voie systémique. Ces observations contribuent à mettre l'accent sur le parallèle pouvant exister entre la nicotine et d'autres drogues à dépendance, telles que l'héroïne ou la cocaïne, dont les effets de dépendance sont liés à l'augmentation du niveau basal de dopamine dans la noyau accumbens.

Toutefois, le profil d'expression cellulaire étendu de la sous-unité beta2 n'a pas permis la définition précise des régions du cerveau contribuant à l'initiation de la dépendance à la nicotine, et la nature des sous-unités alpha associées avec la sous-unité beta2 dans ce procédé n'a pas été identifiée.

Parmi les huit sous-unités alpha du nAChR déjà identifiées dans le cerveau de rongeurs, certaines ont été utilisées, notamment la sous-unité alpha4, pour examiner leurs propriétés.

Dans le cadre de la présente demande, les inventeurs ont choisi de s'intéresser à une sous-unité particulière, la sous-unité alpha6 des récepteurs neuronaux nicotiniques de l'acétylcholine, cette sous-unité pouvant être associée avec la sous-unité bete2 et présentant un profil d'expression restreint aux neurones catécholaminergiques (Le Novere, N. et et., Neuronal nicotinic receptor alpha 6 subunit mRNA is selectively concentrated in catecholaminergic nuclei of the rat brain European Journal of Neuroscience, 1996. 8(11) : p. 2428-39). De façon tout à fait Intéressante dans la cadre de l'invention, les inventeurs ont sélectionné cette sous-unité pour son profil d'expression principalement localisé au niveau de la substance noire, de l'aire tegmentale ventrale et du locus coeruleus.

Des études (Le Novere, N. et al, involvement of alpha6 nicotinic receptor subunit in nicotine-elicited locomotion, demonstrated by in vivo antisense oligonucleotides infusion. NeuroReport, 19999. 10 : p. 2497-2501) ont attesté que des oligonucléotides antisens permettent de démontrer l'importance de la sous-unité alpha6 dans l'effet hyperlocomoteur de la nicotine en environnement habituel, un effet qui serait médié par le système dopaminergique mésencéphalique. Ces éléments ont conduit certains auteurs à émettre l'hypothèse que la sous-unité alpha6 pourrait avoir un rôle dans la régulation de la libération de dopamine par des agonistes nicotiniques (Reuben M. B.S., et al., Nicotinic receptors modulatig somatodendritic and terminal dopamine relase differ pharmacologically. Eur J. Pharmacol, 2000. 383(1-3) : p. 39-49). Cependant, ces auteurs ont observé que le résultat obtenu sur le potentiel et l'efficacité de l'agoniste testé associé avec la libération de dopamine ne ressemble pas à celui qui était obtenu avec des cholinorécepteurs nicotiniques recombinants contenant la sous-unité alpha6.

L'invention propose des moyens permettant l'observation *in vivo* ou dans un système *in vitro* susceptible de se rapprocher des conditions existant *in vivo* pour identifier ou cribler des molécules susceptibles d'interagir, dans une réaction de type ligand/récepteur, avec les récepteur nicotiniques de l'acétylcholine et en particulier avec ceux d'entre eux comportant la sous-unité alpha6 de ces récepteurs.

L'invention a pour objet l'utilisation d'un mammifère non-humain, d'une préparation de synaptosomes ou d'une culture cellulaire, définis dans le cadre de la présente demande, pour étudier les effets de substances déterminées sur les récepteurs nicotiniques.

La demande décrit des mamifères non-humains, en particulier des rongeurs et notamment des souris, qui sont mutés pour le gène codant pour la sous-unité alpha6 du nAChR, ces mammifères étant dépourvus de sous-unité alpha6 fonctionnelle. Dans la suite de la description, ces animaux seront dit « knockout », pour qualifier la mutation qu'ils présentent au niveau du gène codant pour la sous-unité alpha6 des récepteurs nAChR. Ces mammifères peuvent constituer des modèles animaux pour identifier des substances, et notamment des substances ayant des propriétés pharmacologiques, interagissant avec ladite sous-unité alpha6 au sein de récepteurs nAChR. Ces récepteurs comportant la sous-unité alpha6 pourraient être de type 2α6, 2β2, β3 (Le Novere N. et al, European Journal of Neuroscience, 1998. 8(11), p. 2428-39).

En particulier, l'invention fournit au moyen de ces animaux, ou de produits, notamment de cultures cellulaires, qui peuvent en être derivés, un modèle permettant de déterminer la sélectivité de substances d'intérêt vis-à-vis des récepteurs nAChR. présents au niveau de certaines populations neuronales et en particulier au niveau des neurones catéocholaminergiques.

Ainsi, des substances déterminées peuvent être testées sur des animaux exprimant normalement la sous-unité alpha6 des nAChR et le même test peut être réalisé sur les animaux selon l'invention mutants pour cette sous-unité exprimée sous forme fonctionnelle, de façon à comparer les effets respectifs observés, notamment l'éventuelle interaction de la substance testée, avec la transmission nicotinique et la transmission dopaminergique médiée par la sous-unité alpha6 des récepteurs nAChR.

Les moyens selon invention sont particulièrement adaptés au criblage et en particulier au criblage pharmacologique, de substances susceptibles de se comporter comme des ligands des récepteurs nAChR, via leur interaction avec le site de fixation de la nicotine de la sous-unité alpha6 et en particulier susceptibles d'une activité agoniste ou antagoniste de type nicotinique. De façon particulièrement avantageuse, les substances identifiées se fixent aux nAChR par l'intermédiaire de la sous-unité alpha6 et ont une spécificité restreinte aux neurones dopaminergiques. Cette spécificité peut être détectée du fait de l'expression de la sous-unité alpha6 du récepteur nAChR, restreinte à ces neurones dopaminergiques.

On a par exemple observé que la maladie de Parkinson est caractérisée par une dégénérescence sélective des neurones dopaminergiques de la substance noire.

La capacité des substances identifiées à se comporter comme des ligands de la sous-unité alpha6 des nAChR définit, dans le cadre de l'invention, des substances ayant une activité comparable à celle de l'acétylcholine, ou à celles d'agonistes tels que la nicotine, au moins en ce qui concerne la capacité de l'acétylcholine ou de la nicotine, à se lier aux récepteurs nAChR comprenant la sous-unité alpha6. Des substances possédant ces propriétés sont par exemple la nicotine, la cytisine, l'épibatidine, ainsi que leurs dérivés obtenus par modification chimique.

Des substances antagonistes se liant à la susdite sous-unité alpha6 des nAChR sont par exemple la DhβE, l'hexaméthotium, l'alpha-bungarotoxine, la n-bungarotoxine. Des exemples sont mentionnés dans les publications de Spang J. et al. (Chem Biol, 2000. 7(7) : p. 545-555) et Sharples C. et al (J. Neurosci, 2000. 20(8) : p. 2783-91).

Les résultats disponibles sur l'effet neuroprotecteur de la nicotine dans la maladie de Parkinson (Newhouse, P ; et al. Durgs Aging, 1997. 11 (3) : p. 206-228) ouvrent de nouvelles possibilités pour des thérapies différentes avec des agents nicotiniques. La conception de substances nicotiniques, susceptibles d'avoir des propriétés thérapeutiques et d'être utilisées dans la conception d'un médicament, qui présenterait une sélectivité pour des récepteurs contenant la sous-unité alpha6, pourrait présenter un intérêt notamment en évitant des effets secondaires indésirables tels que les risques cardiovasculaires associés à l'exposition à la nicotine.

La demande divulgue un mammifère non-humain, porteur d'une mutation dans le gène codant pour la sous-unité alpha6 du récepteur nicotinique de l'acétylcholine (nAChR), ladite mutation empêchant l'expression chez le mammifère de ladite sous-unité alpha6 du nAChR. sous une forme fonctionnelle.

Par l'expression «forme fonctionnelle », on entend dans le cadre de l'invention, la forme de la sous-unité alpha6 (α6) du nAChR qui permet la reconnaissance et l'interaction entre cette sous-unité et des agonistes ou des antagonistes des récepteurs dans lesquels elle est présente, et plus particulièrement qui permet la fixation de l'acétylcholine. La forme fonctionnelle de la sous-unité alpha6 est donc celle dans laquelle ladite sous-unité comporte un site de fixation fonctionnel de molécules agonistes ou antagonistes.

Le caractère fonctionnel du site de fixation peut être vérifié par le fait que "acétylcholine, présente ou administrée dans des conditions permettant son interaction avec les récepteurs nAChR comportant la sous-unité alpha6, modifie le niveau de libération de dopamine par rapport au niveau de dopamine qui peut être libéré lorsque la sous-unité alpha8 est absente ou non fonctionnelle. En d'autres termes l'absence de la sous-unité alpha6, ou sa présence sous forme non fonctionnelle altère l'amplitude ainsi que la pharmacologie de la réponse des récepteurs nAChR, à la nicotine. A cet égard, les exemples décrivent des tests utilisables.

On utilisera aussi dans la suite, la référence à à forme fonctionnelle » du gène pour traduire la capacité du gène à exprimer une sous-unité alpha6 sous forme fonctionnelle. Les mammifères non-humains sont donc ceux qui ont subi une inactivation génique du gène codant pour la sous-unité alpha6 des nAChR.

Dans un premier mode de réalisation, le mammifère non-humain est caractérisé en ce que le gène codant pour la sous-unité alpha6 du nAChR est muté par délétion de tout ou partie de ses exons.

Un site de fixation de la sous-unité alpha6 de nAChR reconnu par la nicotine et par d'autres agonistes et antagonistes de ce récepteur est codé par les exons 2, 3 et 4 du gène codant pour cette sous-unité alpha6.

En outre, la délétion des exons 1 et 2, par leur destruction, empêche la traduction connecte de l'ARNm (du fait de la destruction de l'ATG ainsi que des sites d'épissage) même dans l'hypothèse d'une transcription du gène. En réalité, l'absence des exons 1 et 2 empêche la transcription du gène.

La demande décrit donc en particulier un mammifère non-humain, caractérisé en ce que le gène codant pour la sous-unité alpha6 du nAChR est muté par délétion d'au moins une partie de l'exon 1 et/ou d'au moins une partie de l'exon 2, suffisante pour empêcher, au niveau de la sous-unité alpha6 du nAChR exprimé chez le mammifère, la liaison de ladite sous-unité alpha6 du nAChR avec ses agonistes et/ou ses antagonistes.

De façon préférée, le mammifère non-humain selon l'invention est muté par délétion essentiellement complète des exons 1 et 2.

En particulier, la demande rapporte un mammifère non-humain, en particulier un rongeur et notamment une souris, caractérisé en ce que la mutation du gène codant pour la sous-unité alpha6 du nAChR est de type homozygote.

La mutation est par exemple telle que le mammifère a de ce fait un, phénotype (α6^{-/-}) et n'exprime plus la sous-unité alpha6 du nAChR, les deux allèles du gène étant mutés de façon semblable.

Est également divulgué un mammifère non-humain caractérisé en ce que la mutation du gène codant pour la sous-unité alpha8 du nAChR est homozygote, ledit mammifère ayant un phénotype (α6^{Δ1-2/Δ1-2}).

Le phénotype (α6^{Δ1-2/Δ1-2}) correspond à l'expression d'un gène codant pour la sous-unité alpha6, ce gène pétant tronqué au niveau des exons 1 et 2, de sorte que si cette sous-unité est effectivement exprimée et notamment comporte la partie transmembranaire de la sous-unité alpha6 native, le récepteur exprimé n'est pas fonctionnel pour la fixation des agonistes laquelle est normalement dépendante de la transcription et de la traduction correcte des exons 1 et 2.

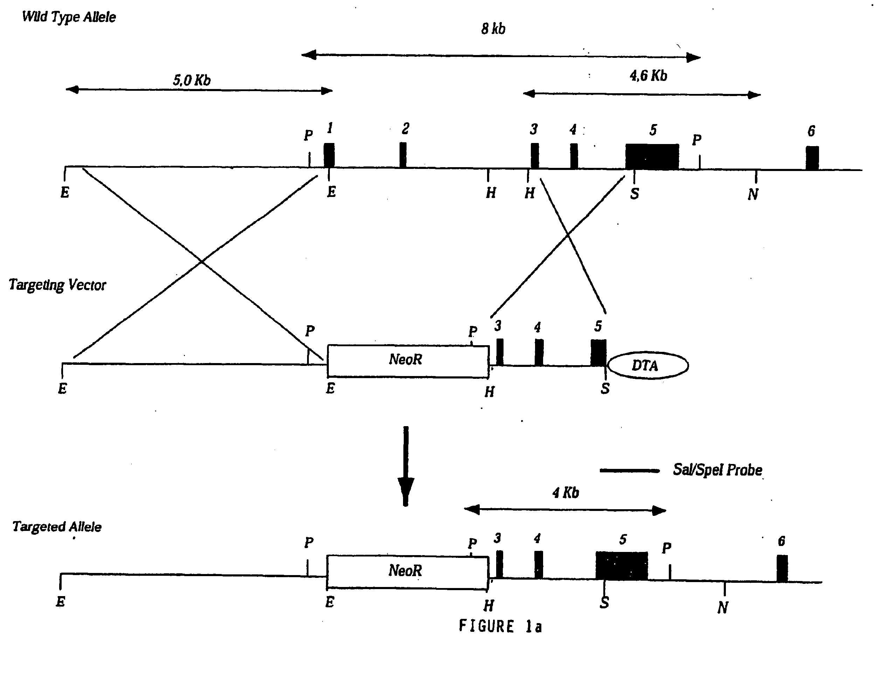

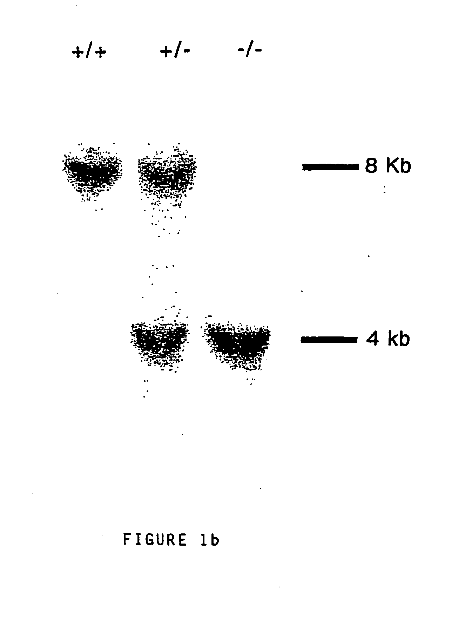

Ces mammifères non-humains peuvent être obtenus par la technique connue de l'homme du métier de la recombinaison homologue du gène sauvage codant pour la sous-unité alpha6 du nAChR, avec une construction du type de la construction cible de la figure 1. Le caractère homozygote de la mutation peut résulter du croisement de deux animaux dont un allèle du gène est muté, la descendance homozygote étant ensuite sélectionnée. Alternativement, le caractère homozygote de la mutation peut être obtenu directement au niveau des cellules Es en augmentant la concentration de néomycine utilisée pour la sélection et en ne retenant que les clones ayant subi une double recombinaison homologue.

Les mammifères non-humains peuvent être obtenus de façon telle que l'expression non fonctionelle de la sous-unité alpha6 du nAChR ou l'absence d'expression de cette sous-unité alpha6 est constitutive, l'animal étant muté pour le gène fonctionnel ou incapable d'exprimer son produit de transcription.

Sont également décrits des mammifères non-humains, caractérisés en ce que la mutation du gène codant pour la sous-unité alpha6 du nAChR est de type inductible ou de type condidonnel.

Le caractère inductible ou conditionnel de la mutation peut être utile notamment pour que l'animal mutant par induction, pour l'expression sous forme fonctionnelle du gène de la sous-unité alpha6, ne soit pas en situation de subir des adaptations comportementales par exemple par compensation et de ce fait, de modifier son comportement vis-à-vis d'agonistes ou d'antagonistes, et de façon générale, de toute substance susceptible d'interagir normalement avec la sous-unité alpha6 native du nAChR.

Des mammifères chez lesquels la mutation en question est inductible peuvent être obtenus notamment en ayant recours à des techniques de recombinaison spécifiques, permettant à la recombinaison destinée à établir la délétion ou la mutation du gène, de se réaliser sous le contrôle de certains agents notamment la tétracycline ou le RU486 (Kellendonk C. et al, J. Mol. Biol, 1999, "285(1): p. 175-82), par exemple dans certaines populations cellulaires neuronales.

L'inactivation génique inductible repose sur l'utilisation d'un promoteur inductible (par exemple le système TRE/rtta (Gossen M. et al, Science, 1995. 268: p. 1766-9) qui contrôle l'expression d'une enzyme, la Cre-recombinase. Dans ce système, on génère des animaux transgéniques exprimant à la fois l'activateur synthétique rtta sous contrôle d'un promoteur spécifique de certains tissus, et la Cre-recombinase sous contrôle du promoteur inductible TRE. Ainsi, en présence de la tétracycline, l'activateur rtta se fixe au promoteur TRE et induit l'expression de la Cre-recombinase dans les seules cellule ou rtta est exprimé. En parallèle, on obtient, via une recombinaison homologue, des animaux chez lesquels le gène codant pour la sous-unité alpha6 est flanqué de deux sites LoxP. Ces sites sont la cible de la Cre-recombinase qui provoque l'excision de tout fragment d'ADN situé entre deux sites LoxP. En croisant ces animaux, on obtient des descendants chez lesquels on peut induire l'excision du gène alpha6 par simple adjonction de tétracycline à l'eau de boisson.

Les sites LoxP en question peuvent, alternativement être insérés dans le gène de façon à encadrer un fragment du gène dont la délétion est suffisante pour inactiver le gène ou conduire à l'expression d'une sous-unité alpha6 non fonctionnelle.

D'autres systèmes équivalents peuvent être utilisés.

Les mammifères non-humains en particulier les rongeurs et notamment les souris, peuvent être obtenus par croisement d'animaux portant les constructions génétiques nécessaires pour obtenir la mutation recherchée du gène codant pour la sous-unité alpha6 du nAChR et ce, de façon à rendre homozygote ladite mutation. La demande rapporte également les animaux de la descendance des susdits animaux.

La demande vise aussi des mammifères non-humains knockout, chez lesquels le gène de la sous-unité alpha 6 est muté de façon hétérozygote Ces animaux montant du fait de cette mutation hétérozygote une sensibilité diminuée aux ligands de la sous-uité alpha6 et sont donc intéressants pour confirmer que la diminution d'un effet, agoniste ou antagoniste vis-à-vis de la sous-unité sipha6, observé avec une substance est effectivement attribuable à la mutation.

Différentes constructions ont été réalisées pour rendre possible la préparation des animaux selon l'invention. Ces constructions sont décrites en détails dans les exemples qui suivent et en particulier la demande vise un mammifère non-humain et notamment une souris, caractérisé en ce que son gène codant pour la sous-unité alpha6 du nAChR est muté par recombinaison homologue avec la séquence désignée a6KO contenue dans la souche E. coli déposée à la CNCM sous le numéro I-2550, le 29 août 2000.

La demande décrit par ailleurs des populations cellulaires et en particulier des cultures cellulaires telles qu'elles peuvent être obtenues à partir d'un mammifère non-humain tel que décrit dans ce qui précède.

Ces populations cellulaires sont notamment les populations neuronales et en particulier les populations cellulaires qui expriment normalement la sous-unité alpha6 des recepteurs nAChR tels que les neurones catécholaminergiques. Des populations cellulaires intéressantes sont également par exemple les cellules neuronales mésencéphaliques.

La demande concerne aussi des préparations de synaptosomes striataux.

Une utilisation particulièrement intéressante de l'invention, est destinée à l'étude des effets de drogues à dépendance sur les récepteurs nicotiniques de l'acétylcholine, ces drogues pouvant être déjà connues et identifiées comme telles en général ou étant connues sans que leurs propriétés soient identifiées, ou encore étant nouvelles dans leur structure et leurs fonctions.

En particulier, l'utilisation des animaux ou des préparations de synaptosomes, ou des cultures cellulaires dérivées permet, par exemple par la mesure du niveau de libération de dopamine, de déterminer l'effet d'agonistes de récepteurs nicotiniques de l'acétylcholine (nAChR), en particulier sur la sous-unité alpha6 de ces récepteurs.

De façon particulièrement intéressante, les animaux, les préparations de synoptosomes, ou les cultures cellulaires sont utilisables dans des protocoles de criblage, notamment pharmacologique, de substances agonistes ou antagonistes des nAChR.

La spécificité d'une substance testée, vis à vis d'un récepteur nAChR, peut être caractérisée par l'observation, pour la substance, d'une forte activité (c'est à dire d'une activité de l'ordre de celle obtenue avec la nicotine, ou plus élevée que celle-ci) sur une souris ou un autre mammifère sauvage et l'absence d'activité sur une souris ou un autre mammifère selon l'invention

De façon plus spécifique, les animaux knockout ou les cultures cellulaires, ou les préparations de synaptosomes permettent d'identifier, parmi les substances agonistes ou antagonistes des récepteurs nicotiniques de l'acétylcholine, celles qui peuvent avoir un effet restreint aux neurones catécholaminergiques notamment restreint aux neurones dopaminergiques.

Les substances testées sont des substances connues, par exemple pour leurs propriétés agonistes ou antagonistes vis-à-vis de récepteurs et en particulier vis-a-vis des récepteur nAChR. Il peut s'agir également de substances nouvelles dont l'invention permettrait d'identifier certaines propriétés en particulier certaines caractéristiques pharmacologiques vis-à-vis des récepteurs nAChR.

L'invention concerne un procédé de criblage de substances susceptibles d'interagir avec la sous-unité alpha6 du nAchR, comprenant :
- la mise en contact de la substance sélectionnée avec un mammifère non-humain tel que défini ci-dessus, dans des conditions permettant l'interaction entre ladite substance et les récepteurs nicotiniques de l'acétylcholine,
- la détection de l'interaction de type liand/récepteur.

L'invention a donc pour objet un procédé de criblage de substances susceptibles d'interagir avec la sous-unite alpha6 du nAChR, sélectionnées parmi les substances interagissant avec les récepteurs nAChR, comprenant:
- la mise en contact de la substance sélectionnée avec un mammifère non-humain tel que défini ci-dessus, dans des conditions permettant l'interaction entre ladite substance et les récepteurs nicotiniques de l'acétylcholine,
- la détection du niveau de la dopamine libérée dans le striatum.

L'invention a également pour objet un procédé de criblage de substances susceptibles d'interagir avec la sous-unité alpha6 du nAChR, sélectionnées parmi les substances interagissant avec les récepteurs nAChR, comprenant :
- la mise en contact de la substance sélectionnée avec une culture cellulaire telle que décrite ci-dessus, dans des conditions permettant l'interaction entre ladite substance et les récepteurs nicotiniques de l'acétylcholine,
- la détection du niveau de la dopamine libérée.

L'invention a également pour objet un procédé de criblage de substances susceptibles d'interagir avec la sous-unité alpha6 du nAChRn sélectionnées parmi les substances interagissant avec les récepteurs nAChR, comprenant:
- la mise en contact de la substance sélectionnée avec une préparation de synaptosomes telle que décrite ci-dessus, dans des conditions permettant l'interaction entre ladite substance et les récepteurs nicotiniques de l'acétylcholine,
- la détection du niveau de la dopamine libérée.

D'autres procédés pour détecter l'interaction des substances criblées avec la sous-unité alpha6 du nAChR. sont par exemple ceux qui mettent en oeuvre le système du double hybride, tels que décrits dans la demande de brevet WO 99/42612.

La variation du niveau de dopamine détectée peut être évaluée par comparaison du niveau basal de dopamine libérée chez un animal exprimant normalement la sous-unité alpha6 du nAChR, ou libérée dans une préparation de synaptosomes effectuée à partir d'un tel animal, ou dans une culture cellulaire exprimant cette sous-unité, avec le niveau de dopamine libérée, lorsque l'animal, la préparation ou la culture en question sont mis en contact avec la substance testée dans des conditions permettant son interaction avec la sous-unité alpha6.

Selon un autre mode de réalisation de l'invention, les procédés de criblage mettent en oeuvre des substances dont on n'a pas préalablement recherché la capacité à se lier à des nAChR.

Selon un autre mode de réalisation, les cultures cellulaires décrites ci-dessus sont utilisées pour étudier ou identifier, parmi les substances agonistes ou antagonistes des récepteurs nicotiniques de l'acétylcholine, celles qui peuvent avoir un effet neuroprotecteur.

L'invention a donc pour objet un procédé de criblage de substances susceptibles d'interagir avec la sous-unité alpha6 du nAChR, sélectionnées parmi les substances interagissant avec les récepteurs nAChR, comprenant:
- la mise en contact de la substance sélectionnée avec une culture cellulaire telle que décrite ci-dessus, dans des conditions permettant l'interaction entre ladite substance et les récepteurs nicotiniques de l'acétylcholine,
- l'application d'une agression à cette culture cellulaire,
- l'observation de l'effet neuroprotecteur de cette substance sur cette culture vis-à-vis de cette agression.

L'agression d'une culture cellulaire est par exemple décrite dans la publication de Akaike A. et al (Brain Res., 1994. 644(2) : p. 181-7). L'effet de cette agression peut être la mort cellulaire. Dans ce cas, l'observation de l'effet neuroprotecteur peut consister en la comparaison de la mort cellulaire engendrée par cette agression sur une culture exprimant la sous-unité alpha6 avec celle engendrée par cette agression sur une culture cellulaire décrite ci-dessus, ces cultures cellulaires ayant été préalablement mises en contact avec la substance criblée.

Le procédé de l'invention ainsi défini, qui peut être mis en oeuvre pour les utilisations qui ont précédemment été décrites de l'invention, permet notamment d'identifier des substances ayant une affinité pour la sous-unité alpha6, et interagissant dans la transmission nicotinique et la transmission dopaminergique de façon sélective par fixation aux récepteurs comportant la sous-unité alpha6 par le biais de cette sous-unité. Ce procédé est particulièrement intéressant pour étudier l'interaction des drogues à dépendance avec la sous-unité alpha6 du nAchR et en particulier pour étudier leur sélectivité vis-à-vis de populations neuronales identifiées.

La détection de la variation éventuelle du niveau de la dopamine libérée dans le striatum d'un animal tel que rapporté ci-dessus peut être faite par des techniques connues et notamment par les techniques décrites dans les exemples qui suivent.

Par exemple, on détectera à l'issue de la mise en oeuvre du procédé, la variation, en particulier l'augmentation, du niveau de dopamine libérée, par rapport au niveau basal de dopamine, détecté chez un animal exprimant normalement la sous-unité alpha6 du récepteur nAChR.

Lorsque la mutation de la sous-unité alpha6 est conditionnelle ou inductible, le niveau basal de dopamine libérée recherché peut être détecté préalablement à l'induction de la mutation et comparé ultérieurement avec le niveau de dopamine chez le même animal chez lequel la mutation aura été induite.

Dans ce cas, la mesure du niveau de dopamine libérée est effectuée par microdialyse (Marshall D. et al., J. Neurochem, 1997. 68(4) : p. 1511-9).

Dans un mode de réalisation préféré du procédé selon l'invention, les substances testées sont des substances dont on recherche l'affinité sélective pour les récepteurs nicotiniques de l'acétylcholine restreints aux neurones dopaminergiques.

Selon un mode de réalisation préféré du procédé de l'invention, les substances testées sont des substances ayant une affinité pour les récepteurs nAChR comportant une sous-unité alpha6 dont on recherche la selectivité vis-à-vis de cette sous-unité, le procédé comprenant :
- a) la mise en contact de la substance sélectionnée avec un mammifère non-humain défini ci-dessus, dans des conditions permettant l'interaction entre ladite substance et les récepteurs nicotiniques de l'acétylcholine,
- b) la détection du niveau de dopamine libérée dans le striatum,
- c) la comparaison du niveau de dopamine libérée dans le striatum à l'étape b), avec le niveau de dopamine libérée dans le striatum de mammifère non-humain, exprimant la sous-unité alpha6 du nAChR.

Selon un autre mode de réalisation, ce procédé comprend :
- a) la mise en contact de la substance sélectionnée avec une préparation de synaptosomes définie dans la demande, dans des conditions permettant l'interaction entre ladite substance et les récepteurs nicotiniques de l'acétylcholine,
- b) la détection du niveau de dopamine libérée,
- c) la comparaison du niveau de dopamine libérée détecté à l'étape b) avec le niveau de dopamine libérée dans une préparation de synaptosomes de mammifère non-humain exprimant la sous-unité alpha6 du nAChR.

Selon un autre mode de réalisation, ce procédé comprend :
- a) la mise en contact de la substance sélectionnée avec une culture cellulaire définie dans la demande, dans des conditions permettant l'interaction, entre ladite substance et les récepteurs nicotoniques de l'acétylcholine,
- b) la détection du niveau de dopamine libérée.
- c) la comparaison du niveau de dopamine libérée détecté à l'étape b) avec le niveau de dopamine libérée dans une culture cellulaire exprimant la sous-unité alpha6 du nAChR.

Les étapes b) et c) des susdits procédés peuvent être remplacées par toute étape permettant la détection de l'interaction ligand/récepteur.

Dans un mode de réalisation particulier des procédés de criblage selon l'invention, les mammifères non-humains, les préparations de synaptosomes, ou les cultures cellulaires sont des contrôles de l'interaction de substances sélectionnées, avec la sous-unité alpha6 des récepteurs nAChR et ces procédés comprennent une étape dans laquelle l'effet de la substance testée sur la sous-unité alpha6 est déterminé sur un animal de type sauvage pour l'expression de ladite sous-unité alpha6 ou sur une préparation de synaptosomes d'un tel animal ou sur une culture cellulaire exprimant normalement cette sous-unité.

L'invention décrit par ailleurs des vecteurs et en particulier des plasmides comportant tout ou partie du gène codant pour la sous-unité alpha6 du récepteur nAChR, notamment des vecteurs portant un gène muté de la sous-unité alpha6, ces vecteurs pouvant constituer notamment des moyens pour la préparation des constructions destinées à la réalisation des animaux selon l'invention.

A cet égard, des plasmides particulièrement intéressants sont ceux sélectionnés parmi les plasmides suivants déposés à la CNCM (Collection Nationale de Culture de Microorganismes, Paris, Frange) :
a6HN : CNCM N° I-2500 déposé le 22 juin 2000
a6EH : CNCM N° 2501 déposé le 22 juin 2000
a6EE : CNCM N° 2502 déposé le 22 juin 2000
a6PP : CNCM N° 2503 déposé le 22 juin 2000
a6KO : CNCM N° 2550 déposé le 29 août 2000,
ou le chromosome artificiel bactérien (BAC) désigné a6BAC, déposé à la CNCM sous le n° I-2504, le 22 juin 2000.

L'invention a également pour objet l'utilisation d'une séquence de nucléotides contenue dans un des plasmides identifiés ci-dessus.

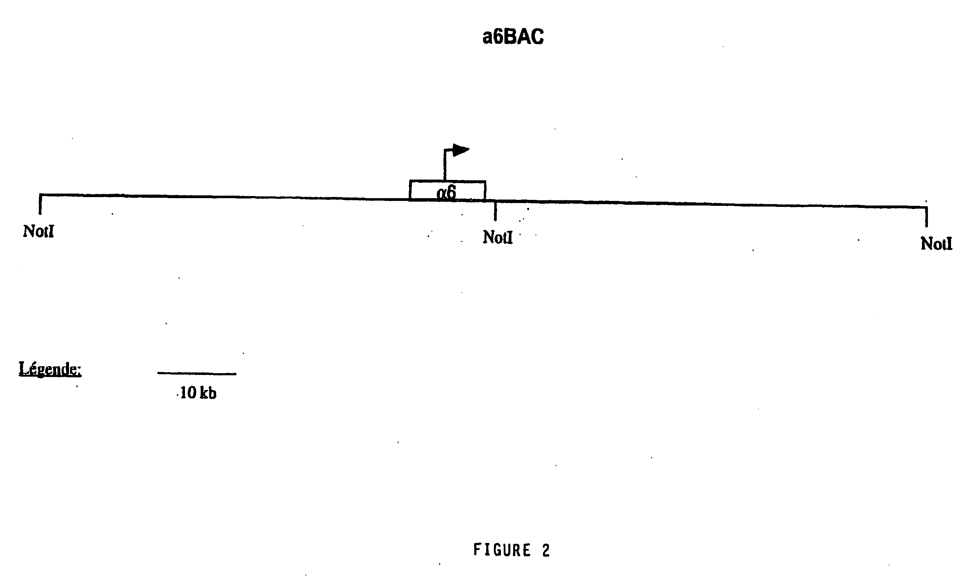

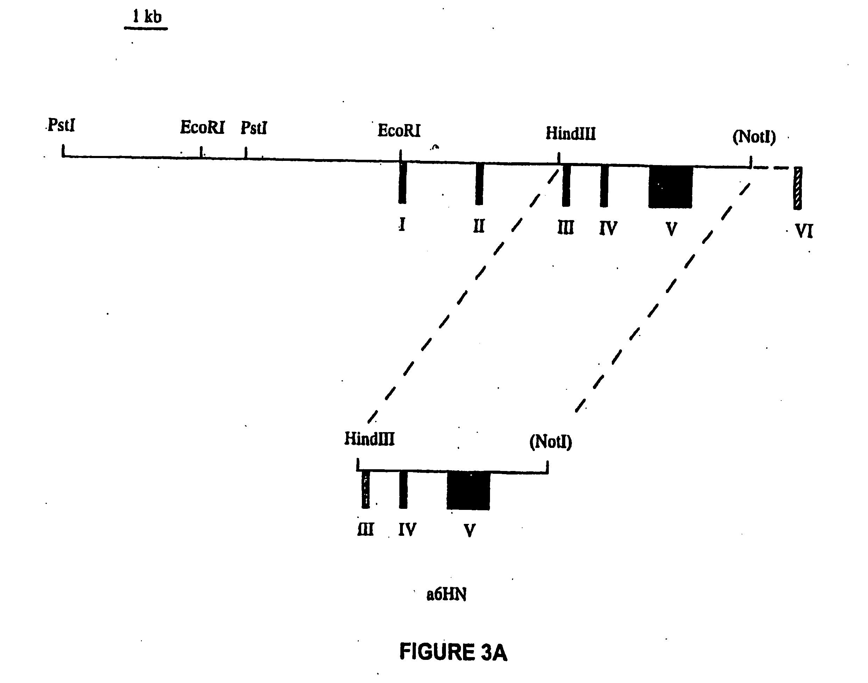

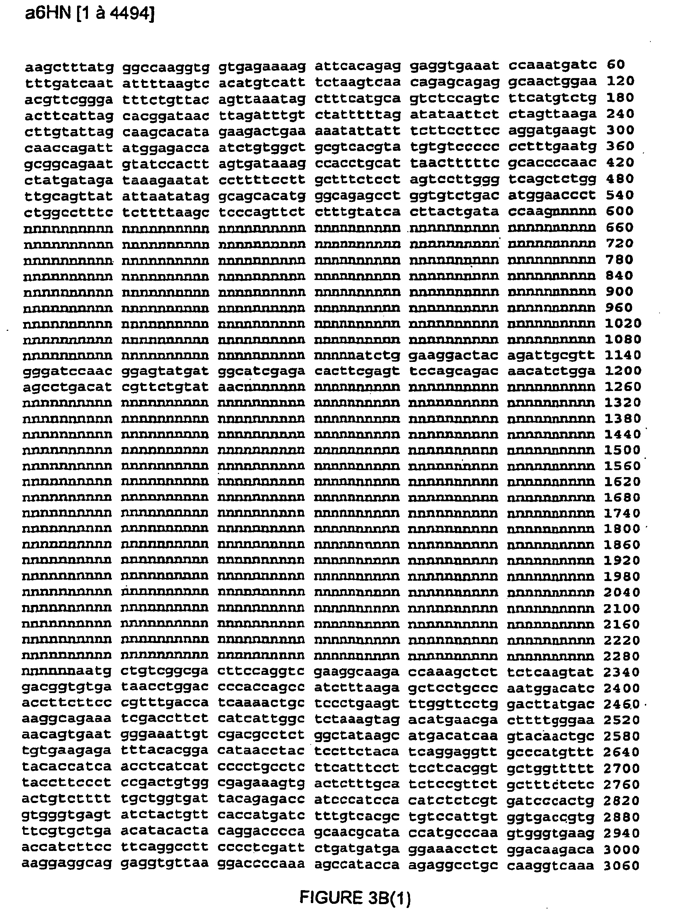

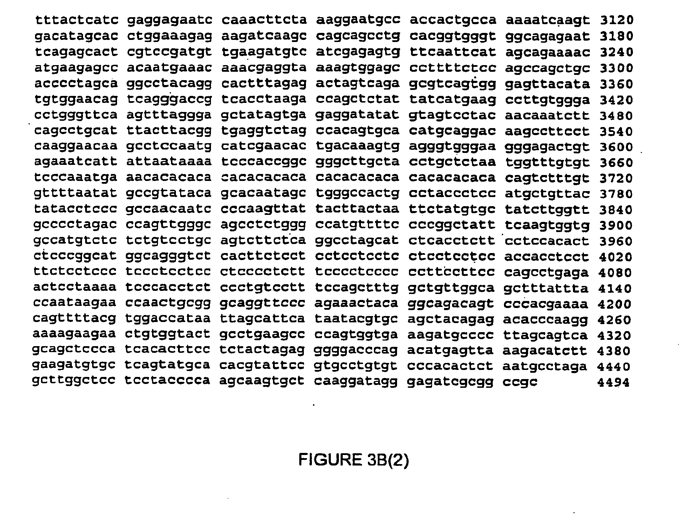

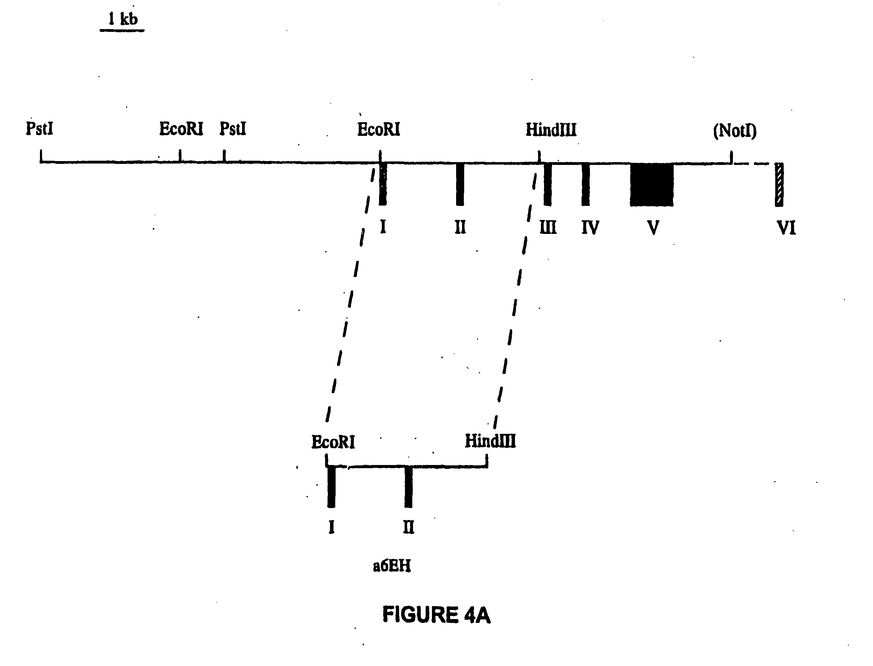

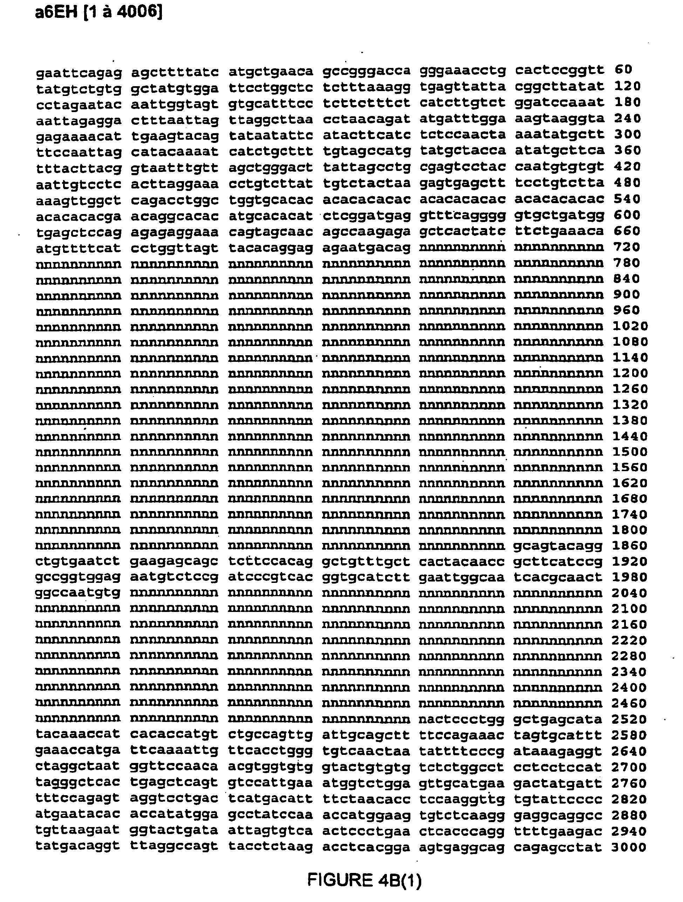

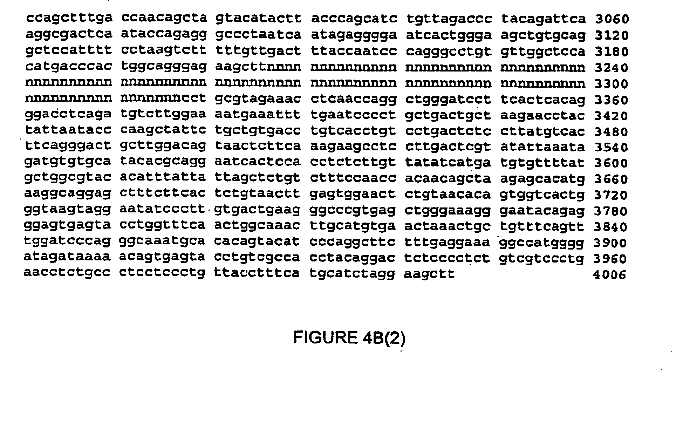

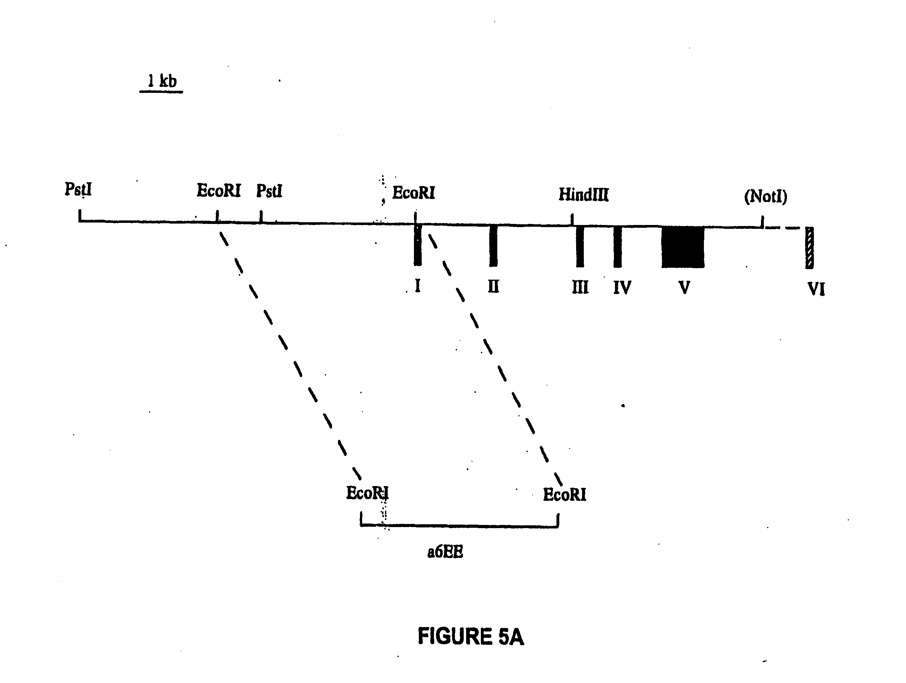

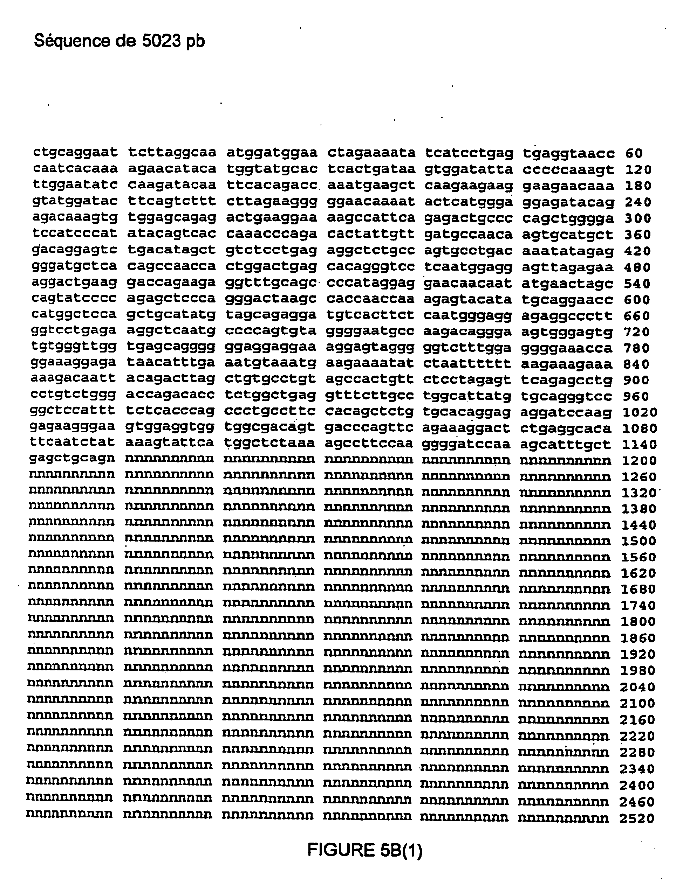

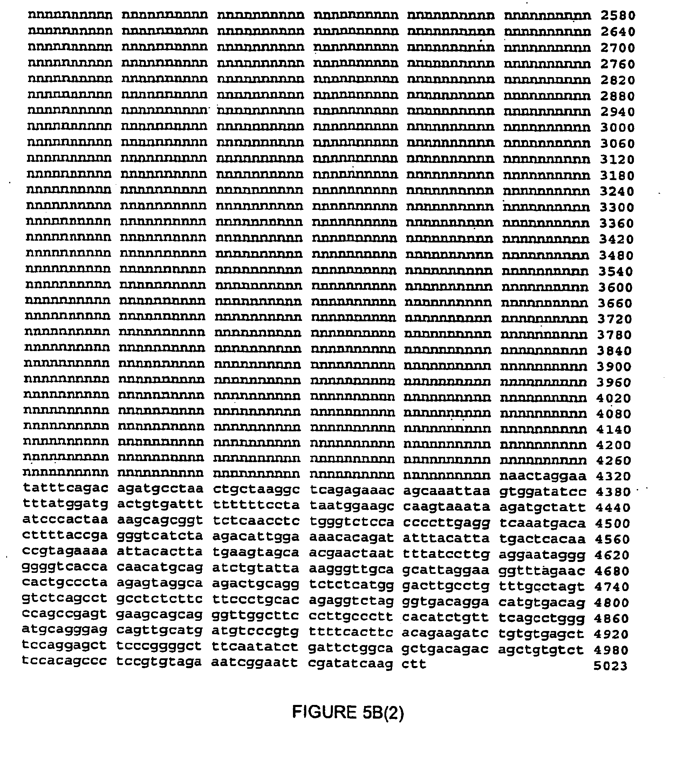

Une séquence de nucléosides préférée est définie en ce qu'elle comprend tout ou perdue des enchaînements représentés dans les figure 2, 3, 4, 5 ou 6 et en particulier les enchaînements séquences de ces figures.

L'invention décrit également les fragments de ces séquences et notamment les exons identifiés du gène codant pour la sous-unité alpha6 du nAChR.

L'invention divulgue par ailleurs des cellules recombinantes comprenant une séquence nucléotidique telle qu'identifiée précédemment ou un vecteur la contenant. Des cellules recombinantes particulièrement intéressantes sont les cellules neuronales et en particulier les neurones dopaminergiques ou les cellules dérivées de la région mésencéphalique du cerveau des animaux définis ci-dessus.

D'autres caractéristiques et propriétés de l'invention apparaissent dans les exemples qui suivent.

### Légende des figures

**Figure 1** Ciblage du gène de la sous-unité alpha6 du récepteur neuronal nAChR.
   a) Structure génomique du gène de la sous-unité alpha6, incluant les exons 1 à 6, ainsi que la structure du vecteur de transfert. Sites de restriction : E, EcoRI ; H, HindIII : S, Slal ; P, Pstl ; N, Notl. Le site Notl indiqué entre parenthèses est dans le phage utilisé pour la construction du vecteur, il est absent de l'allèle de type sauvage. Les fragments EcoRI-EcoRI de 5 kb et HindIII-Notl de 4,6 kb utilisés pour construire les bras d'homologie, sont montrés au-dessus de l'allèle de type sauvage (WT). Le vecteur de recombinaison a été utilisé pour obtenir une mutation par remplacement ; et il contient un gène de résistance à la néomycine (neo') comme marqueur de sélection positif et un gène de la toxine diphérique (DTA) comme marqueur de sélection négatif. Après recombinaison homologue, une délétion de 4 kb incluant les exons 1 et 2 a été formée, Les fragments d'ADN digéré par Pstl, détectés par la sonde Sall-Spel sont indiqués par des lignes en pointillés.
   b) analyse Southern blot identifiant les animaux de type alpha6 homozygotes nuls (-/-), hétérozygotes (+/-) et de type sauvage (+/+).
**Figure 2** **:**
   **A :** Carte de restriction du chromosome artificiel bactérien désigné a6BAC (CNCM I-2504, déposé le 22 juin 2000). Le bac contient environ 130 kb d'insert provenant d'une banque d'ADN de souris de la lignée 129Sv. Cet insert (α6) correspond au locus de la sous-unité alpha6 du récepteur nicotinique de l'acétylcholine (nAchR) de l'exonI à l'exonVI).
   **B :** Séquence de l'insert de 13 kb, orientée dans le sens de la transcription.
**Figure 3** **:**
   **A :** Carte de restriction du plasmide a6HN (CNCM I-2500, déposé le 22 juin 2000).
      Le plasmide a6HN est constitué d'un vecteur KS-Bluescript et d'un insert de 4,6 kb, cloné dans les sites HindIII, Notl, provenant du gène de la sous-unité alpha6 du récepteur nicotinique de l'acétylcholine de souris (ADN provenant de la souche SV/D3 de cellules ES). Ce fragment contient notamment les exons 3 à 5 de la sous-unité alpha6. (Sens de transcription : HindIII vers NotI).
   **B :** Séquence de l'insert de 4,6 kb. Les exons 3, 4 et 5 sont en majuscules. « n » représente des bases non séquencées.
**Figure 4** :
   **A :** Carte de restriction du plasmide a6EH (CNCM I-2501, déposé le 22 juin 2000).
      Le plasmide a6EH est constitué d'un vecteur, SK et d'un insert de 4 kb, cloné dans les sites EcoRI-HindIII. Cet insert est un fragment EcoRI-HindIII du gène de la sous-unité alpha6 du récepteur nicotinique de l'acétylcholine de souris (ADN provenant de la souche 129 SV/D3 de cellules ES). Il contient notamment les deux premiers exons de la sous-unité alpha6. (Sens de transcription : EcoRI vers HindIII).
   **B :** Séquence de l'insert de 4 kb. Les exons 1 et 2 sont en majuscules. « n » représente des bases non séquencées.
**Figure 5** **:**
   **A :** Carte de restriction du plasmide a6EE (CNCM I-2502, déposé le 22 juin 2000).
      Le plasmide a6EE est constitué d'un vecteur, SK-Bluescript et d'un insert de 5 kb, cloné dans le site EcoRI. Cet insert est un fragment EcoRI-EcoRI du gène de la sous-unité alpha6 du récepteur nicotinique de l'acétylcholine de souris (ADN provenant de la souche 129 SV/D3 de cellules ES). Il contient 5 kb de séquence non codante, immédiatement en amont de l'ATG d'alpha6. Ces 5 kb de promoteur sont orientés dans le sens Sacl-Kpnl de SK.
   **B :** Séquence du fragment de 5 kb du promoteur de la sous-unité alpha 6 du nAChR. Les sites de restriction apparaissent en majuscules. La séquence (EcoRI-EcoRI) est orientée de 5' vers 3' dans le sens de la transcription. « n » représente des bases non séquencées.
**Figure 6** **:**
   **A :** Carte de restriction du plasmide a6PP (CNCM I-2503, déposé le 22 juin 2000).
      Le plasmide a6PP est constitué par l'insertion d'un fragment Pstl-Pstl de 4,5 kb de la sous-unité alpha6 du récepteur nicotinique de l'acétylcholine de souris (ADN provenant de la souche 129 SV/D3 de cellules ES), dans le site PstI du vecteur SK-Bluescript. Ces 4,5 kb de séquence appartiennent à la région promotrice d'α6. L'extrémité 3' de ce fragment se trouve à 3,9 kb en 5' de l'ATG du gène α6.
   **B :** Séquence du fragment de 4,5 kb, orientée dans le sens de la transcription. « n » représente des bases non séquencées.
**Figure 7** **:**
   **A :** Carte de restriction du plasmide a6KO (CNCM I-2550, déposé le 29 août 2000) contenu dans des bactéries *E. coli* de la souche DH5α. Le plasmide est utilisé pour la recombinaison homologue dans le locus de la sous-unité alpha6 du récepteur nicotinique de souris. Ce plasmide est constitué d'un vecteur SK-Bluescript et d'un insert de 12 kb. Cet insert contient deux bras d'homologie appartenant à la séquence du gène alpha6 de souris (4 kb et 5 kb) flanquant une cassette de résistance à la néomycine (3,3 kb) (Neo^{r}). Une cassette d'expression de la toxine diphtérique (DTA) est également incluse (1,4 kb).

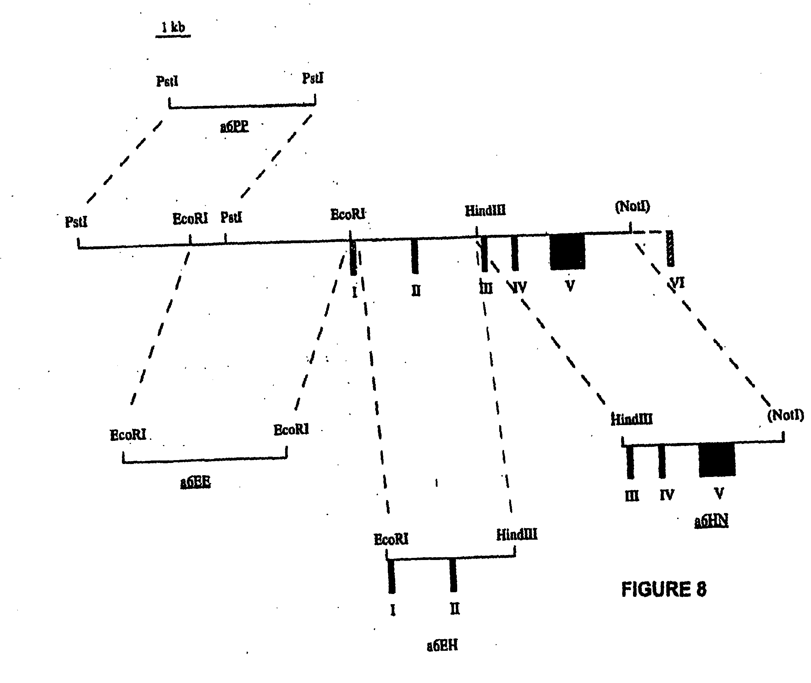

**Figure 8** **:** Localisation des différents fragments de restriction a6PP, a6EE, a6EH, a6HN.

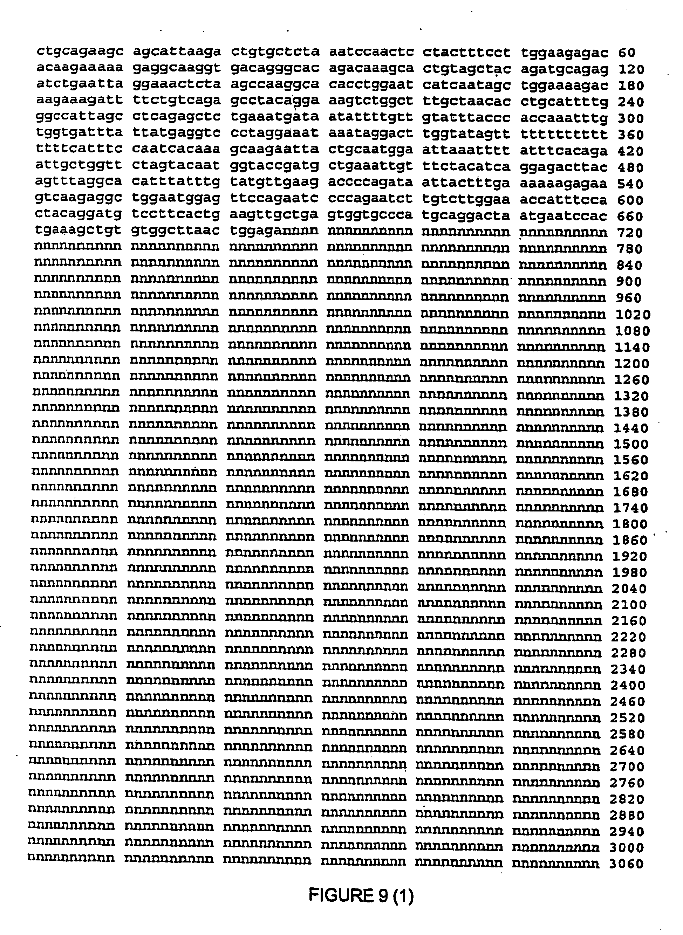

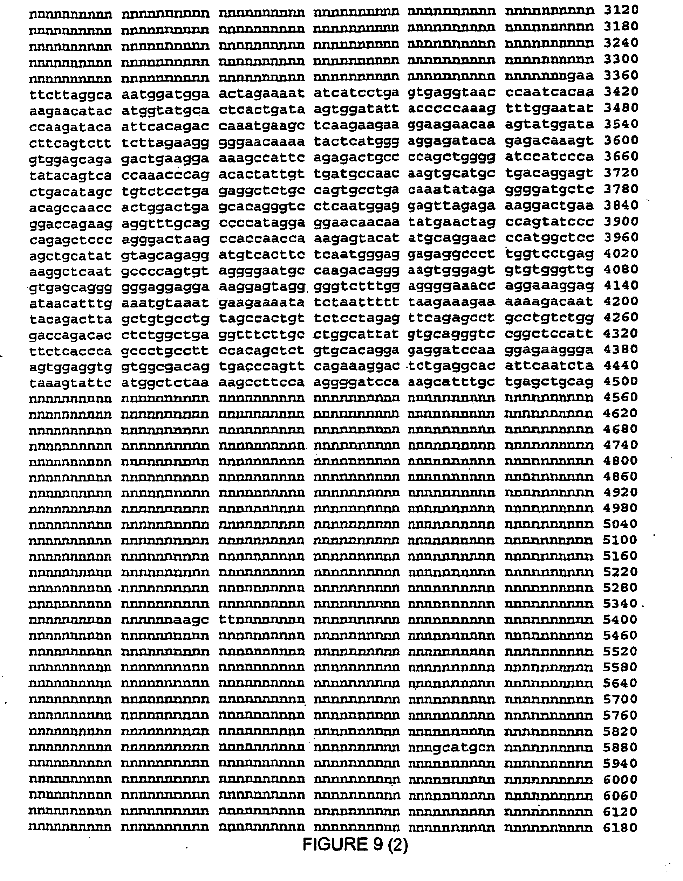

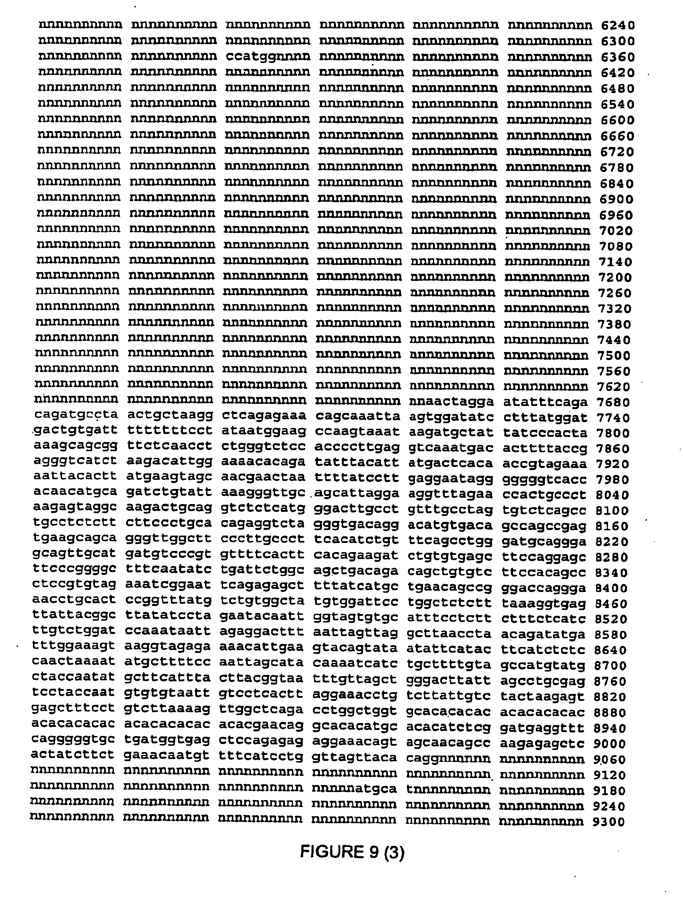

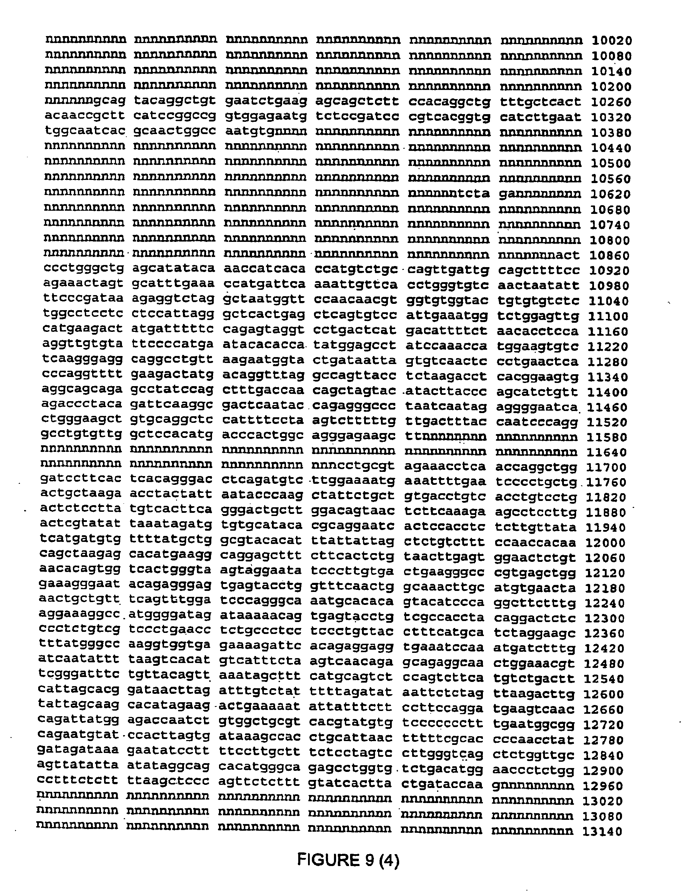

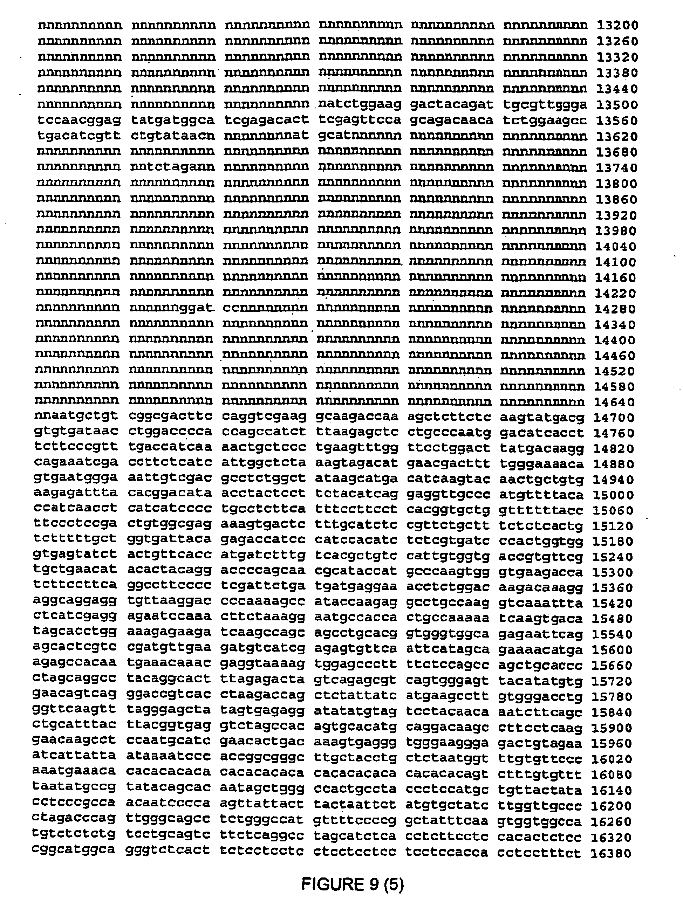

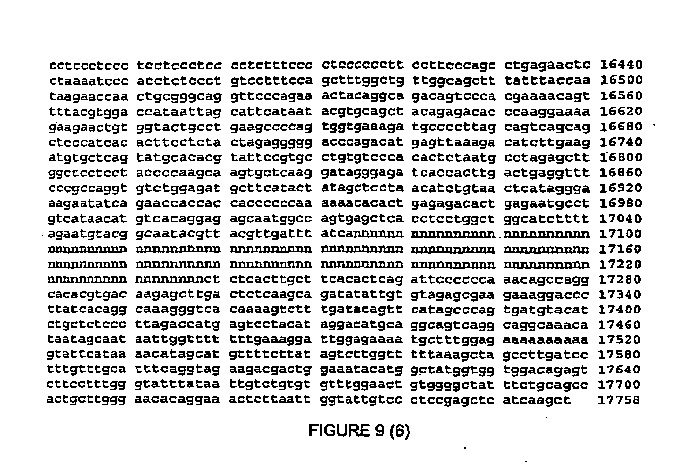

**Figure 9****:** Séquence du locus alpha6 de la sous-unité alpha6 de récepteur nAChR. Les exons sont en lettres capitales
**Figure 10 :** Hybridation *in situ* au moyen d'une sonde oligonucleotidique située dans l'exon6 de la sous-unité a6. On a noté l'expression dans la substance noire et l'aire tegmentale ventrale (SN/VTA) ainsi que dans le locus coeruleus (LC). Dans les animaux knockout, le signal disparaissait entièrement.
**Figure 11 :** Quantification des autoradiographies obtenues à partir d'hybridation *in situ* contre les sous-unités α3, α4, α5, α7, β2 et β4 du récepteur nicotinique. Cx = Cortex ; Th = Thalamus ; MHb = medial habenula ; IPN = interpeduncular nucleus ; Hp = Hippocampe.
**Figure 12 :** Marquage des projections rétinogéniculées au moyen de toxine cholérique fluorescente. Sections coronales représentatives des noyaux genouillés gauche (a,c) et droit (b,d) d'animaux Wt (a,b) et a6-/- (c,d). Les deux yeux ont été injectés avec de la toxine cholérique couplée à 2 fluorophores différents (Rouge pour l'oeil gauche, vert pour l'oeil droit). On a noté l'absence de superposition des marquages, caractéristique d'une ségrégation anormale. [Echelle : 350 µm]
**Figure 13 :** Autoradiographie de la liaison d'αCtxMII sur des coupes de cerveaux d'animaux Wt et mutants. On a noté la présence de sites de liaison dans 3 grands systèmes anatomiques : Le système visuel (dorsolateral geniculate nucleus (dIGN), ventrolateral geniculate nucleus (VLGN), superior colliculus (SC), olivary pretectal nucleus (OPN), optic tract (opt)), le système dopaminergique mésolimbique (Substance noire (SN), aire tegmentale ventrale (VTA), noyau accumbens (Nacc), striatum (Str)) et le système habenulo interpédunculaire (intepeduncular nucleus (IPN) et habenula médiale (MHb)).
**Figure 14:** Déplacement de la liaison de d'épibatidine par l'αCtxMII sur des préparations de membranes de striatum.
**Figure 15 :** Libération de dopamine induite par l'application de 3 µM nicotine sur des synaptosomes striataux en l'absence (a) ou en présence (b) de 100 nM αCtxMII. Courbe dose-réponse de la libération de dopamine en réponse à la nicotine chez les animaux α6-/- (c) ou α4-/- (d). La réponse maximale a été normalisée à 1. On a noté la perte d'affinité d'un ordre de grandeur chez les animaux α4-/-.

### EXEMPLES :

### MATERIEL ET METHODE

Préparation de souris « knockout » (souris mutantes pour le gène codant pour la sous-unité alpha6 des nAChR, l'expression sous forme fonctionnelle de la sous-unité alpha6 du récepteur nAChR étant altérée).

La plupart des protocoles, utilisés pour la préparation des souris knock-out sont décrits dans Laboratory Protocols for Conditional Gene Targeting..., Torres R. et Kühn R., édition Oxford University Press (1997).

### I.1. Construction du vecteur de recombinaison:

Une stratégie basée sur la PCR (Israel, D., A PCR Based method for high stringency screening of DNA libraries. Nucleic Acids Res., 1993. 21 (11) :p. 2627-31) a été utilisée pour cribler une banque d'ADN génomique de souris préparée à partir d'une souche de cellules embryonnaires (cellules ES) de la lignée 129SV/D3. D'autres souris disponibles commercialement (Janvier, Charles River) peuvent être utilisées. Quoi qu'il en soit, il est préférable d'utiliser la même lignée que celle dont dérivent les cellules ES utilisées pour la recombinaison homologue. Des oligonucléotides ont été choisis dans la séquence de l'ADNc de la sous-unité alpha6 du rat.
Amorce amont (Forward primer) :
5'ACTGCTGTGAAGAGATTTACACA3'
Amorce reverse (Reverse primer) :
5'AAGATGGTCTTCACCCACTTG3'
Ce criblage a permis d'isoler un phage contenant approximativement 17 kb du gène de la sous-unité alpha6 du récepteur nitcotinique de l'acétylcholine (α6 nAChR).
Une cartographie de restriction et un séquençage partiel ont permis de déterminer la position des cinq premiers exons. Le sixième exon était en dehors de la séquence clonée (voir figure 1). Un vecteur de recombinaison, du type de remplacement, a ensuite été construit afin d'introduire une délétion de 4 kb dans le locus de la sous-unité alpha6, de façon à supprimer les exons 1 et 2. Pour préparer le vecteur de recombinaison, le fragment HindIII-NotI de 4,6 kb et le fragment EcoRI-EcoRI de 5 kb ont été sous-clonés dans des plasmides KS-Bluescript de façon à générer des plasmides a6HN et a6EE(KS) respectivement. Pour apporter de nouveaux sites de restriction, le fragment Spel-Xhol du plasmide a6EE a ensuite été sous-cloné dans un vecteur bluescript modifié, dont le polylinker avait été remplacé par Sacl-Sfil-Apal-Spel-Xhol-Xbal-Notl, pour constituer le plasmide a6EE(NS). Pendant ce temps, une cassette d'expression Neo^{r} a été introduite dans les sites Xhol-Hindlll de a6HN. Le fragment Xhol-Notl du plasmide résultant a ensuite été sous-cloné dans les sites Sal1-Not1 de a6EE(NS). Finalement, une cassette d'expression de la toxine diphtérique a été introduite dans le site Sall (de l'exon 5 de la sous-unité alpha6)-Notl de ce plasmide, réduisant ainsi la longueur d'homologie du bras droit à 2,5 kb.

### 1.2 Transfection

10⁷ cellules souches embryonnaires pluripotentes (cellules ES) dérivées de la lignée de souris 129sv/Pas (Kress C. et al, « Nonpermissiveness for mouse embryonic stem (ES) cell derivation circumvented by a single backcross to 129/Sv strain : establishment of ES cell lines bearing the Omd conditional lethal mutation ». Mamm Genome, 1998. 9(12) : : p. 998-1001) ont été électroporés avec 20 µg d'ADN de vecteur de recombinaison linéarisé avec Sca I, en utilisant un appareil d'électroporation Bio-Rad (250V, 500 µF). Après 8 jours de sélection dans un milieu contenant l'antibiotique G418 (Gibco-BRL) (400 µg/ml), 200 colonies résistantes à l'antibiotique ont été prélevées et dissociées en trypsine pour être réparties sur des plaques de 96 puits. Après 2 jours de croissance, chaque clone était dissocié en trypsine et réparti sur deux puits d'une plaque de 48 puits. Le contenu de l'un de ces puits a été utilisé pour congélation alors que le contenu de l'autre a été réparti sur une plaque à 24 puits pour la préparation de l'ADN génomique.

### 1.3. Criblage des clones de cellules ES recombinantes par Southern génomique

### a) Préparation de l'ADN

Des puits à confluence de la plaque de 96 puits ont été lavés avec du PBS et incubés à 37°C pendant 12 heures avec 500 µl de tampon de lyse cellulaire (100mM Tris-HCl pH8: 5mM EDTA; 0,2% SDS; 200 mM NaCl, 100 µg/ml proteinase K). L'ADN génomique a ensuite été récupéré avec une précipitation à l'éthanol et resuspendu dans 150 µl d'eau.

### b) Digestion et transfert

50 µl de cette préparation d'ADN génomique ont été utilisés pour la digestion avec 50 unités de Pstl pendant 6 heures à 37°C. Le produit de digestion a ensuite été chargé sur un gel d'agarose à 0,8% et soumis pendant 20 heures à une électrophorèse TAE à 40V. L'ADN digéré a été transféré sur une membrane de nylon (Hybon-N+. Amersham) par transfert Southern dans un tampon 20x SSC. L'accrochage de l'ADN à la membrane a été réalisé par exposition à une lumière UV pendant 5 minutes.

### c) Marquage de la sonde

25 ng de fragment Sall-Spel de 800 pb du gène alpha6 ont été marqués en utilisant un kit d'amorçage aléatoire de Amersham (RPN 1607). Cette sonde est localisée en 3' par rapport au bras droit d'homologie, à l'extérieur de la construction de ciblage.

### d) Hybridation et lavage

Des membranes transférées ont été préhybridéés pendant 30 minutes dans 40 ml de tampon d'hybridation (0,5 M NaHPO₄ (pH 7,6), 7% SDS, 0,1 mg/ml d'ADN de sperme de saumon), à 65°C. L'hybridation a été réalisée dans 10 ml du même tampon contenant 10⁶ cpm/ml de la sonde dénaturée (3 min à 100°C) pendant 15-20 heures à 65°C. Les membranes ont ensuite été lavées deux fois avec 0,1 SSC ; 0,1 % SDS et exposées dans une cassette de Phosphorlmager (Molecular Dynamics) pendant 24 heures, avant révélation.

Cette sonde (fragment Sall-Spel) et cette enzyme de restriction (Pstl) ont permis de détecter deux bandes dans les clones recombinants : une bande de 8 kb correspondant à l'allèle de type sauvage, et une bande de 4 kb correspondant à l'allèle muté.
Les trois clones de cellules ES recombinantes obtenus ont été multipliés et gardés sous forme congelée en petites aliquotes dans de l'azote liquide avant l'utilisation dans l'agrégation.

### 1.4. Préparation de chimères

Les chimères ont été obtenues en utilisant un protocole d'agrégation selon la publication de Khillan J. et Y. Bao, Preparation of animals with a high degree of chimerism by one-step coculture of embryonic stem cells and preimplantation embryos. Biotechniques, 1997. 22(3) p. 544-9), modifié : Une culture pendant une nuit dans un incubateur de culture cellulaire standard au permis à de petites quantités de cellules ES recombinantes d'agréger avec des embryons de préimplantation de type sauvage. Les agrégats s'étant développés sous forme de blastocytes ont été ensuite réimplantés chez des mères porteuses dans un état de pseudo gestation pour préparer des animaux chimères.

### a) Préparation de cellules ES

Deux jours avant l'agrégation, un cryotube contenant 5.10⁶ cellules ES a été utilisé pour ensemencer une boîte de 50 mm contenant du milieu de culture. Le jour suivant, les cellules ont été dissociées et déposées à différentes concentrations sur une plaque de 6 puits (plus particulièrement, des dilutions de 1/3, 1/6, 1/12 et 1/24 ont été déposées sur la plaque de 50 mm initiale pour être utilisées dans les trois jours d'agrégation à venir).

Le jour de l'agrégation, les cellules ES ont été soumises à une dissociation douce (4 min dans la Trypsin) de façon à générer de petits agrégats de cellules ES (4-20). La trypsine a été inhibée par l'addition de milieux contenant du sérum. Après une dissociation douce en utilisant une pipette de 10 ml, les cellules ont été réparties sur une plaque de culture de tissus gélatinisée, et placées dans un incubateur pendant 20 minutes. Cette étape a permis de retirer de façon sélective, les fibroblastes nourriciers et les gros agrégats des cellules ES qui sédimentent plus rapidement. Le surnageant contenant les cellules ES a été récolté et gardé sur la glace pendant la préparation des embryons.

### b) Préparation d'embryons

Cinq jours avant l'agrégation, des souris femelles âgées de 3 semaines (CD1 ou C57BI6 ont été superovulées par injection de 5 unités de PMS suivie 46 heures plus tard, par une injection de 5 unités de HCG (Intervet) puis accouplées avec des souris mâles âgées de deux mois de la lignée correspondante, i.e., CD1 ou C57BI6. Le jour de l'agrégation, (2,5 jours pc), 9 souris superovulées ont été sacrifiées pour récupérer des embryons au stade morules, qui ont été conservées en milieu M2 (Sigma).
On a alors retiré la zone pellucide par une incubation dans une solution de Tyrode (Sigma) : cette étape est réalisée à température ambiante, sous microscope. Dès la disparition de la zone pellucide, les embryons sont transférés dans des gouttes de milieu M2 afin de limiter leur exposition à la solution de Tyrode.

### c) Préparation des plaques d'agrégation

On a placé 6 gouttes (5 µl) de milieu d'agrégation (50 % M16 + 50 % de milieu standard de cellules ES) sur une plaque de culture non traitée de 35 mm. Ces gouttes ont ensuite été couvertes avec de l'huile minérale pour éviter l'évaporation. En utilisant le bout d'une pince de dissection, on a préparé 6-8 petites dépressions (puits d'agrégation) dans chaque goutte.

Chaque puits a reçu un petit agrégat de cellules ES (4-8 cellules pour les embryons de C57BI6 et 8-12 cellules pour les embryons de CD1) et une morule. On a pris soin de maximiser le contact entre les cellules et l'embryon pour permettre l'agrégation. La plaque a ensuite été placée dans un incubateur pendant une nuit.

### d) Réimplantation des agrégats

Le jour suivant l'agrégation, 90% des agrégats s'étaient développés sous forme de blastocytes. Ces blastocytes ont ensuite été réimplantés dans l'utérus de souris B6D2 (2,5 pc) femelles en état de pseudogestation.

### 1.5. Préparation de souris knockout homozygotes

Pour chaque souche (C57BI6 ou CD1), un transmetteur de lignée germinale a été obtenu et croisé avec des souris B6D2/F1 ou CD1 (respectivement) pour une sélection par la couleur du pelage des nouveaux-nés résultants. Les nouveaux-nés ayant une couleur Agouti ont ensuite été analysés par Southern génomique. La mutation a été transmise par croisement, à des souris C57BI/6J pour les expériences ultérieures.

### 2) Libération de dopamine à partir des synaptosomes striataux superfusés

### 2.1. Préparation de synaptosomes

Les striata de deux souris ont été rapidement disséqués sur la glace (poids total humide = 60 mg) et placés dans 10 vol de 0,32 M sucrose / 5 mM HEPES pH7,5 refroidis sur glace. Les striata ont ensuite été homogénéisés dans un homogénéisateur verre/Teflon (15 coups à 900 tours/min). L'homogénat a été centrifugé à 1000g pendant 5 minutes à 4°C pour retirer les noyaux et les débris cellulaires. Le surnageant a ensuite été centrifugé à 12000 g pendant 20 minutes à 4°C. Le culot final, consistant en une préparation synaptosomale brute (P2) est resuspendu dans un tampon de superfusion (6 mg de poids tissulaire initial / ml). Le tampon de superfusion (SB) est composé ainsi (mM) : NaCl 128, kCl 2,4, CaCl₂ 3,2, KH₂PO₄ 1,2, MgSO₄ 0,6, HEPES (pH 7,5) 25, D-Glucose 10, L-acide ascorbique 1 et pargyline 0,1.

### 2.2. Charge des synaptosomes en dopamine tritiée

La préparation de synaptosomes a été incubée avec de la dopamine [³H] (100 nM) pendant 20 min à 37°C puis centrifugée à 12000g pendant 5 minutes. Le culot est resuspendu dans le tampon SB et chargé sur un filtre en fibres de verre A/E de 13 mm de diamètre (Gelman Sciences) sous vide léger. (Les synaptosomes striataux de 2 souris sont généralement suffisants pour 12 filtres).

### 2.3. Superfusion

L'appareil de superfusion est commercialement disponible (Brandel). Il consiste en 6-20 canaux identiques. Chaque canal est constitué d'une chambre de rétention (vol=100µl) contenant les filtres qui portent des synaptosomes. Cette chambre est reliée, via un tube Tygon (diamètre intérieur = 1,14 mm), à une pompe péristaltique multicanaux (Masterflex) et à un dispositif de collecte de l'effluent. Le tampon de superfusion (SB) est pompé de façon continue à travers la chambre, à la vitesse de 500 µl/min.
Après une période de rinçage de 15 minutes, l'effluent a été collecté toutes les minutes, dans des fioles à scintillation contenant 5 ml de scintillant. Après 5 minutes de collecte, la drogue étudiée a été administrée pendant 1 minute et l'effluent collecté sur une nouvelle période de 10 minutes. Enfin, la quantité de dopamine tritiée libérée dans chaque échantillon a été mesurée au moyen d'un compteur à scintillation (Wallac 1214 Rackbeta).

### 2.4. Analyse des données

Le niveau de base de la libération de dopamine a été estimé en ajustant les données des 5 premiers échantillons collectés avant administration de la drogue avec une courbe de décroissance exponentielle (y = a.exp(-bt)). Le pic de libération a alors été défini comme étant le maximum du rapport « 100 x (Dopamine mesurée - ligne de base)/ligne de base » pour les 10 échantillons consécutifs à l'administration de drogue. Cette méthode de calcul permet de s'affranchir des problèmes liés aux différences dans la quantité totale de radioactivité présente sur chaque filtre.

### 3) Caractérisation moléculaire et anatomique des souris knockout pour la sous unité a6 (α6-/-)

### 3.1. Vérification des animaux

L'étude de l'expression de l'ARNm codant pour la sous-unité alpha6 du récepteur nicotinique de l'acétylcholine (nAChR) a permis de confirmer les résultats obtenus chez le rat et le poulet (Le Novere et al., 1996 Eur J Neurosci 8, 2428-39 ; Vailati et al., 1999 Mol Pharmacol 56, 11-19). Chez les animaux de type sauvage (Wt), α6 est exprimée à la fois dans les cellules ganglionnaires de la rétine et les neurones catécholaminergiques (Substantia nigra (SN), ventral tegmental area (VTA) et locus coeruleus (LC)). Dans les animaux α6-/- en revanche, nous avons démontré la disparition totale de l'ARNm de la sous unité α6, confirmant ainsi la fonctionnalité de la délétion effectuée. (Fig. 10).
Les inventeurs ont également vérifié l'absence de compensation dévéloppementale associée à une éventuelle sur-expression d'autres sous unités du nAChR, pour palier la disparition d'α6 chez les animaux mutants. L'hybridation *in situ* au moyen de sondes oligonucléotidiques dirigées contre les sous-unités α3, α4, α5, α7, β2, β4 a permis de démontrer qu'un tel mécanisme n'existait pas chez les animaux α6-/-, tout du moins au niveau transcriptionnel. (Fig. 11).

Sur un plan général, les animaux α6-/- sont viables, présents en proportions mendéliennes dans les portées, grandissent normalement et ne présentent aucun déficit physique ou neurologique majeur.

### 3.2. Absence d'anomalie anatomique dans le système nerveux des α6-/-

Les inventeurs se sont intéressés au développement anatomique des structures neuronales où α6 est normalement exprimée.

### a) Le système visuel

Dans le système visuel, les Inventeurs se sont attachés à l'étude des projections des cellules ganglionnaires de la rétine dans le noyau genouillé dorso-latéral, car l'organisation anatomique de ces projections est connue pour être régulée, au cours du développement, par l'activité de la transmission nicotinique dans la rétine. A la différence de ce qui est observé chez les animaux β2-/- (Rossi et al., 2001 Proc . Natl. Acad. Sci. USA 98, 6453-6458), les mutants α6-/- ne présentent aucune altération du système visuel à ce niveau (Fig. 12).

### b) Le système dopaminergique

Dans le système dopaminergique, les inventeurs ont vérifié l'absence d'anomalie anatomique, tant au niveau des corps cellulaires (dans le mésencéphale) qu'au niveau de leurs projections dans le striatum. Ils ont notamment étudié le profil d'expression de nombreux marqueurs dopaminergiques dont la plupart sont connus pour être influencés par l'administration de nicotine: Ces marqueurs comprennent des enzymes dopaminergiques tyrosine hydroxylase (TH) et transporteur de la dopamine (DAT), les récepteurs de la dopamine D1 et D2 ainsi que les neuropeptides (Cholécystokinine (CCK), Préproenképhaline (PPE) et Préprotakikinine (PPT)). Une fois encore, aucune différence n'a pu être détectée dans le profil d'expression de ces marqueurs dans le cerveau des animaux α6-/-.

### 4) Pharmacologie des récepteurs nicotiniques contenant la sous-unité α6

### 4.1. Profil des liaisons de ligands nicotiniques classiques :

La distribution des sites de liaisons pour divers ligands nicotiniques dans le cerveau des animaux α6-/- a été étudiée. Si la liaison d'[C¹²⁵I]α-bungarotoxine n'est pas modifiée chez les mutants, en revanche, la liaison d'épibatidine, cytisine et nicotine a révélé une diminution du nombre de sites de haute affinité pour ces ligands dans les régions de projection de la rétine (colliculus supérieur (SC) et noyau dorso-latéral du genouillé (dLGN)). En revanche, dans toutes les autres structures, et notamment la SN/VTA et le striatum, aucune autre différence n'a pu être détectée en comparant avec les animaux Wt. Ce résultat, quelque peu surprenant, peut être expliqué par l'abondance de récepteurs nicotiniques de type α4β2 dans ces régions qui pourrait avoir masqué des variations faibles du nombre total de sites.

### 4.2. Liaison d'α-conotoxine MII iodée ([125I]αCtxMII)

Le résultat le plus surprenant de cette étude pharmacologique provient de l'étude de la liaison d'αCtxMII. En effet, cette toxine fut isolée et caractérisée comme étant spécifique des nAChRs contenant une interface α3β2 (Cartier et al., 1996 JBiolChem 271, 7522-8). Cependant, les inventeurs ont démontré dans les animaux α6-/-, une disparition complète de l'ensemble des sites de liaison à haute affinité pour cette toxine (Fig. 13). Par ailleurs, d'autres sites éventuels, dits de basse affinité, ne pouvant être détectés par cette technique d'autoradiographie, le déplacement de la liaison d' [³H]épibatidine par l'αCtxMII non marquée, sur des préparations de membranes de striatum a été étudié. Là encore, les inventeurs ont pu démontrer la disparition totale des sites de haute et basse affinité pour l'αCtxMII (20% du nombre total de sites épibatidine) chez les animaux α6-/-. Un autre résultat est apparu de cette étude : bien que 20% des sites de liaisons pour l'épibatidine soient sensibles à l'αCtxMII et que cette sensiblité disparaisse chez les mutants, les inventeurs n'ont pas pu mettre en évidence de diminution du nombre total de sites épibatidine chez les α6-/-. Il semble en effet que les sites sensibles à l'αCtxMII aient été remplacés chez les mutants par une quantité équivalente de sites résistants (Fig. 14). Le mécanisme de cette compensation reste inconnu même si quelques hypothèses peuvent être émises. Au vu des propres résultats des inventeurs, il est peu vraisemblable que le mécanisme de compensation soit d'origine transcriptionelle. En revanche, (Klink et al., 2001 J Neurosci 21, 1452-63) ont suggéré l'existence de sites du type a4α6(β2)₃ dans les neurones dopaminergiques. En l'absence de la sous unité α6, ces sites ne disparaîtraient pas mais seraient remplacés par des sites du type (α4)₂(β2)₃, insensibles à l'αCtxMII.

### 5) La sous-unité α6 intervient dans la modulation nicotinique de la transmission dopaminergique.

### 5.1. Effet de la nicotine sur la libération de dopamine

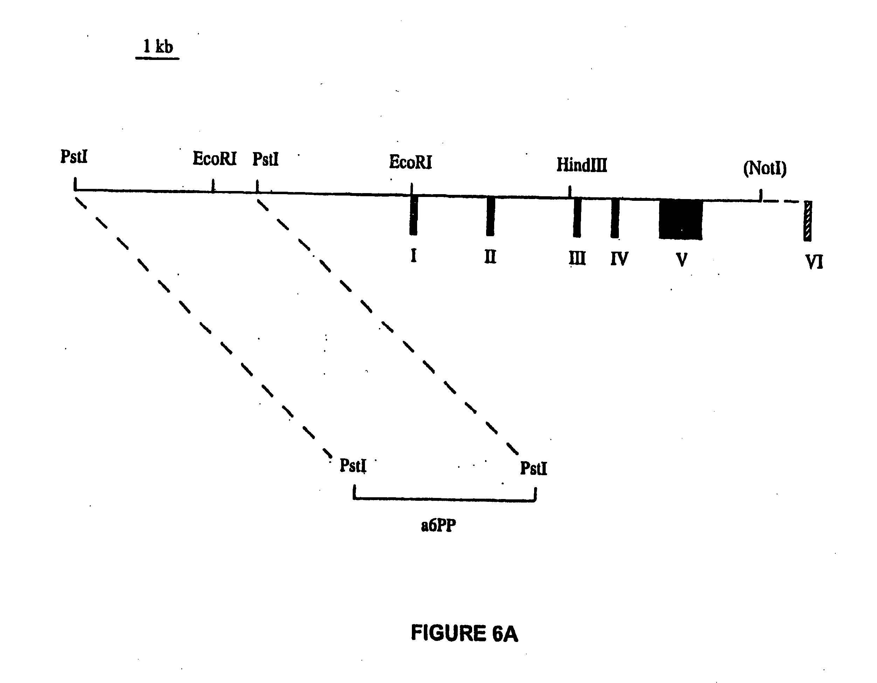

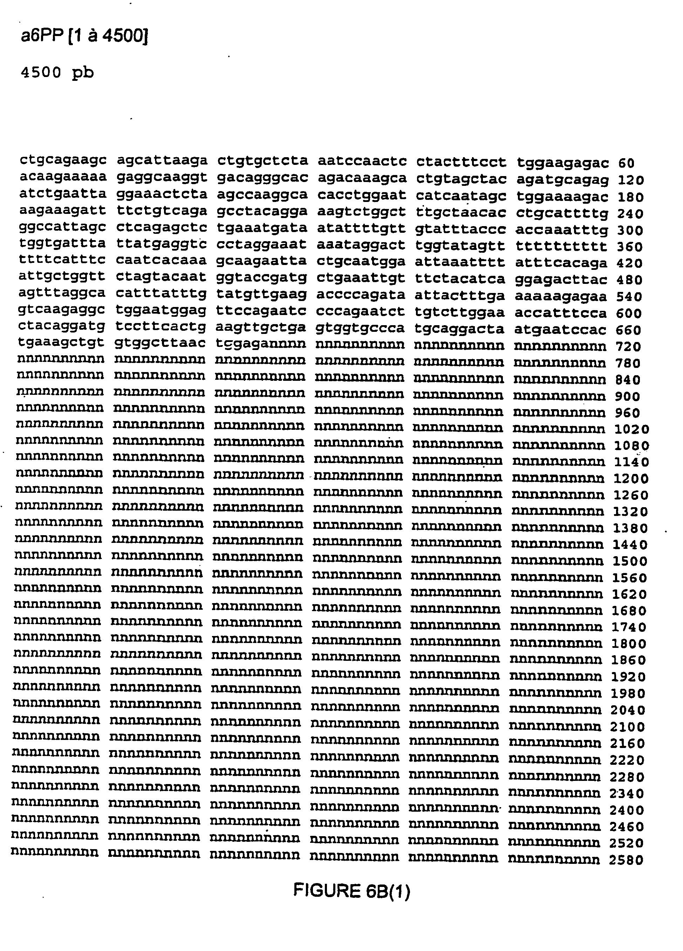

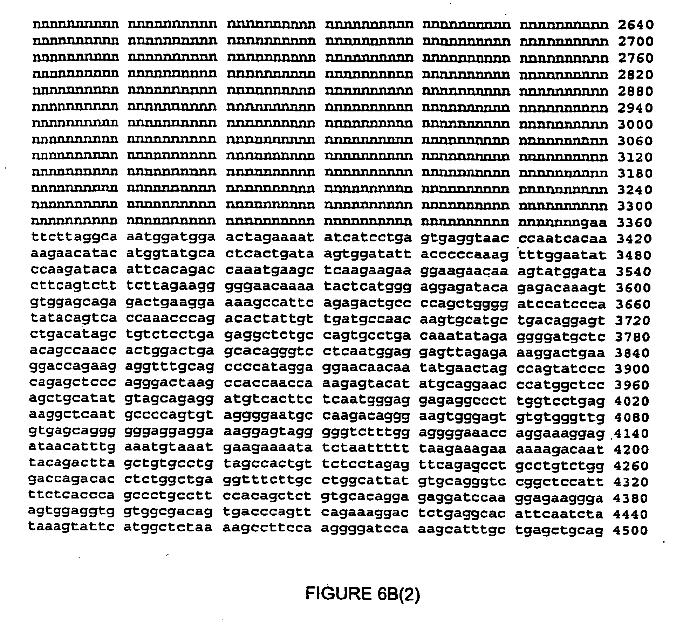

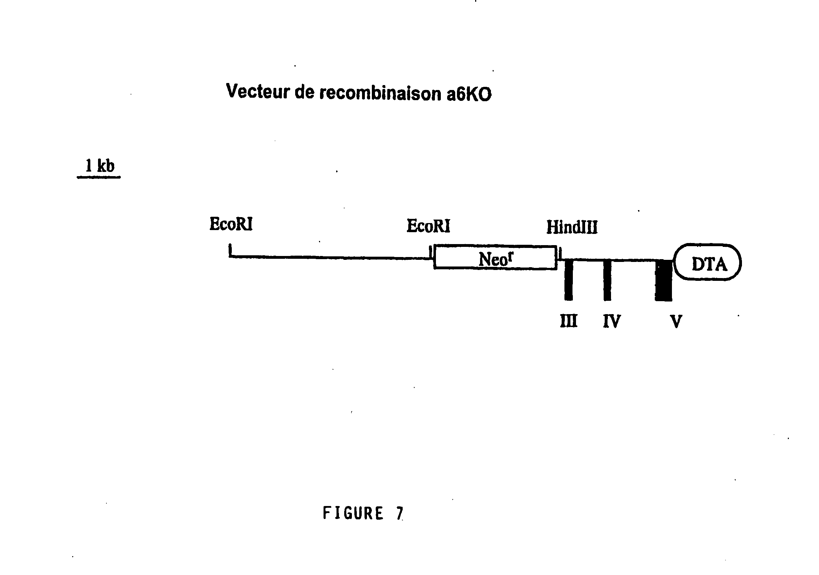

Malgré ces mécanismes de compensation, les inventeurs se sont intéressés à l'étude de la libération de dopamine, induite par la nicotine dans des préparations de synaptosomes striataux. Chez un animal de type sauvage, l'application de nicotine (3µM), induit une augmentation de la libération de dopamine (DA) d'environ 100% au dessus du niveau basal. Chez les animaux α6-/- et α4-/-, ce pic de libération est réduit à 50%. Par ailleurs, chez les doubles mutants α4-/- x α6-/-, ainsi que chez les animaux α2-/-, l'effet de la nicotine est entièrement aboli (Fig. 6a).

### 5.2. Effet de l'αCtxmII sur la libération de dopamine induite par la nicotine

Les inventeurs ont également étudié l'interaction de l'αCtxMII avec la libération de DA induite par la nicotine chez les animaux α4-/-, α6-/- et Wt.

Chez les animaux Wt , l'αCtxMII (100 nM) réduit le pic de libération de DA induit par la nicotine à 50% au-dessus du niveau basal. En revanche chez les animaux α6-/-, la libération de DA induite par la nicotine est complètement insensible à l'αCtxMII, tandis qu'elle est entièrement abolie chez les animaux α4-/- (Fig. 15 a,b).

L'ensemble de ces résultats suggère la présence de 2 types de récepteurs au niveau des terminaisons dopaminergiques dans le striatum, chacun contribuant à 50% de l'effet de la nicotine. Ce sont des récepteurs de type α4β2 insensibles à l'αCtxMII et des récepteurs de type α6β2, bloqués par cette toxine. Cependant, l'examen attentif des courbes dose-réponse chez les knockout α4 et α6 a permis de mettre en évidence un changement d'affinité de la réponse chez les animaux mutants α4-/- (perte d'un ordre de grandeur) (Fig. 15c, d). Au vu des résultats obtenus sur les animaux α6-/-, ceci ne peut s'expliquer que par une modification de la composition des récepteurs nicotiniques impliqués dans la libération de dopamine induite par la nicotine. Ceci a conduit les inventeurs à modifier leur théorie sur la composition des récepteurs nicotiniques présents sur les terminaisons dopaminergiques dans le striatum. Ils proposent deux types de récepteurs (α4)₂(β2)₃ et α6α4(β2)₃ qui contribueraient chacun à 50% de la réponse à la nicotine, et ayant chacun la même affinité pour la nicotine. Ainsi, chez les animaux α6-/-, ne subsisterait que le sous-type (α4)₂(β2)₃ induisant une réponse réduite de 50% insensible à l'αCtxMII. En revanche dans les animaux α4-/-, les inventeurs suggèrent la formation d'un nouveau sous-type (α6)₂(β2)₃ sensible à l'αCtxMII et d'affinité pour la nicotine plus faible (Ce qui permettrait d'expliquer le changement d'affinité de la réponse à la nicotine, observé chez les animaux α4-/-). Ainsi, le mécanisme de compensation décrit chez les animaux α6-/- serait également présent dans les mutants α4-/-. En revanche, la diminution de 50% de la réponse dopaminergiques à l'administration de nicotine chez les α6-/-, indique que la compensation observée chez ces animaux n'est pas fonctionnelle.

### 5.3. Etude de l'expression de cFos en réponse à la nicotine

Les inventeurs se sont intéressés chez les animaux α6-/- à l'expression de gènes dits « précoces », en réponse à la nicotine. Ces gènes sont des facteurs de transcription qui s'expriment dans les cellules en réponse à des stimuli très divers, traduisant une activation « génétique » de la cellule dans laquelle ils s'expriment. Dans le cas de la dépendance aux drogues, on a pu observer une augmentation de l'expression d'un de ces gènes, cFos, en réponse à l'administration de morphine, de cocaïne ou de nicotine, dans les cellules du striatum. Cet effet, bloqué par l'infusion d'antagonistes dopaminergiques dans le striatum, est supposé être responsable des modifications à long terme observées lors de la prise chronique de drogues (Nestler, 2001 Nat Rev Neurosci 2, 119-28). Chez les animaux α6-/-, des résultats préliminaires semblent indiquer une absence d'activation de l'expression de cFos en réponse à la nicotine. Ces résultats demandent cependant à être confirmés sur un plus grand nombre d'animaux et étendus à d'autres gènes précoces.

## Revendications

1. Utilisation d'un mammifère non-humain, porteur d'une mutation dans le gène codant pour la sous-unité alpha6 du récepteur nicotinique de l'acétylcholine (nAChR), ladite mutation empêchant l'expression chez le mammifère de ladite sous-unité alpha6 du nAChR sous une forme fonctionnelle, pour étudier les effets de substances déterminées, susceptibles d'interagir avec la sous-unité alpha6 de nAChR.

2. Utilisation d'un mammifère non-humain selon la revendication 1, **caractérisée en ce que** le gène codant pour la sous-unité alpha6 du nAChR est muté par délétion de tout ou partie de ses exons.

3. Utilisation d'un mammifère non-humain selon la revendication 2, **caractérisée en ce que** le gène codant pour la sous-unité alpha6 du nAChR est muté par délétion d'au moins une partie de l'exon 1 et/ou de l'exon 2, cette délétion étant suffisante pour empêcher, au niveau de la sous-unité alpha6 du nAChR exprimé chez le mammifère, la liaison de ladite sous-unité alpha6 du nAChR avec ses agonistes et/ou ses antagonistes.

4. Utilisation d'un mammifère non-humain selon la revendication 3, **caractérisée en ce que** le gène codant pour la sous-unité alpha6 du nAChR est muté par délétion des exons 1 et 2.

5. Utilisation d'un mammifère non-humain selon l'une quelconque des revendications 1 à 4, **caractérisée en ce que** la mutation du gène codant pour la sous-unité alpha6 du nAChR est homozygote et conduit à l'absence d'expression de la sous-unité alpha6, ledit mammifère ayant un phénotype (α6^{-/-}).

6. Utilisation d'un mammifère non-humain selon l'une quelconque des revendications 1 à 4, **caractérisée en ce que** la mutation du gène codant pour la sous-unité alpha6 du nAChR est homozygote, ledit mammifère ayant un phénotype (α6^{Δ1-2/Δ1-2}).

7. Utilisation d'un mammifère non-humain selon l'une quelconque des revendications 1 à 6, **caractérisée en ce que** la mutation du gène codant pour la sous-unité alpha6 du nAChR est inductible.

8. Utilisation d'un mammifère non-humain selon l'une quelconque des revendications 1 à 7, **caractérisée en ce qu'**il s'agit d'une souris.

9. Utilisation d'un mammifère non-humain selon l'une quelconque des revendications 1 à 8, **caractérisée en ce que** son gène codant pour la sous-unité alpha6 du nAChR est muté par recombinaison homologue avec la séquence contenue dans la souche *E*. *coli* déposée à la CNCM sous le numéro I-2550, le 29 août 2000.

10. Utilisation d'un mammifère non-humain selon l'une quelconque des revendications 1 à 9, **caractérisée en ce que** son gène de la sous-unité alpha6 est muté de façon hétérozygote ou alternativement de façon homozygote.

11. Utilisation d'une culture cellulaire obtenue à partir des cellules d'un mammifère non-humain tel que défini dans l'une quelconque des revendications 1 à 10, **caractérisée en ce qu'**il s'agit de cellules neuronales.

12. Utilisation d'une culture cellulaire selon la revendication 11, **caractérisée en ce que** les cellules sont des neurones mésencéphaliques.

13. Utilisation d'une préparation obtenue à partir des cellules d'un mammifère non-humain tel que défini dans l'une quelconque des revendications 1 à 10, **caractérisée en ce qu'**il s'agit de synaptosomes striataux.

14. Utilisation selon l'une des revendications 1 à 13, pour étudier les effets de drogues à dépendance, sur les récepteurs nicotiniques.

15. Utilisation selon l'une des revendications 1 à 14, pour déterminer l'effet d'agonistes de récepteurs nicotiniques de l'acétylcholine (nAchR) sur la libération de dopamine.

16. Utilisation d'un mammifère non-humain selon l'une quelconque des revendications 1 à 10 ou d'une culture cellulaire selon l'une quelconque des revendications 11 à 12 ou d'une préparation selon la revendication 13, pour le criblage de substances agonistes ou antagonistes des nAchR.

17. Utilisation d'un mammifère non-humain selon l'une quelconque des revendications 1 à 10 ou d'une culture cellulaire selon l'une quelconque des revendications 11 à 12 ou d'une préparation selon la revendication 13, pour le criblage de substances agonistes ou antagonistes des récepteurs nicotiniques de l'acétylcholine des neurones catécholaminergiques ou dopaminergiques.

18. Procédé pour le criblage de substances susceptibles d'interagir avec la sous-unité alpha6 du nAChR, sélectionnées parmi les substances interagissant avec les nAChR, comprenant :
- la mise en contact de la substance sélectionnée avec un mammifère non-humain tel que défini dans l'une quelconque des revendications 1 à 10 ou avec une culture cellulaire telle que définie dans l'une quelconque des revendications 11 à 12, ou avec une préparation telle que définie dans la revendication 13 dans des conditions permettant l'interaction entre ladite substance et les récepteurs nicotiniques de l'acétylcholine, et
- la détection de l'interaction de type ligand/récepteur.

19. Procédé pour le criblage de substances susceptibles d'interagir avec la sous-unité alpha6 du nAChR, sélectionnées parmi les substances interagissant avec les nAChR, comprenant :
- la mise en contact de la substance sélectionnée avec un mammifère non-humain tel que défini dans l'une quelconque des revendications 1 à 10 ou avec une culture cellulaire telle que définie dans l'une quelconque des revendications 11 à 12, ou avec une préparation telle que définie dans la revendication 13 dans des conditions permettant l'interaction entre ladite substance et les récepteurs nicotiniques de l'acétylcholine, et
- la détection du niveau de dopamine libérée, cette détection étant réalisée dans le striatum lorsqu'un mammifère non-humain est utilisé.

20. Procédé selon la revendication 19, **caractérisé en ce que** les substances testées sont des agonistes nicotiniques des nAChR ou des antagonistes des nAChR.

21. Procédé pour l'identification de substance ayant une affinité pour la sous-unité alpha6 des nAChR, sélective vis-à-vis des récepteurs nicotiniques de l'acétylcholine restreints aux neurones dopaminergiques, comprenant :
- la mise en contact de la substance sélectionnée avec un mammifère non-humain tel que défini dans l'une quelconque des revendications 1 à 10 ou avec une culture cellulaire telle que définie dans l'une quelconque des revendications 11 à 12 ou avec une préparation telle que définie dans la revendication 13, dans des conditions permettant l'interaction entre ladite substance et les récepteurs nicotiniques de l'acétylcholine, et
- la détection de l'interaction sélective de type ligand/récepteur.

22. Procédé selon l'une quelconque des revendications 20 ou 21 dans lequel les substances testées sont sélectionnées dans le groupe des drogues nicotiniques sélectives vis à vis des neurones dopaminergiques.

23. Procédé pour l'identification de substance ayant une affinité pour la sous-unité alpha6 des nAChR, sélective vis-à-vis des récepteurs nicotiniques de l'acétylcholine restreints aux neurones dopaminergiques, comprenant :
- la mise en contact de la substance sélectionnée avec un mammifère non-humain tel que défini dans l'une quelconque des revendications 1 à 10 ou avec une culture cellulaire telle que définie dans l'une quelconque des revendications 11 à 12 ou avec une préparation telle que définie dans la revendication 13, dans des conditions permettant l'interaction entre ladite substance et les récepteurs nicotiniques de l'acétylcholine, et
- la détection du niveau de dopamine libérée, cette détection étant réalisée dans le striatum lorsqu'un mammifère non humain est utilisé.

24. Procédé selon la revendication 23 pour l'identification de substances ayant une affinité sélective pour les récepteurs nAChR comportant une sous-unité alpha6, comprenant :
- a) la mise en contact de la substance testée ayant une activité de liaison aux récepteurs nAChR, avec un mammifère non-humain tel que défini dans l'une quelconque des revendications 1 à 10 ou avec une culture cellulaire telle que définie dans l'une quelconque des revendications 11 à 12 ou avec une préparation telle que définie dans la revendication 13, dans des conditions permettant l'interaction entre ladite substance et les récepteurs nicotiniques de l'acétylcholine,
- b) la détection du niveau de dopamine libérée, cette détection étant réalisée dans le striatum lorsqu'un mammifère non-humain est utilisé, et
- c) la comparaison de l'augmentation du niveau de dopamine libérée à l'étape b), avec le niveau de dopamine libérée dans le striatum de mammifère non-humain, dans la culture cellulaire ou dans la préparation, exprimant la sous-unité alpha6 du nAChR.

25. Utilisation selon l'une des revendications 1 à 24 dans laquelle le gène muté codant pour la sous-unité alpha6 du récepteur nAChR, est contenu dans un plasmide sélectionné parmi a6HN (CNCM, I-2500), a6EH (CNCM I-2501), a6EE (CNCM, I-2502), a6PP (CNCM, I-2503), ou a6KO (CNCM, I-2550).

26. Utilisation selon l'une des revendications 1 à 24 dans laquelle le gène muté est sélectionné parmi les séquences SED ID NO : 3, SEQ ID NO : 4, SEQ ID NO : 5, SEQ ID NO : 6 ou toute partie de ces SED ID correspondant à un fragment séquencé du gène de la sous-unité alpha6 du nAChR.

27. Procédé de criblage de substances susceptibles d'interagir avec la sous-unité alpha6 du nAChR, sélectionnées parmi les substances interagissant avec les récepteurs nAChR, comprenant :
- la mise en contact de la substance sélectionnée avec une culture cellulaire définie selon l'une quelconque des revendications 11 ou 12, dans des conditions permettant l'interaction entre ladite substance et les récepteurs nicotiniques de l'acétylcholine,
- l'application d'une agression à cette culture cellulaire, et
- l'observation de l'effet neuroprotecteur de cette substance sur cette culture vis-à-vis de cette agression.

## Claims

1. Use of a non-human mammal carrying a mutation in the gene coding for the alpha6 subunit of the nicotinic acetylcholine receptor (nAChR), said mutation preventing expression in the mammal of said nAChR alpha6 subunit in a functional form, in order to study the effects of specific substances which are capable of interacting with the nAChR alpha6 subunit.

2. Use of a non-human mammal according to claim 1, **characterized in that** the gene coding for the nAChR alpha6 subunit is mutated by deletion of all or a portion of its exons.

3. Use of a non-human mammal according to claim 2, **characterized in that** the gene coding for the nAChR alpha6 subunit is mutated by deletion of at least a portion of exon 1 and/or of exon 2, said deletion being sufficient to prevent binding of said nAChR alpha6 subunit with its agonists and/or antagonists, in the nAChR alpha6 subunit expressed in the mammal.

4. Use of a non-human mammal according to claim 3, **characterized in that** the gene coding for the nAChR alpha6 subunit is mutated by deletion of exons 1 and 2.

5. Use of a non-human mammal according to any one of claims 1 to 4, **characterized in that** the mutation of the gene coding for the nAChR alpha6 subunit is homozygous and results in the absence of expression of the alpha6 subunit, said mammal having a (α6^{-/-}) phenotype.

6. Use of a non-human mammal according to any one of claims 1 to 4, **characterized in that** the mutation in the gene coding for the nAChR alpha6 subunit is homozygous, said mammal having a (α6^{Δ1-2/Δ1-2}) phenotype.

7. Use of a non-human mammal according to any one of claims 1 to 6, **characterized in that** the mutation in the gene coding for the nAChR alpha6 subunit is inducible.

8. Use of a non-human mammal according to any one of claims 1 to 7, **characterized in that** it is a mouse.

9. Use of a non-human mammal according to any one of claims 1 to 8, **characterized in that** its gene coding for the nAChR alpha6 subunit is mutated by homologous recombination with the sequence contained in the *E. coli* strain deposited with the CNCM with accession number I-2550 on 29^{th} August 2000.

10. Use of a non-human mammal according to any one of claims 1 to 9, **characterized in that** its gene for the alpha6 subunit is mutated in a heterozygous manner or alternatively in a homozygous manner.

11. Use of a cell culture obtained from cells of a non-human mammal as defined in any one of claims 1 to 10, **characterized in that** they are neuronal cells.

12. Use of a cell culture according to claim 11, **characterized in that** these cells are mesencephalic neurons.

13. Use of a preparation obtained from cells of a non-human mammal as defined in any one of claims 1 to 10, **characterized in that** they are striatal synaptosomes.

14. Use according to one of claims 1 to 13, for the study of the effect of addictive drugs on nicotinic receptors.

15. Use according to one of claims 1 to 14, to determine the effect of agonists of the nicotinic acetylcholine receptors (nAChR) on dopamine release.

16. Use of a non-human mammal according to any one of claims 1 to 10 or of a cell culture according to claim 11 or claim 12 or of a preparation according to claim 13, in screening nAChR agonist or antagonist substances.

17. Use of a non-human mammal according to any one of claims 1 to 10 or of a cell culture according to claim 11 or claim 12 or of a preparation according to claim 13, in screening agonist or antagonist substances of the nicotinic acetylcholine receptors of catecholaminergic or dopaminergic neurons.

18. A method for screening substances which are capable of interacting with the nAChR alpha6 subunit, selected from substances interacting with nAChRs, comprising:
• bringing the selected substance into contact with a non-human mammal as defined in any one of claims 1 to 10 or with a cell culture as defined in claim 11 or 12, or with a preparation as defined in claim 13, under conditions that allow interaction between said substance and the nicotinic acetylcholine receptors; and
• detecting the ligand/receptor type interaction.

19. A method for screening substances which are capable of interacting with the nAChR alpha6 subunit, selected from substances interacting with nAChRs, comprising:
• bringing the selected substance into contact with a non-human mammal as defined in any one of claims 1 to 10 or with a cell culture as defined in claim 11 or 12, or with a preparation as defined in claim 13, under conditions that allow interaction between said substance and the nicotinic acetylcholine receptors; and
• detecting the amount of dopamine released, said detection being carried out in the striatum when a non-human mammal is used.

20. A method according to claim 19, **characterized in that** the test substances are nicotinic nAChR agonists or nAChR antagonists.

21. A method for identifying a substance having an affinity for the nAChR alpha6 subunit, which is selective as regards nicotinic acetylcholine receptors restricted to dopaminergic neurons, comprising:
• bringing the selected substance into contact with a non-human mammal as defined in any one of claims 1 to 10 or with a cell culture as defined in claim 11 or 12, or with a preparation as defined in claim 13, under conditions allowing interaction between said substance and the nicotinic acetylcholine receptors; and
• detecting the selective ligand/receptor type interaction.

22. A method according to claim 20 or 21, in which the test substances are selected from the group of nicotinic drugs which are selective as regards dopaminergic neurons.

23. A method for identifying a substance having an affinity for the nAChR alpha6 subunits which is selective as regards nicotinic acetylcholine receptors restricted to dopaminergic neurons, comprising:
• bringing the selected substance into contact with a non-human mammal as defined in any one of claims 1 to 10 or with a cell culture as defined in claim 11 or 12, or with a preparation as defined in claim 13, under conditions allowing interaction between said substance and the nicotinic acetylcholine receptors; and
• detecting the amount of dopamine released, said detection being carried out in the striatum when a non-human mammal is used.

24. A method according to claim 23 for identifying substances having a selective affinity for nAChR receptors comprising an alpha6 subunit, comprising:
a) bringing the test substance with a binding activity to nAChR receptors into contact with a non-human mammal as defined in any one of claims 1 to 10 or with a cell culture as defined claim 11 or 12 or with a preparation as defined in claim 13, under conditions allowing interaction between said substance and the nicotinic acetylcholine receptors;
b) detecting the amount of dopamine released, said detection being carried out in the striatum when a non-human mammal is used; and
c) comparing the increase in the amount of dopamine released in step b) with the amount of dopamine released in the striatum of the non-human mammal, in the cell culture or in the preparation, expressing the nAChR alpha6 subunit.

25. Use according to one of claims 1 to 24, in which the mutated gene coding for the alpha6 subunit of the nAChR receptor is contained in a plasmid selected from a6HN (CNCM, I-2500), a6EH (CNCM, I-2501), a6EE (CNCM, I-2502), a6PP (CNCM, I-2503) or a6KO (CNCM, I-2550).

26. Use according to one of claims 1 to 24, in which the mutated gene is selected from sequences SEQ ID NO: 3, SEQ ID NO: 4, SEQ ID NO: 5, SEQ ID NO: 6 or any part of said SEQ IDs corresponding to a sequenced fragment of a gene of the nAChR alpha6 subunit.

27. A method for screening substances which are capable of interacting with the nAChR alpha6 subunit, selected from substances interacting with nAChR receptors, comprising:
• bringing the selected substance into contact with a cell culture defined in claim 11 or 12, under conditions allowing interaction between said substance and nicotinic alpha6 receptors;
• subjecting said cell culture to a challenge; and
• observing the neuroprotective effect of said substance on said culture towards said challenge.

## Patentansprüche

1. Verwendung eines nicht-menschlichen Säugetiers, welches Träger einer Mutation in dem Gen ist, das für die alpha6-Untereinheit des nikotinischen Acetylcholinrezeptors (nAChR) kodiert, wobei die Mutation bei dem Säugetier die Expression der alpha6-Untereinheit des nAChR in einer funktionellen Form verhindert, um die Wirkungen bestimmter Substanzen zu untersuchen, die in der Lage sind, mit der alpha6-Untereinheit des nAChR zu interagieren.

2. Verwendung eines nicht-menschlichen Säugetiers gemäß Anspruch 1, **dadurch gekennzeichnet, dass** das Gen, das für die alpha6-Untereinheit des nAChR kodiert, durch Deletion aller oder eines Teils der Exone mutiert ist.

3. Verwendung eines nicht-menschlichen Säugetiers gemäß Anspruch 2, **dadurch gekennzeichnet, dass** das Gen, das für die alpha6-Untereinheit des nAChR kodiert, durch Deletion wenigstens eines Teils des Exons 1 und/oder des Exons 2 mutiert ist, wobei diese Deletion ausreichend ist, um auf Ebene der alpha6-Untereinheit des nAChR, die bei dem Säugetier exprimiert ist, die Bindung der alpha6-Untereinheit des nAChR mit seinen Agonisten und/oder seinen Antagonisten zu verhindern.

4. Verwendung eines nicht-menschlichen Säugetiers gemäß Anspruch 3, **dadurch gekennzeichnet, dass** das Gen, das für die alpha6-Untereinheit des nAChR kodiert, durch Deletion der Exone 1 und 2 mutiert ist.

5. Verwendung eines nicht-menschlichen Säugetiers gemäß einem der Ansprüche 1 bis 4, **dadurch gekennzeichnet, dass** die Mutation des Gens, das für die alpha6-Untereinheit des nAChR kodiert, homozygot ist und zum Fehlen der Expression der alpha6-Untereinheit führt, wobei das Säugetier einen Phänotyp (α6^{-/-}) aufweist.

6. Verwendung eines nicht-menschlichen Säugetiers gemäß einem der Ansprüche 1 bis 4, **dadurch gekennzeichnet, dass** die Mutation des Gens, das für die alpha6-Untereinheit des nAChR kodiert, homozygot ist, wobei das Säugetier einen Phänotyp (α6^{Δ1-2/Δ1-2}) aufweist.

7. Verwendung eines nicht-menschlichen Säugetiers gemäß einem der Ansprüche 1 bis 6, **dadurch gekennzeichnet, dass** die Mutation des Gens, das für die alpha6-Untereinheit des nAChR kodiert, induzierbar ist.

8. Verwendung eines nicht-menschlichen Säugetiers gemäß einem der Ansprüche 1 bis 7, **dadurch gekennzeichnet, dass** es sich um eine Maus handelt.

9. Verwendung eines nicht-menschlichen Säugetiers gemäß einem der Ansprüche 1 bis 8, **dadurch gekennzeichnet, dass** das Gen, das für die alpha6-Untereinheit des nAChR kodiert, durch homologe Rekombination mit der Sequenz mutiert ist, die in dem Stamm *E. coli,* hinterlegt beim CNCM am 29. August 2000 unter der Nummer I-2550, enthalten ist.

10. Verwendung eines nicht-menschlichen Säugetiers gemäß einem der Ansprüche 1 bis 9, **dadurch gekennzeichnet, dass** das Gen der alpha6-Untereinheit auf heterozygote Weise oder alternativ auf homozygote Weise mutiert ist.

11. Verwendung einer Zellkultur, die aus Zellen eines nicht-menschlichen Säugetiers gemäß einem der Ansprüche 1 bis 10 erhalten wird, **dadurch gekennzeichnet, dass** es sich um Nervenzellen handelt.

12. Verwendung einer Zellkultur gemäß Anspruch 11, **dadurch gekennzeichnet, dass** die Zellen mesencephale Nervenzellen sind.

13. Verwendung einer Zubereitung, die aus Zellen eines nicht-menschlichen Säugetiers gemäß einem der Ansprüche 1 bis 10 erhalten wird, **dadurch gekennzeichnet, dass** es sich um striatale Synaptosome handelt.

14. Verwendung gemäß einem der Ansprüche 1 bis 13, um die Wirkungen süchtigmachender Drogen auf die nikotinischen Rezeptoren zu untersuchen.

15. Verwendung gemäß einem der Ansprüche 1 bis 14, um die Wirkung von Agonisten von nikotinischen Acetylcholinrezeptoren (nAChR) auf die Freisetzung von Dopamin zu bestimmen.

16. Verwendung eines nicht-menschlichen Säugetiers gemäß einem der Ansprüche 1 bis 10 oder einer Zellkultur gemäß einem der Ansprüche 11 oder 12 oder einer Zubereitung gemäß Anspruch 13 für das Screening agonistischer oder antagonistischer Substanzen von nAChR.

17. Verwendung eines nicht-menschlichen Säugetiers gemäß einem der Ansprüche 1 bis 10 oder einer Zellkultur gemäß einem der Ansprüche 11 oder 12 oder einer Zubereitung gemäß Anspruch 13 für das Screening agonistischer oder antagonistischer Substanzen von nikotinischen Acetylcholinrezeptoren der Dopamin oder Katecholamin ausschüttenden Nervenzellen.

18. Verfahren für das Screening von Substanzen, die in der Lage sind, mit der alpha6-Untereinheit von nAChR zu interagieren, und die aus den Substanzen gewählt sind, die mit nAChR interagieren, welches aufweist:
- das Inkontaktbringen der gewählten Substanz mit einem nicht-menschlichen Säugetier gemäß einem der Ansprüche 1 bis 10 oder einer Zellkultur gemäß einem der Ansprüche 11 oder 12 oder einer Zubereitung gemäß Anspruch 13 unter Konditionen, die die Interaktion der Substanz mit den nikotinischen Acetylcholinrezeptoren ermöglichen, und
- die Detektion der Interaktion der Art Ligand/Rezeptor.

19. Verfahren für das Screening von Substanzen, die in der Lage sind, mit der alpha6-Untereinheit von nAChR zu interagieren, und die aus den Substanzen gewählt sind, die mit nAChR interagieren, welches aufweist:
- das Inkontaktbringen der gewählten Substanz mit einem nicht-menschlichen Säugetier gemäß einem der Ansprüche 1 bis 10 oder einer Zellkultur gemäß einem der Ansprüche 11 oder 12 oder einer Zubereitung gemäß Anspruch 13 unter Konditionen, die die Interaktion der Substanz mit den nikotinischen Acetylcholinrezeptoren ermöglichen, und
- die Detektion der Höhe an freigesetztem Dopamin, wobei diese Detektion in dem Striatum durchgeführt wird, wenn ein nicht-menschliches Säugetier verwendet wird.

20. Verfahren gemäß Anspruch 19, **dadurch gekennzeichnet, dass** die getesteten Substanzen nikotinische Agonisten von nAChR oder Antagonisten von nAChR sind.

21. Verfahren zur Identifikation der Substanz, die eine Affinität für die alpha6-Untereinheit von nAChR aufweist, die gegenüber nikotinischen Acetylcholinrezeptoren selektiv ist, die auf Dopamin ausschüttende Nervenzellen beschränkt sind, welches aufweist:
- das Inkontaktbringen der gewählten Substanz mit einem nicht-menschlichen Säugetier gemäß einem der Ansprüche 1 bis 10 oder einer Zellkultur gemäß einem der Ansprüche 11 oder 12 oder einer Zubereitung gemäß Anspruch 13 unter Konditionen, die die Interaktion der Substanz mit den nikotinischen Acetylcholinrezeptoren ermöglichen, und
- die Detektion der selektiven Interaktion der Art Ligand/Rezeptor.

22. Verfahren gemäß einem der Ansprüche 20 oder 21, bei welchem die getesteten Substanzen aus der Gruppe nikotinischer Drogen gewählt ist, die gegenüber Dopamin ausschüttenden Nervenzellen selektiv sind.

23. Verfahren zur Identifikation der Substanz, die eine Affinität für die alpha6-Untereinheit von nAChR aufweist, die gegenüber nikotinischen Acetylcholinrezeptoren selektiv ist, die auf Dopamin ausschüttende Nervenzellen beschränkt sind, welches aufweist:
- das Inkontaktbringen der gewählten Substanz mit einem nicht-menschlichen Säugetier gemäß einem der Ansprüche 1 bis 10 oder einer Zellkultur gemäß einem der Ansprüche 11 oder 12 oder einer Zubereitung gemäß Anspruch 13 unter Konditionen, die die Interaktion der Substanz mit den nikotinischen Acetylcholinrezeptoren ermöglichen, und
- die Detektion der Höhe an freigesetztem Dopamin, wobei diese Detektion in dem Striatum durchgeführt wird, wenn ein nicht-menschliches Säugetier verwendet wird.

24. Verfahren gemäß Anspruch 23 für die Identifikation von Substanzen mit einer selektiven Affinität für nACh-Rezeptoren, die eine alpha6-Untereinheit umfassen, welches aufweist:
a) das Inkontaktbringen der getesteten Substanz, die eine Aktivität zur Bindung an die nACh-Rezeptoren aufweist, mit einem nicht-menschlichen Säugetier gemäß einem der Ansprüche 1 bis 10 oder einer Zellkultur gemäß einem der Ansprüche 11 oder 12 oder einer Zubereitung gemäß Anspruch 13 unter Konditionen, die die Interaktion der Substanz mit den nikotinischen Acetylcholinrezeptoren ermöglichen,
b) die Detektion der Höhe an freigesetztem Dopamin, wobei diese Detektion in dem Striatum durchgeführt wird, wenn ein nicht-menschliches Säugetier verwendet wird, und
c) den Vergleich des Anstiegs der Höhe an freigesetztem Dopamin bei Schritt b) mit der Höhe an in dem Striatum des nicht-menschlichen Säugetiers, der Zellkultur oder der Zubereitung, die die alpha6-Untereinheit von nAChR exprimieren, freigesetztem Dopamin.

25. Verwendung gemäß einem der Ansprüche 1 bis 24, bei welcher das mutierte Gen, das für die alpha6-Untereinheit des nACh-Rezeptors kodiert, in einem Plasmid enthalten ist, das gewählt wird aus a6HN (CNCM, I-2500), a6EH (CNCM, I-2501), a6EE (CNCM, I-2502), a6PP (CNCM, I-2503) oder a6KO (CNCM, I-2550).

26. Verwendung gemäß einem der Ansprüche 1 bis 24, bei welcher das mutierte Gen aus den Sequenzen SEQ ID NO : 3, SEQ ID NO : 4, SEQ ID NO : 5, SEQ ID NO : 6 oder jedem Teil dieser SEQ ID, der einem sequenzierten Fragment des Gens der alpha6-Untereinheit von nAChR entspricht, gewählt ist.

27. Screening-Verfahren von Substanzen, die in der Lage sind, mit der alpha6-Untereinheit von nAChR zu interagieren, die aus den Substanzen gewählt sind, die mit den nACh-Rezeptoren interagieren, welches aufweist:
- das Inkontaktbringen der gewählten Substanz mit einer Zellkultur gemäß Anspruch 11 oder 12 unter Konditionen, die die Interaktion zwischen dieser Substanz und den nikotinischen Acetylcholinrezeptoren ermöglichen,
- das Anwenden einer Aggression auf diese Zellkultur, und
- das Beobachten der Neuroprotektorwirkung dieser Substanz auf die Kultur gegenüber dieser Aggression.


### LISTE DE SEQUENCES

<110> INSTITUT PASTEUR
   CENTRE NATIONAL DE LA RECHERCHE SCIENTIFIQUE
<120> MAMMIFERE NON-HUMAIN MUTANT POUR L'EXPRESSION DE LA SOUS-UNITE ALPHA6 DU RECEPTEUR NICOTINIQUE DE L'ACETYLCHOLINE ET UTILISATION POUR LE CRIBLAGE DE SUBSTANCES SUSCEPTIBLES D'INTERAGIR AVEC CE RECEPTE
<130> B4644 INSTITUT PASTEUR
<140>
   <141>
<150> 0011247
   <151> 2000-09-04
<160> 7
<170> PatentIn Ver. 2.1
<210> 1
   <211> 23
   <212> ADN
   <213> Séquence artificielle
<220>
   <223> Description de la séquence artificielle: oligonucléotide
<220>
   <221> misc_feature
   <222> (1) .. (23)
   <223> Amorce amont
<400> 1
   actgctgtga agagatttac aca 23
<210> 2
   <211> 21
   <212> ADN
   <213> Séquence artificielle
<220>
   <223> Description de la séquence artificielle: oligonucléotide
<220>
   <221> misc_feature
   <222> (1) .. (21)
   <223> Amorce reverse
<400> 2
   aagatggtct tcacccactt g 21
<210> 3
   <211> 4494
   <212> ADN
   <213> souris
<400> 3
   aagctttatg ggccaaggtg gtgagaaaag attcacagag gaggtgaaat ccaaatgatc 60
   tttgatcaat attttaagtc acatgtcatt tctaagtcaa cagagcagag gcaactggaa 120
   acgttcggga tttctgttac agttaaatag ctttcatgca gtctccagtc ttcatgtctg 180
   acttcattag cacggataac ttagatttgt ctatttttag atataattct ctagttaaga 240
   cttgtattag caagcacata gaagactgaa aaatattatt tcttccttcc aggatgaagt 300
   caaccagatt atggagacca atctgtggct gcgtcacgta tgtgtccccc cctttgaatg 360
   gcggcagaat gtatccactt agtgataaag ccacctgcat taactttttc gcaccccaac 420
   ctatgataga taaagaatat ccttttcctt gctttctcct agtccttggg tcagctctgg 480
   ttgcagttat attaatatag gcagcacatg ggcagagcct ggtgtctgac atggaaccct 540
   ctggcctttc tcttttaagc tcccagttct ctttgtatca cttactgata ccaagnnnnn 600
   nnnnnnnnnn nnnnnnnnnn nnnnnnnnnn nnnnnnnnnn nnnnnnnnnn nnnnnnnnnn 660
   nnnnnnnnnn nnnnnnnnnn nnnnnnnnnn nnnnnnnnnn nnnnnnnnnn nnnnnnnnnn 720
   nnnnnnnnnn nnnnnnnnnn nnnnnnnnnn nnnnnnnnnn nnnnnnnnnn nnnnnnnnnn 780
   nnnnnnnnnn nnnnnnnnnn nnnnnnnnnn nnnnnnnnnn nnnnnnnnnn nnnnnnnnnn 840
   nnnnnnnnnn nnnnnnnnnn nnnnnnnnnn nnnnnnnnnn nnnnnnnnnn nnnnnnnnnn 900
   nnnnnnnnnn nnnnnnnnnn nnnnnnnnnn nnnnnnnnnn nnnnnnnnnn nnnnnnnnnn 960
   nnnnnnnnnn nnnnnnnnnn nnnnnnnnnn nnnnnnnnnn nnnnnnnnnn nnnnnnnnnn 1020
   nnnnnnnnnn nnnnnnnnnn nnnnnnnnnn nnnnnnnnnn nnnnnnnnnn nnnnnnnnnn 1080
   nnnnnnnnnn nnnnnnnnnn nnnnnnnnnn nnnnnatctg gaaggactac agattgcgtt 1140
   gggatccaac ggagtatgat ggcatcgaga cacttcgagt tccagcagac aacatctgga 1200
   agcctgacat cgttctgtat aacnnnnnnn nnnnnnnnnn nnnnnnnnnn nnnnnnnnnn 1260
   nnnnnnnnnn nnnnnnnnnn nnnnnnnnnn nnnnnnnnnn nnnnnnnnnn nnnnnnnnnn 1320
   nnnnnnnnnn nnnnnnnnnn nnnnnnnnnn nnnnnnnnnn nnnnnnnnnn nnnnnnnnnn 1380
   nnnnnnnnnn nnnnnnnnnn nnnnnnnnnn nnnnnnnnnn nnnnnnnnnn nnnnnnnnnn 1440
   nnnnnnnnnn nnnnnnnnnn nnnnnnnnnn nnnnnnnnnn nnnnnnnnnn nnnnnnnnnn 1500
   nnnnnnnnnn nnnnnnnnnn nnnnnnnnnn nnnnnnnnnn nnnnnnnnnn nnnnnnnnnn 1560
   nnnnnnnnnn nnnnnnnnnn nnnnnnnnnn nnnnnnnnnn nnnnnnnnnn nnnnnnnnnn 1620
   nnnnnnnnnn nnnnnnnnnn nnnnnnnnnn nnnnnnnnnn nnnnnnnnnn nnnnnnnnnn 1680
   nnnnnnnnnn nnnnnnnnnn nnnnnnnnnn nnnnnnnnnn nnnnnnnnnn nnnnnnnnnn 1740
   nnnnnnnnnn nnnnnnnnnn nnnnnnnnnn nnnnnnnnnn nnnnnnnnnn nnnnnnnnnn 1800
   nnnnnnnnnn nnnnnnnnnn nnnnnnnnnn nnnnnnnnnn nnnnnnnnnn nnnnnnnnnn 1860
   nnnnnnnnnn nnnnnnnnnn nnnnnnnnnn nnnnnnnnnn nnnnnnnnnn nnnnnnnnnn 1920
   nnnnnnnnnn nnnnnnnnnn nnnnnnnnnn nnnnnnnnnn nnnnnnnnnn nnnnnnnnnn 1980
   nnnnnnnnnn nnnnnnnnnn nnnnnnnnnn nnnnnnnnnn nnnnnnnnnn nnnnnnnnnn 2040
   nnnnnnnnnn nnnnnnnnnn nnnnnnnnnn nnnnnnnnnn nnnnnnnnnn nnnnnnnnnn 2100
   nnnnnnnnnn nnnnnnnnnn nnnnnnnnnn nnnnnnnnnn nnnnnnnnnn nnnnnnnnnn 2160
   nnnnnnnnnn nnnnnnnnnn nnnnnnnnnn nnnnnnnnnn nnnnnnnnnn nnnnnnnnnn 2220
   nnnnnnnnnn nnnnnnnnnn nnnnnnnnnn nnnnnnnnnn nnnnnnnnnn nnnnnnnnnn 2280
   nnnnnnaatg ctgtcggcga cttccaggtc gaaggcaaga ccaaagctct tctcaagtat 2340
   gacggtgtga taacctggac cccaccagcc atctttaaga gctcctgccc aatggacatc 2400
   accttcttcc cgtttgacca tcaaaactgc tccctgaagt ttggttcctg gacttatgac 2460
   aaggcagaaa tcgaccttct catcattggc tctaaagtag acatgaacga cttttgggaa 2520
   aacagtgaat gggaaattgt cgacgcctct ggctataagc atgacatcaa gtacaactgc 2580
   tgtgaagaga tttacacgga cataacctac tccttctaca tcaggaggtt gcccatgttt 2640
   tacaccatca acctcatcat cccctgcctc ttcatttcct tcctcacggt gctggttttt 2700
   taccttccct ccgactgtgg cgagaaagtg actctttgca tctccgttct gctttctctc 2760
   actgtctttt tgctggtgat tacagagacc atcccatcca catctctcgt gatcccactg 2820
   gtgggtgagt atctactgtt caccatgatc tttgtcacgc tgtccattgt ggtgaccgtg 2880
   ttcgtgctga acatacacta caggacccca gcaacgcata ccatgcccaa gtgggtgaag 2940
   accatcttcc ttcaggcctt cccctcgatt ctgatgatga ggaaacctct ggacaagaca 3000
   aaggaggcag gaggtgttaa ggaccccaaa agccatacca agaggcctgc caaggtcaaa 3060
   tttactcatc gaggagaatc caaacttcta aaggaatgcc accactgcca aaaatcaagt 3120
   gacatagcac ctggaaagag aagatcaagc cagcagcctg cacggtgggt ggcagagaat 3180
   tcagagcact cgtccgatgt tgaagatgtc atcgagagtg ttcaattcat agcagaaaac 3240
   atgaagagcc acaatgaaac aaacgaggta aaagtggagc ccttttctcc agccagctgc 3300
   acccctagca ggcctacagg cactttagag actagtcaga gcgtcagtgg gagttacata 3360
   tgtggaacag tcagggaccg tcacctaaga ccagctctat tatcatgaag ccttgtggga 3420
   cctgggttca agtttaggga gctatagtga gaggatatat gtagtcctac aacaaatctt 3480
   cagcctgcat ttacttacgg tgaggtctag ccacagtgca catgcaggac aagccttcct 3540
   caaggaacaa gcctccaatg catcgaacac tgacaaagtg agggtgggaa gggagactgt 3600
   agaaatcatt attaataaaa tcccaccggc gggcttgcta cctgctctaa tggtttgtgt 3660
   tcccaaatga aacacacaca cacacacaca cacacacaca cacacacaca cagtctttgt 3720
   gttttaatat gccgtataca gcacaatagc tgggccactg cctaccctcc atgctgttac 3780
   tatacctccc gccaacaatc cccaagttat tacttactaa ttctatgtgc tatcttggtt 3840
   gcccctagac ccagttgggc agcctctggg ccatgttttc cccggctatt tcaagtggtg 3900
   gccatgtctc tctgtcctgc agtcttctca ggcctagcat ctcacctctt cctccacact 3960
   ctcccggcat ggcagggtct cacttctcct cctcctcctc ctcctcctcc accacctcct 4020
   ttctcctccc tccctcctcc ctcccctctt tcccctcccc ccttccttcc cagcctgaga 4080
   actcctaaaa tcccacctct ccctgtcctt tccagctttg gctgttggca gctttattta 4140
   ccaataagaa ccaactgcgg gcaggttccc agaaactaca ggcagacagt cccacgaaaa 4200
   cagttttacg tggaccataa ttagcattca taatacgtgc agctacagag acacccaagg 4260
   aaaagaagaa ctgtggtact gcctgaagcc ccagtggtga aagatgcccc ttagcagtca 4320
   gcagctccca tcacacttcc tctactagag ggggacccag acatgagtta aagacatctt 4380
   gaagatgtgc tcagtatgca cacgtattcc gtgcctgtgt cccacactct aatgcctaga 4440
   gcttggctcc tcctacccca agcaagtgct caaggatagg gagatcgcgg ccgc 4494
<210> 4
   <211> 4006
   <212> ADN
   <213> souris
<400> 4
   gaattcagag agcttttatc atgctgaaca gccgggacca gggaaacctg cactccggtt 60
   tatgtctgtg gctatgtgga ttcctggctc tctttaaagg tgagttatta cggcttatat 120
   cctagaatac aattggtagt gtgcatttcc tcttctttct catcttgtct ggatccaaat 180
   aattagagga ctttaattag ttaggcttaa cctaacagat atgatttgga aagtaaggta 240
   gagaaaacat tgaagtacag tataatattc atacttcatc tctccaacta aaatatgctt 300
   ttccaattag catacaaaat catctgcttt tgtagccatg tatgctacca atatgcttca 360
   tttacttacg gtaatttgtt agctgggact tattagcctg cgagtcctac caatgtgtgt 420
   aattgtcctc acttaggaaa cctgtcttat tgtctactaa gagtgagctt tcctgtctta 480
   aaagttggct cagacctggc tggtgcacac acacacacac acacacacac acacacacac 540
   acacacacga acaggcacac atgcacacat ctcggatgag gtttcagggg gtgctgatgg 600
   tgagctccag agagaggaaa cagtagcaac agccaagaga gctcactatc ttctgaaaca 660
   atgttttcat cctggttagt tacacaggag agaatgacag nnnnnnnnnn nnnnnnnnnn 720
   nnnnnnnnnn nnnnnnnnnn nnnnnnnnnn nnnnnnnnnn nnnnnnnnnn nnnnnnnnnn 780
   nnnnnnnnnn nnnnnnnnnn nnnnnnnnnn nnnnnnnnnn nnnnnnnnnn nnnnnnnnnn 840
   nnnnnnnnnn nnnnnnnnnn nnnnnnnnnn nnnnnnnnnn nnnnnnnnnn nnnnnnnnnn 900
   nnnnnnnnnn nnnnnnnnnn nnnnnnnnnn nnnnnnnnnn nnnnnnnnnn nnnnnnnnnn 960
   nnnnnnnnnn nnnnnnnnnn nnnnnnnnnn nnnnnnnnnn nnnnnnnnnn nnnnnnnnnn 1020
   nnnnnnnnnn nnnnnnnnnn nnnnnnnnnn nnnnnnnnnn nnnnnnnnnn nnnnnnnnnn 1080
   nnnnnnnnnn nnnnnnnnnn nnnnnnnnnn nnnnnnnnnn nnnnnnnnnn nnnnnnnnnn 1140
   nnnnnnnnnn nnnnnnnnnn nnnnnnnnnn nnnnnnnnnn nnnnnnnnnn nnnnnnnnnn 1200
   nnnnnnnnnn nnnnnnnnnn nnnnnnnnnn nnnnnnnnnn nnnnnnnnnn nnnnnnnnnn 1260
   nnnnnnnnnn nnnnnnnnnn nnnnnnnnnn nnnnnnnnnn nnnnnnnnnn nnnnnnnnnn 1320
   nnnnnnnnnn nnnnnnnnnn nnnnnnnnnn nnnnnnnnnn nnnnnnnnnn nnnnnnnnnn 1380
   nnnnnnnnnn nnnnnnnnnn nnnnnnnnnn nnnnnnnnnn nnnnnnnnnn nnnnnnnnnn 1440
   nnnnnnnnnn nnnnnnnnnn nnnnnnnnnn nnnnnnnnnn nnnnnnnnnn nnnnnnnnnn 1500
   nnnnnnnnnn nnnnnnnnnn nnnnnnnnnn nnnnnnnnnn nnnnnnnnnn nnnnnnnnnn 1560
   nnnnnnnnnn nnnnnnnnnn nnnnnnnnnn nnnnnnnnnn nnnnnnnnnn nnnnnnnnnn 1620
   nnnnnnnnnn nnnnnnnnnn nnnnnnnnnn nnnnnnnnnn nnnnnnnnnn nnnnnnnnnn 1680
   nnnnnnnnnn nnnnnnnnnn nnnnnnnnnn nnnnnnnnnn nnnnnnnnnn nnnnnnnnnn 1740
   nnnnnnnnnn nnnnnnnnnn nnnnnnnnnn nnnnnnnnnn nnnnnnnnnn nnnnnnnnnn 1800
   nnnnnnnnnn nnnnnnnnnn nnnnnnnnnn nnnnnnnnnn nnnnnnnnnn gcagtacagg 1860
   ctgtgaatct gaagagcagc tcttccacag gctgtttgct cactacaacc gcttcatccg 1920
   gccggtggag aatgtctccg atcccgtcac ggtgcatctt gaattggcaa tcacgcaact 1980
   ggccaatgtg nnnnnnnnnn nnnnnnnnnn nnnnnnnnnn nnnnnnnnnn nnnnnnnnnn 2040
   nnnnnnnnnn nnnnnnnnnn nnnnnnnnnn nnnnnnnnnn nnnnnnnnnn nnnnnnnnnn 2100
   nnnnnnnnnn nnnnnnnnnn nnnnnnnnnn nnnnnnnnnn nnnnnnnnnn nnnnnnnnnn 2160
   nnnnnnnnnn nnnnnnnnnn nnnnnnnnnn nnnnnnnnnn nnnnnnnnnn nnnnnnnnnn 2220
   nnnnnnnnnn nnnnnnnnnn nnnnnnnnnn nnnnnnnnnn nnnnnnnnnn nnnnnnnnnn 2280
   nnnnnnnnnn nnnnnnnnnn nnnnnnnnnn nnnnnnnnnn nnnnnnnnnn nnnnnnnnnn 2340
   nnnnnnnnnn nnnnnnnnnn nnnnnnnnnn nnnnnnnnnn nnnnnnnnnn nnnnnnnnnn 2400
   nnnnnnnnnn nnnnnnnnnn nnnnnnnnnn nnnnnnnnnn nnnnnnnnnn nnnnnnnnnn 2460
   nnnnnnnnnn nnnnnnnnnn nnnnnnnnnn nnnnnnnnnn nactccctgg gctgagcata 2520
   tacaaaccat cacaccatgt ctgccagttg attgcagctt ttccagaaac tagtgcattt 2580
   gaaaccatga ttcaaaattg ttcacctggg tgtcaactaa tattttcccg ataaagaggt 2640
   ctaggctaat ggttccaaca acgtggtgtg gtactgtgtg tctctggcct cctcctccat 2700
   tagggctcac tgagctcagt gtccattgaa atggtctgga gttgcatgaa gactatgatt 2760
   tttccagagt aggtcctgac tcatgacatt ttctaacacc tccaaggttg tgtattcccc 2820
   atgaatacac accatatgga gcctatccaa accatggaag tgtctcaagg gaggcaggcc 2880
   tgttaagaat ggtactgata attagtgtca actccctgaa ctcacccagg ttttgaagac 2940
   tatgacaggt ttaggccagt tacctctaag acctcacgga agtgaggcag cagagcctat 3000
   ccagctttga ccaacagcta gtacatactt acccagcatc tgttagaccc tacagattca 3060
   aggcgactca ataccagagg gccctaatca atagagggga atcactggga agctgtgcag 3120
   gctccatttt cctaagtctt tttgttgact ttaccaatcc cagggcctgt gttggctcca 3180
   catgacccac tggcagggag aagcttnnnn nnnnnnnnnn nnnnnnnnnn nnnnnnnnnn 3240
   nnnnnnnnnn nnnnnnnnnn nnnnnnnnnn nnnnnnnnnn nnnnnnnnnn nnnnnnnnnn 3300
   nnnnnnnnnn nnnnnnncct gcgtagaaac ctcaaccagg ctgggatcct tcactcacag 3360
   ggacctcaga tgtcttggaa aatgaaattt tgaatcccct gctgactgct aagaacctac 3420
   tattaatacc caagctattc tgctgtgacc tgtcacctgt cctgactctc cttatgtcac 3480
   ttcagggact gcttggacag taactcttca aagaagcctc cttgactcgt atattaaata 3540
   gatgtgtgca tacacgcagg aatcactcca cctctcttgt tatatcatga tgtgttttat 3600
   gctggcgtac acatttatta ttagctctgt ctttccaacc acaacagcta agagcacatg 3660
   aaggcaggag ctttcttcac tctgtaactt gagtggaact ctgtaacaca gtggtcactg 3720
   ggtaagtagg aatatccctt gtgactgaag ggcccgtgag ctgggaaagg gaatacagag 3780
   ggagtgagta cctggtttca actggcaaac ttgcatgtga actaaactgc tgtttcagtt 3840
   tggatcccag ggcaaatgca cacagtacat cccaggcttc tttgaggaaa ggccatgggg 3900
   atagataaaa acagtgagta cctgtcgcca cctacaggac tctcccctct gtcgtccctg 3960
   aacctctgcc ctcctccctg ttacctttca tgcatctagg aagctt 4006
<210> 5
   <211> 5023
   <212> ADN
   <213> souris
<400> 5
   ctgcaggaat tcttaggcaa atggatggaa ctagaaaata tcatcctgag tgaggtaacc 60
   caatcacaaa agaacataca tggtatgcac tcactgataa gtggatatta cccccaaagt 120
   ttggaatatc caagatacaa ttcacagacc aaatgaagct caagaagaag gaagaacaaa 180
   gtatggatac ttcagtcttt cttagaaggg ggaacaaaat actcatggga ggagatacag 240
   agacaaagtg tggagcagag actgaaggaa aagccattca gagactgccc cagctgggga 300
   tccatcccat atacagtcac caaacccaga cactattgtt gatgccaaca agtgcatgct 360
   gacaggagtc tgacatagct gtctcctgag aggctctgcc agtgcctgac aaatatagag 420
   gggatgctca cagccaacca ctggactgag cacagggtcc tcaatggagg agttagagaa 480
   aggactgaag gaccagaaga ggtttgcagc cccataggag gaacaacaat atgaactagc 540
   cagtatcccc agagctccca gggactaagc caccaaccaa agagtacata tgcaggaacc 600
   catggctcca gctgcatatg tagcagagga tgtcacttct caatgggagg agaggcccct 660
   ggtcctgaga aggctcaatg ccccagtgta ggggaatgcc aagacaggga agtgggagtg 720
   tgtgggttgg tgagcagggg ggaggaggaa aggagtaggg ggtctttgga ggggaaacca 780
   ggaaaggaga taacatttga aatgtaaatg aagaaaatat ctaatttttt aagaaagaaa 840
   aaagacaatt acagacttag ctgtgcctgt agccactgtt ctcctagagt tcagagcctg 900
   cctgtctggg accagacacc tctggctgag gtttcttgcc tggcattatg tgcagggtcc 960
   ggctccattt tctcacccag ccctgccttc cacagctctg tgcacaggag aggatccaag 1020
   gagaagggaa gtggaggtgg tggcgacagt gacccagttc agaaaggact ctgaggcaca 1080
   ttcaatctat aaagtattca tggctctaaa agccttccaa ggggatccaa agcatttgct 1140
   gagctgcagn nnnnnnnnnn nnnnnnnnnn nnnnnnnnnn nnnnnnnnnn nnnnnnnnnn 1200
   nnnnnnnnnn nnnnnnnnnn nnnnnnnnnn nnnnnnnnnn nnnnnnnnnn nnnnnnnnnn 1260
   nnnnnnnnnn nnnnnnnnnn nnnnnnnnnn nnnnnnnnnn nnnnnnnnnn nnnnnnnnnn 1320
   nnnnnnnnnn nnnnnnnnnn nnnnnnnnnn nnnnnnnnnn nnnnnnnnnn nnnnnnnnnn 1380
   nnnnnnnnnn nnnnnnnnnn nnnnnnnnnn nnnnnnnnnn nnnnnnnnnn nnnnnnnnnn 1440
   nnnnnnnnnn nnnnnnnnnn nnnnnnnnnn nnnnnnnnnn nnnnnnnnnn nnnnnnnnnn 1500
   nnnnnnnnnn nnnnnnnnnn nnnnnnnnnn nnnnnnnnnn nnnnnnnnnn nnnnnnnnnn 1560
   nnnnnnnnnn nnnnnnnnnn nnnnnnnnnn nnnnnnnnnn nnnnnnnnnn nnnnnnnnnn 1620
   nnnnnnnnnn nnnnnnnnnn nnnnnnnnnn nnnnnnnnnn nnnnnnnnnn nnnnnnnnnn 1680
   nnnnnnnnnn nnnnnnnnnn nnnnnnnnnn nnnnnnnnnn nnnnnnnnnn nnnnnnnnnn 1740
   nnnnnnnnnn nnnnnnnnnn nnnnnnnnnn nnnnnnnnnn nnnnnnnnnn nnnnnnnnnn 1800
   nnnnnnnnnn nnnnnnnnnn nnnnnnnnnn nnnnnnnnnn nnnnnnnnnn nnnnnnnnnn 1860
   nnnnnnnnnn nnnnnnnnnn nnnnnnnnnn nnnnnnnnnn nnnnnnnnnn nnnnnnnnnn 1920
   nnnnnnnnnn nnnnnnnnnn nnnnnnnnnn nnnnnnnnnn nnnnnnnnnn nnnnnnnnnn 1980
   nnnnnnnnnn nnnnnnnnnn nnnnnnnnnn nnnnnnnnnn nnnnnnnnnn nnnnnnnnnn 2040
   nnnnnnnnnn nnnnnnnnnn nnnnnnnnnn nnnnnnnnnn nnnnnnnnnn nnnnnnnnnn 2100
   nnnnnnnnnn nnnnnnnnnn nnnnnnnnnn nnnnnnnnnn nnnnnnnnnn nnnnnnnnnn 2160
   nnnnnnnnnn nnnnnnnnnn nnnnnnnnnn nnnnnnnnnn nnnnnnnnnn nnnnnnnnnn 2220
   nnnnnnnnnn nnnnnnnnnn nnnnnnnnnn nnnnnnnnnn nnnnnnnnnn nnnnnnnnnn 2280
   nnnnnnnnnn nnnnnnnnnn nnnnnnnnnn nnnnnnnnnn nnnnnnnnnn nnnnnnnnnn 2340
   nnnnnnnnnn nnnnnnnnnn nnnnnnnnnn nnnnnnnnnn nnnnnnnnnn nnnnnnnnnn 2400
   nnnnnnnnnn nnnnnnnnnn nnnnnnnnnn nnnnnnnnnn nnnnnnnnnn nnnnnnnnnn 2460
   nnnnnnnnnn nnnnnnnnnn nnnnnnnnnn nnnnnnnnnn nnnnnnnnnn nnnnnnnnnn 2520
   nnnnnnnnnn nnnnnnnnnn nnnnnnnnnn nnnnnnnnnn nnnnnnnnnn nnnnnnnnnn 2580
   nnnnnnnnnn nnnnnnnnnn nnnnnnnnnn nnnnnnnnnn nnnnnnnnnn nnnnnnnnnn 2640
   nnnnnnnnnn nnnnnnnnnn nnnnnnnnnn nnnnnnnnnn nnnnnnnnnn nnnnnnnnnn 2700
   nnnnnnnnnn nnnnnnnnnn nnnnnnnnnn nnnnnnnnnn nnnnnnnnnn nnnnnnnnnn 2760
   nnnnnnnnnn nnnnnnnnnn nnnnnnnnnn nnnnnnnnnn nnnnnnnnnn nnnnnnnnnn 2820
   nnnnnnnnnn nnnnnnnnnn nnnnnnnnnn nnnnnnnnnn nnnnnnnnnn nnnnnnnnnn 2880
   nnnnnnnnnn nnnnnnnnnn nnnnnnnnnn nnnnnnnnnn nnnnnnnnnn nnnnnnnnnn 2940
   nnnnnnnnnn nnnnnnnnnn nnnnnnnnnn nnnnnnnnnn nnnnnnnnnn nnnnnnnnnn 3000
   nnnnnnnnnn nnnnnnnnnn nnnnnnnnnn nnnnnnnnnn nnnnnnnnnn nnnnnnnnnn 3060
   nnnnnnnnnn nnnnnnnnnn nnnnnnnnnn nnnnnnnnnn nnnnnnnnnn nnnnnnnnnn 3120
   nnnnnnnnnn nnnnnnnnnn nnnnnnnnnn nnnnnnnnnn nnnnnnnnnn nnnnnnnnnn 3180
   nnnnnnnnnn nnnnnnnnnn nnnnnnnnnn nnnnnnnnnn nnnnnnnnnn nnnnnnnnnn 3240
   nnnnnnnnnn nnnnnnnnnn nnnnnnnnnn nnnnnnnnnn nnnnnnnnnn nnnnnnnnnn 3300
   nnnnnnnnnn nnnnnnnnnn nnnnnnnnnn nnnnnnnnnn nnnnnnnnnn nnnnnnnnnn 3360
   nnnnnnnnnn nnnnnnnnnn nnnnnnnnnn nnnnnnnnnn nnnnnnnnnn nnnnnnnnnn 3420
   nnnnnnnnnn nnnnnnnnnn nnnnnnnnnn nnnnnnnnnn nnnnnnnnnn nnnnnnnnnn 3480
   nnnnnnnnnn nnnnnnnnnn nnnnnnnnnn nnnnnnnnnn nnnnnnnnnn nnnnnnnnnn 3540
   nnnnnnnnnn nnnnnnnnnn nnnnnnnnnn nnnnnnnnnn nnnnnnnnnn nnnnnnnnnn 3600
   nnnnnnnnnn nnnnnnnnnn nnnnnnnnnn nnnnnnnnnn nnnnnnnnnn nnnnnnnnnn 3660
   nnnnnnnnnn nnnnnnnnnn nnnnnnnnnn nnnnnnnnnn nnnnnnnnnn nnnnnnnnnn 3720
   nnnnnnnnnn nnnnnnnnnn nnnnnnnnnn nnnnnnnnnn nnnnnnnnnn nnnnnnnnnn 3780
   nnnnnnnnnn nnnnnnnnnn nnnnnnnnnn nnnnnnnnnn nnnnnnnnnn nnnnnnnnnn 3840
   nnnnnnnnnn nnnnnnnnnn nnnnnnnnnn nnnnnnnnnn nnnnnnnnnn nnnnnnnnnn 3900
   nnnnnnnnnn nnnnnnnnnn nnnnnnnnnn nnnnnnnnnn nnnnnnnnnn nnnnnnnnnn 3960
   nnnnnnnnnn nnnnnnnnnn nnnnnnnnnn nnnnnnnnnn nnnnnnnnnn nnnnnnnnnn 4020
   nnnnnnnnnn nnnnnnnnnn nnnnnnnnnn nnnnnnnnnn nnnnnnnnnn nnnnnnnnnn 4080
   nnnnnnnnnn nnnnnnnnnn nnnnnnnnnn nnnnnnnnnn nnnnnnnnnn nnnnnnnnnn 4140
   nnnnnnnnnn nnnnnnnnnn nnnnnnnnnn nnnnnnnnnn nnnnnnnnnn nnnnnnnnnn 4200
   nnnnnnnnnn nnnnnnnnnn nnnnnnnnnn nnnnnnnnnn nnnnnnnnnn nnnnnnnnnn 4260
   nnnnnnnnnn nnnnnnnnnn nnnnnnnnnn nnnnnnnnnn nnnnnnnnnn naactaggaa 4320
   tatttcagac agatgcctaa ctgctaaggc tcagagaaac agcaaattaa gtggatatcc 4380
   tttatggatg actgtgattt tttttcccta taatggaagc caagtaaata agatgctatt 4440
   atcccactaa aagcagcggt tctcaacctc tgggtctcca ccccttgagg tcaaatgaca 4500
   cttttaccga gggtcatcta agacattgga aaacacagat atttacatta tgactcacaa 4560
   ccgtagaaaa attacactta tgaagtagca acgaactaat tttatccttg aggaataggg 4620
   ggggtcacca caacatgcag atctgtatta aagggttgca gcattaggaa ggtttagaac 4680
   cactgcccta agagtaggca agactgcagg tctctcatgg gacttgcctg tttgcctagt 4740
   gtctcagcct gcctctcttc ttccctgcac agaggtctag ggtgacagga catgtgacag 4800
   ccagccgagt gaagcagcag ggttggcttc ccttgccctt cacatctgtt tcagcctggg 4860
   atgcagggag cagttgcatg atgtcccgtg ttttcacttc acagaagatc tgtgtgagct 4920
   tccaggagct tcccggggct ttcaatatct gattctggca gctgacagac agctgtgtct 4980
   tccacagccc tccgtgtaga aatcggaatt cgatatcaag ctt                   5023
<210> 6
   <211> 4500
   <212> ADN
   <213> souris
<400> 6
   ctgcagaagc agcattaaga ctgtgctcta aatccaactc ctactttcct tggaagagac 60
   acaagaaaaa gaggcaaggt gacagggcac agacaaagca ctgtagctac agatgcagag 120
   atctgaatta ggaaactcta agccaaggca cacctggaat catcaatagc tggaaaagac 180
   aagaaagatt ttctgtcaga gcctacagga aagtctggct ttgctaacac ctgcattttg 240
   ggccattagc ctcagagctc tgaaatgata atattttgtt gtatttaccc accaaatttg 300
   tggtgattta ttatgaggtc cctaggaaat aaataggact tggtatagtt tttttttttt 360
   ttttcatttc caatcacaaa gcaagaatta ctgcaatgga attaaatttt atttcacaga 420
   attgctggtt ctagtacaat ggtaccgatg ctgaaattgt ttctacatca ggagacttac 480
   agtttaggca catttatttg tatgttgaag accccagata attactttga aaaaagagaa 540
   gtcaagaggc tggaatggag ttccagaatc cccagaatct tgtcttggaa accatttcca 600
   ctacaggatg tccttcactg aagttgctga gtggtgccca tgcaggacta atgaatccac 660
   tgaaagctgt gtggcttaac tggagannnn nnnnnnnnnn nnnnnnnnnn nnnnnnnnnn 720
   nnnnnnnnnn nnnnnnnnnn nnnnnnnnnn nnnnnnnnnn nnnnnnnnnn nnnnnnnnnn 780
   nnnnnnnnnn nnnnnnnnnn nnnnnnnnnn nnnnnnnnnn nnnnnnnnnn nnnnnnnnnn 840
   nnnnnnnnnn nnnnnnnnnn nnnnnnnnnn nnnnnnnnnn nnnnnnnnnn nnnnnnnnnn 900
   nnnnnnnnnn nnnnnnnnnn nnnnnnnnnn nnnnnnnnnn nnnnnnnnnn nnnnnnnnnn 960
   nnnnnnnnnn nnnnnnnnnn nnnnnnnnnn nnnnnnnnnn nnnnnnnnnn nnnnnnnnnn 1020
   nnnnnnnnnn nnnnnnnnnn nnnnnnnnnn nnnnnnnnnn nnnnnnnnnn nnnnnnnnnn 1080
   nnnnnnnnnn nnnnnnnnnn nnnnnnnnnn nnnnnnnnnn nnnnnnnnnn nnnnnnnnnn 1140
   nnnnnnnnnn nnnnnnnnnn nnnnnnnnnn nnnnnnnnnn nnnnnnnnnn nnnnnnnnnn 1200
   nnnnnnnnnn nnnnnnnnnn nnnnnnnnnn nnnnnnnnnn nnnnnnnnnn nnnnnnnnnn 1260
   nnnnnnnnnn nnnnnnnnnn nnnnnnnnnn nnnnnnnnnn nnnnnnnnnn nnnnnnnnnn 1320
   nnnnnnnnnn nnnnnnnnnn nnnnnnnnnn nnnnnnnnnn nnnnnnnnnn nnnnnnnnnn 1380
   nnnnnnnnnn nnnnnnnnnn nnnnnnnnnn nnnnnnnnnn nnnnnnnnnn nnnnnnnnnn 1440
   nnnnnnnnnn nnnnnnnnnn nnnnnnnnnn nnnnnnnnnn nnnnnnnnnn nnnnnnnnnn 1500
   nnnnnnnnnn nnnnnnnnnn nnnnnnnnnn nnnnnnnnnn nnnnnnnnnn nnnnnnnnnn 1560
   nnnnnnnnnn nnnnnnnnnn nnnnnnnnnn nnnnnnnnnn nnnnnnnnnn nnnnnnnnnn 1620
   nnnnnnnnnn nnnnnnnnnn nnnnnnnnnn nnnnnnnnnn nnnnnnnnnn nnnnnnnnnn 1680
   nnnnnnnnnn nnnnnnnnnn nnnnnnnnnn nnnnnnnnnn nnnnnnnnnn nnnnnnnnnn 1740
   nnnnnnnnnn nnnnnnnnnn nnnnnnnnnn nnnnnnnnnn nnnnnnnnnn nnnnnnnnnn 1800
   nnnnnnnnnn nnnnnnnnnn nnnnnnnnnn nnnnnnnnnn nnnnnnnnnn nnnnnnnnnn 1860
   nnnnnnnnnn nnnnnnnnnn nnnnnnnnnn nnnnnnnnnn nnnnnnnnnn nnnnnnnnnn 1920
   nnnnnnnnnn nnnnnnnnnn nnnnnnnnnn nnnnnnnnnn nnnnnnnnnn nnnnnnnnnn 1980
   nnnnnnnnnn nnnnnnnnnn nnnnnnnnnn nnnnnnnnnn nnnnnnnnnn nnnnnnnnnn 2040
   nnnnnnnnnn nnnnnnnnnn nnnnnnnnnn nnnnnnnnnn nnnnnnnnnn nnnnnnnnnn 2100
   nnnnnnnnnn nnnnnnnnnn nnnnnnnnnn nnnnnnnnnn nnnnnnnnnn nnnnnnnnnn 2160
   nnnnnnnnnn nnnnnnnnnn nnnnnnnnnn nnnnnnnnnn nnnnnnnnnn nnnnnnnnnn 2220
   nnnnnnnnnn nnnnnnnnnn nnnnnnnnnn nnnnnnnnnn nnnnnnnnnn nnnnnnnnnn 2280
   nnnnnnnnnn nnnnnnnnnn nnnnnnnnnn nnnnnnnnnn nnnnnnnnnn nnnnnnnnnn 2340
   nnnnnnnnnn nnnnnnnnnn nnnnnnnnnn nnnnnnnnnn nnnnnnnnnn nnnnnnnnnn 2400
   nnnnnnnnnn nnnnnnnnnn nnnnnnnnnn nnnnnnnnnn nnnnnnnnnn nnnnnnnnnn 2460
   nnnnnnnnnn nnnnnnnnnn nnnnnnnnnn nnnnnnnnnn nnnnnnnnnn nnnnnnnnnn 2520
   nnnnnnnnnn nnnnnnnnnn nnnnnnnnnn nnnnnnnnnn nnnnnnnnnn nnnnnnnnnn 2580
   nnnnnnnnnn nnnnnnnnnn nnnnnnnnnn nnnnnnnnnn nnnnnnnnnn nnnnnnnnnn 2640
   nnnnnnnnnn nnnnnnnnnn nnnnnnnnnn nnnnnnnnnn nnnnnnnnnn nnnnnnnnnn 2700
   nnnnnnnnnn nnnnnnnnnn nnnnnnnnnn nnnnnnnnnn nnnnnnnnnn nnnnnnnnnn 2760
   nnnnnnnnnn nnnnnnnnnn nnnnnnnnnn nnnnnnnnnn nnnnnnnnnn nnnnnnnnnn 2820
   nnnnnnnnnn nnnnnnnnnn nnnnnnnnnn nnnnnnnnnn nnnnnnnnnn nnnnnnnnnn 2880
   nnnnnnnnnn nnnnnnnnnn nnnnnnnnnn nnnnnnnnnn nnnnnnnnnn nnnnnnnnnn 2940
   nnnnnnnnnn nnnnnnnnnn nnnnnnnnnn nnnnnnnnnn nnnnnnnnnn nnnnnnnnnn 3000
   nnnnnnnnnn nnnnnnnnnn nnnnnnnnnn nnnnnnnnnn nnnnnnnnnn nnnnnnnnnn 3060
   nnnnnnnnnn nnnnnnnnnn nnnnnnnnnn nnnnnnnnnn nnnnnnnnnn nnnnnnnnnn 3120
   nnnnnnnnnn nnnnnnnnnn nnnnnnnnnn nnnnnnnnnn nnnnnnnnnn nnnnnnnnnn 3180
   nnnnnnnnnn nnnnnnnnnn nnnnnnnnnn nnnnnnnnnn nnnnnnnnnn nnnnnnnnnn 3240
   nnnnnnnnnn nnnnnnnnnn nnnnnnnnnn nnnnnnnnnn nnnnnnnnnn nnnnnnnnnn 3300
   nnnnnnnnnn nnnnnnnnnn nnnnnnnnnn nnnnnnnnnn nnnnnnnnnn nnnnnnngaa 3360
   ttcttaggca aatggatgga actagaaaat atcatcctga gtgaggtaac ccaatcacaa 3420
   aagaacatac atggtatgca ctcactgata agtggatatt acccccaaag tttggaatat 3480
   ccaagataca attcacagac caaatgaagc tcaagaagaa ggaagaacaa agtatggata 3540
   cttcagtctt tcttagaagg gggaacaaaa tactcatggg aggagataca gagacaaagt 3600
   gtggagcaga gactgaagga aaagccattc agagactgcc ccagctgggg atccatccca 3660
   tatacagtca ccaaacccag acactattgt tgatgccaac aagtgcatgc tgacaggagt 3720
   ctgacatagc tgtctcctga gaggctctgc cagtgcctga caaatataga ggggatgctc 3780
   acagccaacc actggactga gcacagggtc ctcaatggag gagttagaga aaggactgaa 3840
   ggaccagaag aggtttgcag ccccatagga ggaacaacaa tatgaactag ccagtatccc 3900
   cagagctccc agggactaag ccaccaacca aagagtacat atgcaggaac ccatggctcc 3960
   agctgcatat gtagcagagg atgtcacttc tcaatgggag gagaggccct tggtcctgag 4020
   aaggctcaat gccccagtgt aggggaatgc caagacaggg aagtgggagt gtgtgggttg 4080
   gtgagcaggg gggaggagga aaggagtagg gggtctttgg aggggaaacc aggaaaggag 4140
   ataacatttg aaatgtaaat gaagaaaata tctaattttt taagaaagaa aaaagacaat 4200
   tacagactta gctgtgcctg tagccactgt tctcctagag ttcagagcct gcctgtctgg 4260
   gaccagacac ctctggctga ggtttcttgc ctggcattat gtgcagggtc cggctccatt 4320
   ttctcaccca gccctgcctt ccacagctct gtgcacagga gaggatccaa ggagaaggga 4380
   agtggaggtg gtggcgacag tgacccagtt cagaaaggac tctgaggcac attcaatcta 4440
   taaagtattc atggctctaa aagccttcca aggggatcca aagcatttgc tgagctgcag 4500
<210> 7
   <211> 17758
   <212> ADN
   <213> souris
<400> 7
   ctgcagaagc agcattaaga ctgtgctcta aatccaactc ctactttcct tggaagagac 60
   acaagaaaaa gaggcaaggt gacagggcac agacaaagca ctgtagctac agatgcagag 120
   atctgaatta ggaaactcta agccaaggca cacctggaat catcaatagc tggaaaagac 180
   aagaaagatt ttctgtcaga gcctacagga aagtctggct ttgctaacac ctgcattttg 240
   ggccattagc ctcagagctc tgaaatgata atattttgtt gtatttaccc accaaatttg 300
   tggtgattta ttatgaggtc cctaggaaat aaataggact tggtatagtt tttttttttt 360
   ttttcatttc caatcacaaa gcaagaatta ctgcaatgga attaaatttt atttcacaga 420
   attgctggtt ctagtacaat ggtaccgatg ctgaaattgt ttctacatca ggagacttac 480
   agtttaggca catttatttg tatgttgaag accccagata attactttga aaaaagagaa 540
   gtcaagaggc tggaatggag ttccagaatc cccagaatct tgtcttggaa accatttcca 600
   ctacaggatg tccttcactg aagttgctga gtggtgccca tgcaggacta atgaatccac 660
   tgaaagctgt gtggcttaac tggagannnn nnnnnnnnnn nnnnnnnnnn nnnnnnnnnn 720
   nnnnnnnnnn nnnnnnnnnn nnnnnnnnnn nnnnnnnnnn nnnnnnnnnn nnnnnnnnnn 780
   nnnnnnnnnn nnnnnnnnnn nnnnnnnnnn nnnnnnnnnn nnnnnnnnnn nnnnnnnnnn 840
   nnnnnnnnnn nnnnnnnnnn nnnnnnnnnn nnnnnnnnnn nnnnnnnnnn nnnnnnnnnn 900
   nnnnnnnnnn nnnnnnnnnn nnnnnnnnnn nnnnnnnnnn nnnnnnnnnn nnnnnnnnnn 960
   nnnnnnnnnn nnnnnnnnnn nnnnnnnnnn nnnnnnnnnn nnnnnnnnnn nnnnnnnnnn 1020
   nnnnnnnnnn nnnnnnnnnn nnnnnnnnnn nnnnnnnnnn nnnnnnnnnn nnnnnnnnnn 1080
   nnnnnnnnnn nnnnnnnnnn nnnnnnnnnn nnnnnnnnnn nnnnnnnnnn nnnnnnnnnn 1140
   nnnnnnnnnn nnnnnnnnnn nnnnnnnnnn nnnnnnnnnn nnnnnnnnnn nnnnnnnnnn 1200
   nnnnnnnnnn nnnnnnnnnn nnnnnnnnnn nnnnnnnnnn nnnnnnnnnn nnnnnnnnnn 1260
   nnnnnnnnnn nnnnnnnnnn nnnnnnnnnn nnnnnnnnnn nnnnnnnnnn nnnnnnnnnn 1320
   nnnnnnnnnn nnnnnnnnnn nnnnnnnnnn nnnnnnnnnn nnnnnnnnnn nnnnnnnnnn 1380
   nnnnnnnnnn nnnnnnnnnn nnnnnnnnnn nnnnnnnnnn nnnnnnnnnn nnnnnnnnnn 1440
   nnnnnnnnnn nnnnnnnnnn nnnnnnnnnn nnnnnnnnnn nnnnnnnnnn nnnnnnnnnn 1500
   nnnnnnnnnn nnnnnnnnnn nnnnnnnnnn nnnnnnnnnn nnnnnnnnnn nnnnnnnnnn 1560
   nnnnnnnnnn nnnnnnnnnn nnnnnnnnnn nnnnnnnnnn nnnnnnnnnn nnnnnnnnnn 1620
   nnnnnnnnnn nnnnnnnnnn nnnnnnnnnn nnnnnnnnnn nnnnnnnnnn nnnnnnnnnn 1680
   nnnnnnnnnn nnnnnnnnnn nnnnnnnnnn nnnnnnnnnn nnnnnnnnnn nnnnnnnnnn 1740
   nnnnnnnnnn nnnnnnnnnn nnnnnnnnnn nnnnnnnnnn nnnnnnnnnn nnnnnnnnnn 1800
   nnnnnnnnnn nnnnnnnnnn nnnnnnnnnn nnnnnnnnnn nnnnnnnnnn nnnnnnnnnn 1860
   nnnnnnnnnn nnnnnnnnnn nnnnnnnnnn nnnnnnnnnn nnnnnnnnnn nnnnnnnnnn 1920
   nnnnnnnnnn nnnnnnnnnn nnnnnnnnnn nnnnnnnnnn nnnnnnnnnn nnnnnnnnnn 1980
   nnnnnnnnnn nnnnnnnnnn nnnnnnnnnn nnnnnnnnnn nnnnnnnnnn nnnnnnnnnn 2040
   nnnnnnnnnn nnnnnnnnnn nnnnnnnnnn nnnnnnnnnn nnnnnnnnnn nnnnnnnnnn 2100
   nnnnnnnnnn nnnnnnnnnn nnnnnnnnnn nnnnnnnnnn nnnnnnnnnn nnnnnnnnnn 2160
   nnnnnnnnnn nnnnnnnnnn nnnnnnnnnn nnnnnnnnnn nnnnnnnnnn nnnnnnnnnn 2220
   nnnnnnnnnn nnnnnnnnnn nnnnnnnnnn nnnnnnnnnn nnnnnnnnnn nnnnnnnnnn 2280
   nnnnnnnnnn nnnnnnnnnn nnnnnnnnnn nnnnnnnnnn nnnnnnnnnn nnnnnnnnnn 2340
   nnnnnnnnnn nnnnnnnnnn nnnnnnnnnn nnnnnnnnnn nnnnnnnnnn nnnnnnnnnn 2400
   nnnnnnnnnn nnnnnnnnnn nnnnnnnnnn nnnnnnnnnn nnnnnnnnnn nnnnnnnnnn 2460
   nnnnnnnnnn nnnnnnnnnn nnnnnnnnnn nnnnnnnnnn nnnnnnnnnn nnnnnnnnnn 2520
   nnnnnnnnnn nnnnnnnnnn nnnnnnnnnn nnnnnnnnnn nnnnnnnnnn nnnnnnnnnn 2580
   nnnnnnnnnn nnnnnnnnnn nnnnnnnnnn nnnnnnnnnn nnnnnnnnnn nnnnnnnnnn 2640
   nnnnnnnnnn nnnnnnnnnn nnnnnnnnnn nnnnnnnnnn nnnnnnnnnn nnnnnnnnnn 2700
   nnnnnnnnnn nnnnnnnnnn nnnnnnnnnn nnnnnnnnnn nnnnnnnnnn nnnnnnnnnn 2760
   nnnnnnnnnn nnnnnnnnnn nnnnnnnnnn nnnnnnnnnn nnnnnnnnnn nnnnnnnnnn 2820
   nnnnnnnnnn nnnnnnnnnn nnnnnnnnnn nnnnnnnnnn nnnnnnnnnn nnnnnnnnnn 2880
   nnnnnnnnnn nnnnnnnnnn nnnnnnnnnn nnnnnnnnnn nnnnnnnnnn nnnnnnnnnn 2940
   nnnnnnnnnn nnnnnnnnnn nnnnnnnnnn nnnnnnnnnn nnnnnnnnnn nnnnnnnnnn 3000
   nnnnnnnnnn nnnnnnnnnn nnnnnnnnnn nnnnnnnnnn nnnnnnnnnn nnnnnnnnnn 3060
   nnnnnnnnnn nnnnnnnnnn nnnnnnnnnn nnnnnnnnnn nnnnnnnnnn nnnnnnnnnn 3120
   nnnnnnnnnn nnnnnnnnnn nnnnnnnnnn nnnnnnnnnn nnnnnnnnnn nnnnnnnnnn 3180
   nnnnnnnnnn nnnnnnnnnn nnnnnnnnnn nnnnnnnnnn nnnnnnnnnn nnnnnnnnnn 3240
   nnnnnnnnnn nnnnnnnnnn nnnnnnnnnn nnnnnnnnnn nnnnnnnnnn nnnnnnnnnn 3300
   nnnnnnnnnn nnnnnnnnnn nnnnnnnnnn nnnnnnnnnn nnnnnnnnnn nnnnnnngaa 3360
   ttcttaggca aatggatgga actagaaaat atcatcctga gtgaggtaac ccaatcacaa 3420
   aagaacatac atggtatgca ctcactgata agtggatatt acccccaaag tttggaatat 3480
   ccaagataca attcacagac caaatgaagc tcaagaagaa ggaagaacaa agtatggata 3540
   cttcagtctt tcttagaagg gggaacaaaa tactcatggg aggagataca gagacaaagt 3600
   gtggagcaga gactgaagga aaagccattc agagactgcc ccagctgggg atccatccca 3660
   tatacagtca ccaaacccag acactattgt tgatgccaac aagtgcatgc tgacaggagt 3720
   ctgacatagc tgtctcctga gaggctctgc cagtgcctga caaatataga ggggatgctc 3780
   acagccaacc actggactga gcacagggtc ctcaatggag gagttagaga aaggactgaa 3840
   ggaccagaag aggtttgcag ccccatagga ggaacaacaa tatgaactag ccagtatccc 3900
   cagagctccc agggactaag ccaccaacca aagagtacat atgcaggaac ccatggctcc 3960
   agctgcatat gtagcagagg atgtcacttc tcaatgggag gagaggccct tggtcctgag 4020
   aaggctcaat gccccagtgt aggggaatgc caagacaggg aagtgggagt gtgtgggttg 4080
   gtgagcaggg gggaggagga aaggagtagg gggtctttgg aggggaaacc aggaaaggag 4140
   ataacatttg aaatgtaaat gaagaaaata tctaattttt taagaaagaa aaaagacaat 4200
   tacagactta gctgtgcctg tagccactgt tctcctagag ttcagagcct gcctgtctgg 4260
   gaccagacac ctctggctga ggtttcttgc ctggcattat gtgcagggtc cggctccatt 4320
   ttctcaccca gccctgcctt ccacagctct gtgcacagga gaggatccaa ggagaaggga 4380
   agtggaggtg gtggcgacag tgacccagtt cagaaaggac tctgaggcac attcaatcta 4440
   taaagtattc atggctctaa aagccttcca aggggatcca aagcatttgc tgagctgcag 4500
   nnnnnnnnnn nnnnnnnnnn nnnnnnnnnn nnnnnnnnnn nnnnnnnnnn nnnnnnnnnn 4560
   nnnnnnnnnn nnnnnnnnnn nnnnnnnnnn nnnnnnnnnn nnnnnnnnnn nnnnnnnnnn 4620
   nnnnnnnnnn nnnnnnnnnn nnnnnnnnnn nnnnnnnnnn nnnnnnnnnn nnnnnnnnnn 4680
   nnnnnnnnnn nnnnnnnnnn nnnnnnnnnn nnnnnnnnnn nnnnnnnnnn nnnnnnnnnn 4740
   nnnnnnnnnn nnnnnnnnnn nnnnnnnnnn nnnnnnnnnn nnnnnnnnnn nnnnnnnnnn 4800
   nnnnnnnnnn nnnnnnnnnn nnnnnnnnnn nnnnnnnnnn nnnnnnnnnn nnnnnnnnnn 4860
   nnnnnnnnnn nnnnnnnnnn nnnnnnnnnn nnnnnnnnnn nnnnnnnnnn nnnnnnnnnn 4920
   nnnnnnnnnn nnnnnnnnnn nnnnnnnnnn nnnnnnnnnn nnnnnnnnnn nnnnnnnnnn 4980
   nnnnnnnnnn nnnnnnnnnn nnnnnnnnnn nnnnnnnnnn nnnnnnnnnn nnnnnnnnnn 5040
   nnnnnnnnnn nnnnnnnnnn nnnnnnnnnn nnnnnnnnnn nnnnnnnnnn nnnnnnnnnn 5100
   nnnnnnnnnn nnnnnnnnnn nnnnnnnnnn nnnnnnnnnn nnnnnnnnnn nnnnnnnnnn 5160
   nnnnnnnnnn nnnnnnnnnn nnnnnnnnnn nnnnnnnnnn nnnnnnnnnn nnnnnnnnnn 5220
   nnnnnnnnnn nnnnnnnnnn nnnnnnnnnn nnnnnnnnnn nnnnnnnnnn nnnnnnnnnn 5280
   nnnnnnnnnn nnnnnnnnnn nnnnnnnnnn nnnnnnnnnn nnnnnnnnnn nnnnnnnnnn 5340
   nnnnnnnnnn nnnnnnaagc ttnnnnnnnn nnnnnnnnnn nnnnnnnnnn nnnnnnnnnn 5400
   nnnnnnnnnn nnnnnnnnnn nnnnnnnnnn nnnnnnnnnn nnnnnnnnnn nnnnnnnnnn 5460
   nnnnnnnnnn nnnnnnnnnn nnnnnnnnnn nnnnnnnnnn nnnnnnnnnn nnnnnnnnnn 5520
   nnnnnnnnnn nnnnnnnnnn nnnnnnnnnn nnnnnnnnnn nnnnnnnnnn nnnnnnnnnn 5580
   nnnnnnnnnn nnnnnnnnnn nnnnnnnnnn nnnnnnnnnn nnnnnnnnnn nnnnnnnnnn 5640
   nnnnnnnnnn nnnnnnnnnn nnnnnnnnnn nnnnnnnnnn nnnnnnnnnn nnnnnnnnnn 5700
   nnnnnnnnnn nnnnnnnnnn nnnnnnnnnn nnnnnnnnnn nnnnnnnnnn nnnnnnnnnn 5760
   nnnnnnnnnn nnnnnnnnnn nnnnnnnnnn nnnnnnnnnn nnnnnnnnnn nnnnnnnnnn 5820
   nnnnnnnnnn nnnnnnnnnn nnnnnnnnnn nnnnnnnnnn nnngcatgcn nnnnnnnnnn 5880
   nnnnnnnnnn nnnnnnnnnn nnnnnnnnnn nnnnnnnnnn nnnnnnnnnn nnnnnnnnnn 5940
   nnnnnnnnnn nnnnnnnnnn nnnnnnnnnn nnnnnnnnnn nnnnnnnnnn nnnnnnnnnn 6000
   nnnnnnnnnn nnnnnnnnnn nnnnnnnnnn nnnnnnnnnn nnnnnnnnnn nnnnnnnnnn 6060
   nnnnnnnnnn nnnnnnnnnn nnnnnnnnnn nnnnnnnnnn nnnnnnnnnn nnnnnnnnnn 6120
   nnnnnnnnnn nnnnnnnnnn nnnnnnnnnn nnnnnnnnnn nnnnnnnnnn nnnnnnnnnn 6180
   nnnnnnnnnn nnnnnnnnnn nnnnnnnnnn nnnnnnnnnn nnnnnnnnnn nnnnnnnnnn 6240
   nnnnnnnnnn nnnnnnnnnn nnnnnnnnnn nnnnnnnnnn nnnnnnnnnn nnnnnnnnnn 6300
   nnnnnnnnnn nnnnnnnnnn ccatggnnnn nnnnnnnnnn nnnnnnnnnn nnnnnnnnnn 6360
   nnnnnnnnnn nnnnnnnnnn nnnnnnnnnn nnnnnnnnnn nnnnnnnnnn nnnnnnnnnn 6420
   nnnnnnnnnn nnnnnnnnnn nnnnnnnnnn nnnnnnnnnn nnnnnnnnnn nnnnnnnnnn 6480
   nnnnnnnnnn nnnnnnnnnn nnnnnnnnnn nnnnnnnnnn nnnnnnnnnn nnnnnnnnnn 6540
   nnnnnnnnnn nnnnnnnnnn nnnnnnnnnn nnnnnnnnnn nnnnnnnnnn nnnnnnnnnn 6600
   nnnnnnnnnn nnnnnnnnnn nnnnnnnnnn nnnnnnnnnn nnnnnnnnnn nnnnnnnnnn 6660
   nnnnnnnnnn nnnnnnnnnn nnnnnnnnnn nnnnnnnnnn nnnnnnnnnn nnnnnnnnnn 6720
   nnnnnnnnnn nnnnnnnnnn nnnnnnnnnn nnnnnnnnnn nnnnnnnnnn nnnnnnnnnn 6780
   nnnnnnnnnn nnnnnnnnnn nnnnnnnnnn nnnnnnnnnn nnnnnnnnnn nnnnnnnnnn 6840
   nnnnnnnnnn nnnnnnnnnn nnnnnnnnnn nnnnnnnnnn nnnnnnnnnn nnnnnnnnnn 6900
   nnnnnnnnnn nnnnnnnnnn nnnnnnnnnn nnnnnnnnnn nnnnnnnnnn nnnnnnnnnn 6960
   nnnnnnnnnn nnnnnnnnnn nnnnnnnnnn nnnnnnnnnn nnnnnnnnnn nnnnnnnnnn 7020
   nnnnnnnnnn nnnnnnnnnn nnnnnnnnnn nnnnnnnnnn nnnnnnnnnn nnnnnnnnnn 7080
   nnnnnnnnnn nnnnnnnnnn nnnnnnnnnn nnnnnnnnnn nnnnnnnnnn nnnnnnnnnn 7140
   nnnnnnnnnn nnnnnnnnnn nnnnnnnnnn nnnnnnnnnn nnnnnnnnnn nnnnnnnnnn 7200
   nnnnnnnnnn nnnnnnnnnn nnnnnnnnnn nnnnnnnnnn nnnnnnnnnn nnnnnnnnnn 7260
   nnnnnnnnnn nnnnnnnnnn nnnnnnnnnn nnnnnnnnnn nnnnnnnnnn nnnnnnnnnn 7320
   nnnnnnnnnn nnnnnnnnnn nnnnnnnnnn nnnnnnnnnn nnnnnnnnnn nnnnnnnnnn 7380
   nnnnnnnnnn nnnnnnnnnn nnnnnnnnnn nnnnnnnnnn nnnnnnnnnn nnnnnnnnnn 7440
   nnnnnnnnnn nnnnnnnnnn nnnnnnnnnn nnnnnnnnnn nnnnnnnnnn nnnnnnnnnn 7500
   nnnnnnnnnn nnnnnnnnnn nnnnnnnnnn nnnnnnnnnn nnnnnnnnnn nnnnnnnnnn 7560
   nnnnnnnnnn nnnnnnnnnn nnnnnnnnnn nnnnnnnnnn nnnnnnnnnn nnnnnnnnnn 7620
   nnnnnnnnnn nnnnnnnnnn nnnnnnnnnn nnnnnnnnnn nnaactagga atatttcaga 7680
   cagatgccta actgctaagg ctcagagaaa cagcaaatta agtggatatc ctttatggat 7740
   gactgtgatt tttttttcct ataatggaag ccaagtaaat aagatgctat tatcccacta 7800
   aaagcagcgg ttctcaacct ctgggtctcc accccttgag gtcaaatgac acttttaccg 7860
   agggtcatct aagacattgg aaaacacaga tatttacatt atgactcaca accgtagaaa 7920
   aattacactt atgaagtagc aacgaactaa ttttatcctt gaggaatagg gggggtcacc 7980
   acaacatgca gatctgtatt aaagggttgc agcattagga aggtttagaa ccactgccct 8040
   aagagtaggc aagactgcag gtctctcatg ggacttgcct gtttgcctag tgtctcagcc 8100
   tgcctctctt cttccctgca cagaggtcta gggtgacagg acatgtgaca gccagccgag 8160
   tgaagcagca gggttggctt cccttgccct tcacatctgt ttcagcctgg gatgcaggga 8220
   gcagttgcat gatgtcccgt gttttcactt cacagaagat ctgtgtgagc ttccaggagc 8280
   ttcccggggc tttcaatatc tgattctggc agctgacaga cagctgtgtc ttccacagcc 8340
   ctccgtgtag aaatcggaat tcagagagct tttatcatgc tgaacagccg ggaccaggga 8400
   aacctgcact ccggtttatg tctgtggcta tgtggattcc tggctctctt taaaggtgag 8460
   ttattacggc ttatatccta gaatacaatt ggtagtgtgc atttcctctt ctttctcatc 8520
   ttgtctggat ccaaataatt agaggacttt aattagttag gcttaaccta acagatatga 8580
   tttggaaagt aaggtagaga aaacattgaa gtacagtata atattcatac ttcatctctc 8640
   caactaaaat atgcttttcc aattagcata caaaatcatc tgcttttgta gccatgtatg 8700
   ctaccaatat gcttcattta cttacggtaa tttgttagct gggacttatt agcctgcgag 8760
   tcctaccaat gtgtgtaatt gtcctcactt aggaaacctg tcttattgtc tactaagagt 8820
   gagctttcct gtcttaaaag ttggctcaga cctggctggt gcacacacac acacacacac 8880
   acacacacac acacacacac acacgaacag gcacacatgc acacatctcg gatgaggttt 8940
   cagggggtgc tgatggtgag ctccagagag aggaaacagt agcaacagcc aagagagctc 9000
   actatcttct gaaacaatgt tttcatcctg gttagttaca caggnnnnnn nnnnnnnnnn 9060
   nnnnnnnnnn nnnnnnnnnn nnnnnnnnnn nnnnnnnnnn nnnnnnnnnn nnnnnnnnnn 9120
   nnnnnnnnnn nnnnnnnnnn nnnnnnnnnn nnnnnatgca tnnnnnnnnn nnnnnnnnnn 9180
   nnnnnnnnnn nnnnnnnnnn nnnnnnnnnn nnnnnnnnnn nnnnnnnnnn nnnnnnnnnn 9240
   nnnnnnnnnn nnnnnnnnnn nnnnnnnnnn nnnnnnnnnn nnnnnnnnnn nnnnnnnnnn 9300
   nnnnnnnnnn nnnnnnnnnn nnnnnnnnnn nnnnnnnnnn nnnnnnnnnn nnnnnnnnnn 9360
   nnnnnnnnnn nnnnnnnnnn nnnnnnnnnn nnnnnnnnnn nnnnnnnnnn nnnnnnnnnn 9420
   nnnnnnnnnn nnnnnnnnnn nnnnnnnnnn nnnnnncata tgnnnnnnnn nnnnnnnnnn 9480
   nnnnnnnnnn nnnnnnnnnn nnnnnnnnnn nnnnnnnnnn nnnnnnnnnn nnnnnnnnnn 9540
   nnnnnnnnnn nnnnnnnnnn nnnnnnnnnn nnnnnnnnnn nnnnnnnnnn nnnnnnnnnn 9600
   nnnnnnnnnn nnnnnnnnnn nnnnnnnnnn nnnnnnnnnn nnnnnnnnnn nnnnnnnnnn 9660
   nnnnnnnnnn nnnnnnnnnn nnnnnnnnnn nnnnnnnnnn nnnnnnnnnn nnnnnnnnnn 9720
   nnnnnnnnnn nnnnnnnnnn nnnnnnnnnn nnnnnnnnnn nnnnnnnnnn nnnnnnnnnn 9780
   nnnnnnnnnn nnnnnnnnnn nnnnnnnnnn nnnnnnnnnn nnnnnnnnnn nnnnnnnnnn 9840
   nnnnnnnnnn nnnnnnnnnn nnnnnnnnnn nnnnnnnnnn nnnnnnnnnn nnnnnnnnnn 9900
   nnnnnnnnnn nnnnnnnnnn nnnnnnnnnn nnnnnnnnnn nnnnnnnnnn nnnnnnnnnn 9960
   nnnnnnnnnn nnnnnnnnnn nnnnnnnnnn nnnnnnnnnn nnnnnnnnnn nnnnnnnnnn 10020
   nnnnnnnnnn nnnnnnnnnn nnnnnnnnnn nnnnnnnnnn nnnnnnnnnn nnnnnnnnnn 10080
   nnnnnnnnnn nnnnnnnnnn nnnnnnnnnn nnnnnnnnnn nnnnnnnnnn nnnnnnnnnn 10140
   nnnnnnnnnn nnnnnnnnnn nnnnnnnnnn nnnnnnnnnn nnnnnnnnnn nnnnnnnnnn 10200
   nnnnnngcag tacaggctgt gaatctgaag agcagctctt ccacaggctg tttgctcact 10260
   acaaccgctt catccggccg gtggagaatg tctccgatcc cgtcacggtg catcttgaat 10320
   tggcaatcac gcaactggcc aatgtgnnnn nnnnnnnnnn nnnnnnnnnn nnnnnnnnnn 10380
   nnnnnnnnnn nnnnnnnnnn nnnnnnnnnn nnnnnnnnnn nnnnnnnnnn nnnnnnnnnn 10440
   nnnnnnnnnn nnnnnnnnnn nnnnnnnnnn nnnnnnnnnn nnnnnnnnnn nnnnnnnnnn 10500
   nnnnnnnnnn nnnnnnnnnn nnnnnnnnnn nnnnnnnnnn nnnnnnnnnn nnnnnnnnnn 10560
   nnnnnnnnnn nnnnnnnnnn nnnnnnnnnn nnnnnnnnnn nnnnnntcta gannnnnnnn 10620
   nnnnnnnnnn nnnnnnnnnn nnnnnnnnnn nnnnnnnnnn nnnnnnnnnn nnnnnnnnnn 10680
   nnnnnnnnnn nnnnnnnnnn nnnnnnnnnn nnnnnnnnnn nnnnnnnnnn nnnnnnnnnn 10740
   nnnnnnnnnn nnnnnnnnnn nnnnnnnnnn nnnnnnnnnn nnnnnnnnnn nnnnnnnnnn 10800
   nnnnnnnnnn nnnnnnnnnn nnnnnnnnnn nnnnnnnnnn nnnnnnnnnn nnnnnnnact 10860
   ccctgggctg agcatataca aaccatcaca ccatgtctgc cagttgattg cagcttttcc 10920
   agaaactagt gcatttgaaa ccatgattca aaattgttca cctgggtgtc aactaatatt 10980
   ttcccgataa agaggtctag gctaatggtt ccaacaacgt ggtgtggtac tgtgtgtctc 11040
   tggcctcctc ctccattagg gctcactgag ctcagtgtcc attgaaatgg tctggagttg 11100
   catgaagact atgatttttc cagagtaggt cctgactcat gacattttct aacacctcca 11160
   aggttgtgta ttccccatga atacacacca tatggagcct atccaaacca tggaagtgtc 11220
   tcaagggagg caggcctgtt aagaatggta ctgataatta gtgtcaactc cctgaactca 11280
   cccaggtttt gaagactatg acaggtttag gccagttacc tctaagacct cacggaagtg 11340
   aggcagcaga gcctatccag ctttgaccaa cagctagtac atacttaccc agcatctgtt 11400
   agaccctaca gattcaaggc gactcaatac cagagggccc taatcaatag aggggaatca 11460
   ctgggaagct gtgcaggctc cattttccta agtctttttg ttgactttac caatcccagg 11520
   gcctgtgttg gctccacatg acccactggc agggagaagc ttnnnnnnnn nnnnnnnnnn 11580
   nnnnnnnnnn nnnnnnnnnn nnnnnnnnnn nnnnnnnnnn nnnnnnnnnn nnnnnnnnnn 11640
   nnnnnnnnnn nnnnnnnnnn nnnnnnnnnn nnncctgcgt agaaacctca accaggctgg 11700
   gatccttcac tcacagggac ctcagatgtc ttggaaaatg aaattttgaa tcccctgctg 11760
   actgctaaga acctactatt aatacccaag ctattctgct gtgacctgtc acctgtcctg 11820
   actctcctta tgtcacttca gggactgctt ggacagtaac tcttcaaaga agcctccttg 11880
   actcgtatat taaatagatg tgtgcataca cgcaggaatc actccacctc tcttgttata 11940
   tcatgatgtg ttttatgctg gcgtacacat ttattattag ctctgtcttt ccaaccacaa 12000
   cagctaagag cacatgaagg caggagcttt cttcactctg taacttgagt ggaactctgt 12060
   aacacagtgg tcactgggta agtaggaata tcccttgtga ctgaagggcc cgtgagctgg 12120
   gaaagggaat acagagggag tgagtacctg gtttcaactg gcaaacttgc atgtgaacta 12180
   aactgctgtt tcagtttgga tcccagggca aatgcacaca gtacatccca ggcttctttg 12240
   aggaaaggcc atggggatag ataaaaacag tgagtacctg tcgccaccta caggactctc 12300
   ccctctgtcg tccctgaacc tctgccctcc tccctgttac ctttcatgca tctaggaagc 12360
   tttatgggcc aaggtggtga gaaaagattc acagaggagg tgaaatccaa atgatctttg 12420
   atcaatattt taagtcacat gtcatttcta agtcaacaga gcagaggcaa ctggaaacgt 12480
   tcgggatttc tgttacagtt aaatagcttt catgcagtct ccagtcttca tgtctgactt 12540
   cattagcacg gataacttag atttgtctat ttttagatat aattctctag ttaagacttg 12600
   tattagcaag cacatagaag actgaaaaat attatttctt ccttccagga tgaagtcaac 12660
   cagattatgg agaccaatct gtggctgcgt cacgtatgtg tccccccctt tgaatggcgg 12720
   cagaatgtat ccacttagtg ataaagccac ctgcattaac tttttcgcac cccaacctat 12780
   gatagataaa gaatatcctt ttccttgctt tctcctagtc cttgggtcag ctctggttgc 12840
   agttatatta atataggcag cacatgggca gagcctggtg tctgacatgg aaccctctgg 12900
   cctttctctt ttaagctccc agttctcttt gtatcactta ctgataccaa gnnnnnnnnn 12960
   nnnnnnnnnn nnnnnnnnnn nnnnnnnnnn nnnnnnnnnn nnnnnnnnnn nnnnnnnnnn 13020
   nnnnnnnnnn nnnnnnnnnn nnnnnnnnnn nnnnnnnnnn nnnnnnnnnn nnnnnnnnnn 13080
   nnnnnnnnnn nnnnnnnnnn nnnnnnnnnn nnnnnnnnnn nnnnnnnnnn nnnnnnnnnn 13140
   nnnnnnnnnn nnnnnnnnnn nnnnnnnnnn nnnnnnnnnn nnnnnnnnnn nnnnnnnnnn 13200
   nnnnnnnnnn nnnnnnnnnn nnnnnnnnnn nnnnnnnnnn nnnnnnnnnn nnnnnnnnnn 13260
   nnnnnnnnnn nnnnnnnnnn nnnnnnnnnn nnnnnnnnnn nnnnnnnnnn nnnnnnnnnn 13320
   nnnnnnnnnn nnnnnnnnnn nnnnnnnnnn nnnnnnnnnn nnnnnnnnnn nnnnnnnnnn 13380
   nnnnnnnnnn nnnnnnnnnn nnnnnnnnnn nnnnnnnnnn nnnnnnnnnn nnnnnnnnnn 13440
   nnnnnnnnnn nnnnnnnnnn nnnnnnnnnn natctggaag gactacagat tgcgttggga 13500
   tccaacggag tatgatggca tcgagacact tcgagttcca gcagacaaca tctggaagcc 13560
   tgacatcgtt ctgtataacn nnnnnnnnat gcatnnnnnn nnnnnnnnnn nnnnnnnnnn 13620
   nnnnnnnnnn nnnnnnnnnn nnnnnnnnnn nnnnnnnnnn nnnnnnnnnn nnnnnnnnnn 13680
   nnnnnnnnnn nntctagann nnnnnnnnnn nnnnnnnnnn nnnnnnnnnn nnnnnnnnnn 13740
   nnnnnnnnnn nnnnnnnnnn nnnnnnnnnn nnnnnnnnnn nnnnnnnnnn nnnnnnnnnn 13800
   nnnnnnnnnn nnnnnnnnnn nnnnnnnnnn nnnnnnnnnn nnnnnnnnnn nnnnnnnnnn 13860
   nnnnnnnnnn nnnnnnnnnn nnnnnnnnnn nnnnnnnnnn nnnnnnnnnn nnnnnnnnnn 13920
   nnnnnnnnnn nnnnnnnnnn nnnnnnnnnn nnnnnnnnnn nnnnnnnnnn nnnnnnnnnn 13980
   nnnnnnnnnn nnnnnnnnnn nnnnnnnnnn nnnnnnnnnn nnnnnnnnnn nnnnnnnnnn 14040
   nnnnnnnnnn nnnnnnnnnn nnnnnnnnnn nnnnnnnnnn nnnnnnnnnn nnnnnnnnnn 14100
   nnnnnnnnnn nnnnnnnnnn nnnnnnnnnn nnnnnnnnnn nnnnnnnnnn nnnnnnnnnn 14160
   nnnnnnnnnn nnnnnnnnnn nnnnnnnnnn nnnnnnnnnn nnnnnnnnnn nnnnnnnnnn 14220
   nnnnnnnnnn nnnnnnggat ccnnnnnnnn nnnnnnnnnn nnnnnnnnnn nnnnnnnnnn 14280
   nnnnnnnnnn nnnnnnnnnn nnnnnnnnnn nnnnnnnnnn nnnnnnnnnn nnnnnnnnnn 14340
   nnnnnnnnnn nnnnnnnnnn nnnnnnnnnn nnnnnnnnnn nnnnnnnnnn nnnnnnnnnn 14400
   nnnnnnnnnn nnnnnnnnnn nnnnnnnnnn nnnnnnnnnn nnnnnnnnnn nnnnnnnnnn 14460
   nnnnnnnnnn nnnnnnnnnn nnnnnnnnnn nnnnnnnnnn nnnnnnnnnn nnnnnnnnnn 14520
   nnnnnnnnnn nnnnnnnnnn nnnnnnnnnn nnnnnnnnnn nnnnnnnnnn nnnnnnnnnn 14580
   nnnnnnnnnn nnnnnnnnnn nnnnnnnnnn nnnnnnnnnn nnnnnnnnnn nnnnnnnnnn 14640
   nnaatgctgt cggcgacttc caggtcgaag gcaagaccaa agctcttctc aagtatgacg 14700
   gtgtgataac ctggacccca ccagccatct ttaagagctc ctgcccaatg gacatcacct 14760
   tcttcccgtt tgaccatcaa aactgctccc tgaagtttgg ttcctggact tatgacaagg 14820
   cagaaatcga ccttctcatc attggctcta aagtagacat gaacgacttt tgggaaaaca 14880
   gtgaatggga aattgtcgac gcctctggct ataagcatga catcaagtac aactgctgtg 14940
   aagagattta cacggacata acctactcct tctacatcag gaggttgccc atgttttaca 15000
   ccatcaacct catcatcccc tgcctcttca tttccttcct cacggtgctg gttttttacc 15060
   ttccctccga ctgtggcgag aaagtgactc tttgcatctc cgttctgctt tctctcactg 15120
   tctttttgct ggtgattaca gagaccatcc catccacatc tctcgtgatc ccactggtgg 15180
   gtgagtatct actgttcacc atgatctttg tcacgctgtc cattgtggtg accgtgttcg 15240
   tgctgaacat acactacagg accccagcaa cgcataccat gcccaagtgg gtgaagacca 15300
   tcttccttca ggccttcccc tcgattctga tgatgaggaa acctctggac aagacaaagg 15360
   aggcaggagg tgttaaggac cccaaaagcc ataccaagag gcctgccaag gtcaaattta 15420
   ctcatcgagg agaatccaaa cttctaaagg aatgccacca ctgccaaaaa tcaagtgaca 15480
   tagcacctgg aaagagaaga tcaagccagc agcctgcacg gtgggtggca gagaattcag 15540
   agcactcgtc cgatgttgaa gatgtcatcg agagtgttca attcatagca gaaaacatga 15600
   agagccacaa tgaaacaaac gaggtaaaag tggagccctt ttctccagcc agctgcaccc 15660
   ctagcaggcc tacaggcact ttagagacta gtcagagcgt cagtgggagt tacatatgtg 15720
   gaacagtcag ggaccgtcac ctaagaccag ctctattatc atgaagcctt gtgggacctg 15780
   ggttcaagtt tagggagcta tagtgagagg atatatgtag tcctacaaca aatcttcagc 15840
   ctgcatttac ttacggtgag gtctagccac agtgcacatg caggacaagc cttcctcaag 15900
   gaacaagcct ccaatgcatc gaacactgac aaagtgaggg tgggaaggga gactgtagaa 15960
   atcattatta ataaaatccc accggcgggc ttgctacctg ctctaatggt ttgtgttccc 16020
   aaatgaaaca cacacacaca cacacacaca cacacacaca cacacacagt ctttgtgttt 16080
   taatatgccg tatacagcac aatagctggg ccactgccta ccctccatgc tgttactata 16140
   cctcccgcca acaatcccca agttattact tactaattct atgtgctatc ttggttgccc 16200
   ctagacccag ttgggcagcc tctgggccat gttttccccg gctatttcaa gtggtggcca 16260
   tgtctctctg tcctgcagtc ttctcaggcc tagcatctca cctcttcctc cacactctcc 16320
   cggcatggca gggtctcact tctcctcctc ctcctcctcc tcctccacca cctcctttct 16380
   cctccctccc tcctccctcc cctctttccc ctcccccctt ccttcccagc ctgagaactc 16440
   ctaaaatccc acctctccct gtcctttcca gctttggctg ttggcagctt tatttaccaa 16500
   taagaaccaa ctgcgggcag gttcccagaa actacaggca gacagtccca cgaaaacagt 16560
   tttacgtgga ccataattag cattcataat acgtgcagct acagagacac ccaaggaaaa 16620
   gaagaactgt ggtactgcct gaagccccag tggtgaaaga tgccccttag cagtcagcag 16680
   ctcccatcac acttcctcta ctagaggggg acccagacat gagttaaaga catcttgaag 16740
   atgtgctcag tatgcacacg tattccgtgc ctgtgtccca cactctaatg cctagagctt 16800
   ggctcctcct accccaagca agtgctcaag gatagggaga tcaccacttg actgaggttt 16860
   cccgccaggt gtctggagat gcttcatact atagctccta acatctgtaa ctcataggga 16920
   aagaatatca gaaccaccac caccccccaa aaaacacact gagagacact gagaatgcct 16980
   gtcataacat gtcacaggag agcaatggcc agtgagctca cctcctggct ggcatctttt 17040
   agaatgtacg gcaatacgtt acgttgattt atcannnnnn nnnnnnnnnn nnnnnnnnnn 17100
   nnnnnnnnnn nnnnnnnnnn nnnnnnnnnn nnnnnnnnnn nnnnnnnnnn nnnnnnnnnn 17160
   nnnnnnnnnn nnnnnnnnnn nnnnnnnnnn nnnnnnnnnn nnnnnnnnnn nnnnnnnnnn 17220
   nnnnnnnnnn nnnnnnnnct ctcacttgct tcacactcag attcccccca aacagccagg 17280
   cacacgtgac aagagcttga ctctcaagca gatatattgt gtagagcgaa gaaaggaccc 17340
   ttatcacagg caaagggtca caaaagtctt tgatacagtt catagcccag tgatgtacat 17400
   ctgctctccc ttagaccatg agtcctacat aggacatgca ggcagtcagg caggcaaaca 17460
   taatagcaat aattggtttt tttgaaagga ttggagaaaa tgctttggag aaaaaaaaaa 17520
   gtattcataa aacatagcat gttttcttat agtcttggtt tttaaagcta gccttgatcc 17580
   tttgtttgca tttcaggtag aagacgactg gaaatacatg gctatggtgg tggacagagt 17640
   cttcctttgg gtatttataa ttgtctgtgt gtttggaact gtggggctat ttctgcagcc 17700
   actgcttggg aacacaggaa actcttaatt ggtattgtcc ctccgagctc atcaagct   17758